# EUROPEAN PATENT SPECIFICATION

(11) **EP 1 572 912 B1**
(45) Date of publication and mention of the grant of the patent: **30.09.2015**
(21) Application number: 03716849.9
(22) Date of filing: 27.03.2003
(51) Int. Cl.: C12N 15/09, C12N 5/10, C12N 15/63

(54) **SELECTION FREE GROWTH OF HOST CELLS CONTAINING MULTIPLE INTEGRATING VECTORS**
SELEKTIONSFREIES WACHSTUM VON MEHRERE INTEGRATIONSVEKTOREN ENTHALTENDEN WIRTSZELLEN
CROISSANCE SANS SELECTION DE CELLULES HOTES CONTENANT DE MULTIPLES VECTEURS D'INTEGRATION

(30) Priority: 28.03.2002 US 368357; 26.03.2003 US 397079
(43) Date of publication of application: 14.09.2005
(73) Proprietor: Catalent Pharma Solutions, LLC, Somerset, NJ 08873 (US)
(72) Inventor: BREMEL, Robert, D., Hillpoint, Wisconsin 53937 (US); YORK, Dona, Wisconsin Dells, WI 53965 (US); BLECK, Gregory, T., Baraboo, WI 53913 (US); EAKLE, Kurt, A., Prairie Du Sac, WI 53578 (US)
(74) Representative: Steinecke, Peter
(86) International application number: PCT/US2003/009325
(87) International publication number: WO 2005/007803

(56) References cited:
- US-A- 6 051 427
- US-A- 6 080 912
- US-B1- 6 238 858
- US-B1- 6 291 740
- US-B1- 6 730 297
- US-B2- 6 852 510
- DREXLER I ET AL: "Modified vaccinia virus Ankara for delivery of human tyrosinase as melanoma-associated antigen: Induction of tyrosinase- and melanoma-specific human leukocyte antigen A*0201-restricted cytotoxic T-cells in vitro and in vivo" CANCER RESEARCH, AMERICAN ASSOCIATION FOR CANCER RESEARCH, BALTIMORE, MD, US, vol. 59, 1 October 1999 (1999-10-01), pages 4955-4963, XP002185047 ISSN: 0008-5472
- BREMEL R D ET AL: "PRODUCTION OF HIGH EXPRESSING, STABLE MAMMALIAN CELL LINES USING GPEX TECHNOLOGY" AMERICAN CHEMICAL SOCIETY. ABSTRACTS OF PAPER. AT THE NATIONAL MEETING, AMERICAN CHEMICAL SOCIETY, WASHINGTON, DC, US, vol. 224, no. 1/2, 2002, page BIOT48, XP009065919 ISSN: 0065-7727
- MIZUARAI S. ET AL.: 'Production of transgenic quails with high frequency of germ-line transmission using VSV-G pseudotyped retroviral vector' BIOCHEM AND BIPHYS RES. COMMUNICATION vol. 286, 2001, pages 456 - 463, XP002973599
- LEE H. ET AL.: 'Efficient gene transfer of VSV-G pseudotyped retroviral vector to human brain tumor' GENE THER vol. 8, no. 4, February 2001, pages 268 - 273, XP003004429
- BARRETTE S. ET AL.: 'Lentivirus-based vectors transduce mouse hematopoietic stem cells with similar efficiency to moloney murine leukemia virus-based vectors' BLOOD vol. 96, no. 10, 2000, pages 3385 - 3391, XP003004430
- VAN HENNIK P.B. ET AL.: 'Highly efficient transduction of the green fluorescent protein gene in human umbilical cord blood stem cells capable of cobblestone formation in long-term cultures and multilineage engraftment of immunodeficient mice' BLOOD vol. 92, no. 11, 1998, pages 4013 - 4022, XP003004431

## Description

### FIELD OF THE INVENTION

The present invention relates to the production of proteins in host cells, and more particularly to the use of host cells containing multiple integrated copies of an integrating vector comprising an exogenous gene. The present application further discloses the use of integrating vectors lacking a selectable marker and growth of host cells containing such vectors in the absence of selection.

### BACKGROUND OF THE INVENTION

The pharmaceutical biotechnology industry is based on the production of recombinant proteins in mammalian cells. These proteins are essential to the therapeutic treatment of many diseases and conditions. In many cases, the market for these proteins exceeds a billion dollars a year. Examples of proteins produced recombinantly in mammalian cells include erythropoietin, factor VIII, factor IX, and insulin. For many of these proteins, expression in mammalian cells is preferred over expression in prokaryotic cells because of the need for correct post-translational modification (*e.g.,* glycosylation or silation; *see, e.g.,* U.S. Pat. No. 5,721,121).

Several methods are known for creating host cells that express recombinant proteins. In the most basic methods, a nucleic acid construct containing a gene encoding a heterologous protein and appropriate regulatory regions is introduced into the host cell and allowed to integrate. Methods of introduction include calcium phosphate precipitation, microinjection, lipofection, and electroporation. In other methods, a selection scheme is used to amplify the introduced nucleic acid construct. In these methods, the cells are co-transfected with a gene encoding an amplifiable selection marker and a gene encoding a heterologous protein (*See, e.g.,* Schroder and Fried1, Biotech. Bioeng. 53(6):547-59 [1997]). After selection of the initial tranformants, the transfected genes are amplified by the stepwise increase of the selective agent (*e.g*., dihydrofolate reductase) in the culture medium. In some cases, the exogenous gene may be amplified several hundred-fold by these procedures. Other methods of recombinant protein expression in mammalian cells utilize transfection with episomal vectors (*e.g*., plasmids).

Current methods for creating mammalian cell lines for expression of recombinant proteins suffer from several drawbacks. (*See, e.g.,* Mielke et al., Biochem. 35:2239-52 [1996]). Episomal systems allow for high expression levels of the recombinant protein, but are frequently only stable for a short time period (*See, e.g.,* Klehr and Bode, Mol. Genet. (Life Sci. Adv.) 7:47-52 [1988]). Mammalian cell lines containing integrated exogenous genes are somewhat more stable, but there is increasing evidence that stability depends on the presence of only a few copies or even a single copy of the exogenous gene.

Standard transfection techniques favor the introduction of multiple copies of the transgene into the genome of the host cell. Multiple integration of the transgene has, in many cases, proven to be intrinsically unstable. This intrinsic instability may be due to the characteristic head-to-tail mode of integration which promotes the loss of coding sequences by homologous recombination (*See, e.g.,* Weidle et al., Gene 66:193-203 [1988]) especially when the transgenes are transcribed (*See, e.g.,* McBurney et al., Somatic Cell Molec. Genet. 20:529-40 [1994]). Host cells also have epigenetic defense mechanisms directed against multiple copy integration events. In plants, this mechanism has been termed "cosuppression." (*See, e.g.,* Allen et al., Plant Cell 5:603-13 [1993]). Indeed, it is not uncommon that the level of expression is inversely related to copy number. These observations are consistent with findings that multiple copies of exogenous genes become inactivated by methylation (*See, e.g.,* Mehtali et al., Gene 91:179-84 [1990]) and subsequent mutagenesis (*See, e.g.,* Kricker et al., Proc. Natl. Acad. Sci. 89:1075-79 [1992]) or silenced by heterochromatin formation (*See, e.g.,* Dorer and Henikoff, Cell 77:993-1002 [1994]).

Accordingly, what is needed in the art are improved methods for making host cells that express recombinant proteins. Preferably, the host cells will be stable over extended periods of time and express the protein encoded by a transgene at high levels.

### SUMMARY OF THE INVENTION

The present invention relates to the production of proteins in host cells, and more particularly to the use of host cells containing multiple integrated copies of an integrating vector comprising an exogenous gene. The present application further discloses the use of integrating vectors lacking a selectable marker and growth of host cells containing such vectors in the absence of selection.

For example, in some embodiments, the present invention relates to the use of a stable host cell for production and purification of a protein of interest, said host cell comprising a genome, the genome comprising at least ten integrated pseudotyped retroviral vectors wherein the integrating vector comprises at least one exogenous gene encoding said protein of interest operably linked to a promoter, and wherein the pseudotyped retroviral vectors lacks a gene encoding a selectable marker. In some embodiments, the integrating vector further comprises a secretion signal sequence operably linked to the exogenous gene. In some embodiments, the integrating vector further comprises an RNA stabilizing element operably linked to the exogenous gene. In certain embodiments, the pseudotyped retroviral vector comprises a G glycoprotein selected from the group including, but not limited to, vesicular stomatitis virus, Piry virus, Chandipura virus, Spring viremia of carp virus and Mokola virus G glycoproteins. In further embodiments, the retroviral vector comprises long terminal repeats selected from the group including, but not limited to, MoMLV, MoMuSV, and MMTV long terminal repeats. In some embodiments, the host cell is clonally derived. In other embodiments, the host cell is non-clonally derived. In some preferred embodiments, the genome is stable for greater than 10 passages, and preferably, stable for greater than 100 passages. In certain particularly preferred embodiments, the integrated exogenous gene is stable in the absence of selection. In some embodiments, the at least one exogenous gene is selected from the group consisting of genes encoding antigen binding proteins, pharmaceutical proteins, kinases, phosphatases, nucleic acid binding proteins, membrane receptor proteins, signal transduction proteins, ion channel proteins, and oncoproteins. In some embodiments, the genome comprises at least 5, and preferably, at least 100 integrated integrating vectors. In some preferred embodiments, the host cell expresses greater than about 3, and preferabley, greater than about 10 picograms of the exogenous protein per day.

The present application also discloses a method for transfecting host cells comprising providing a plurality of host cells comprising a genome, and a plurality of integrating vectors, wherein the integrating vectors comprise at least one exogenous gene, and wherein the integrating vectors lack a gene encoding a selectable marker; contacting the host cell with the plurality of integrating vectors to generate transfected host cells comprising at least one integrated copy of the integrating vector; and clonally selecting the transfected host cells. In some preferred embodiments, the integrated exogenous gene is stable in the absence of selection. In some embodiments, the host is contacted with the integrating vectors at a multiplicity of infection of greater than 10. In some embodiments, the host cells are contacted with the plurality of integrating vectors under conditions such that at least 2, preferably 5, and even more preferably 10, integrating vectors integrate into the genome of the host cell. In some embodiments, the clonally selecting comprises detecting nucleic acid of the exogenous gene. In some embodiments, detecting nucleic acid of the exogenous gene comprises a detection assay selected from the group consisting of a PCR assay and a hybridization assay. In other embodiments, the clonally selecting comprises detecting protein expressed by the exogenous gene. In some embodiments, detecting protein expressed by the exogenous gene comprises a detection assay selected from the group consisting of an immunoassay and a biochemical assay. In some embodiments, the immunoassay is selected from the group consisting of ELISA and Western blot. In some embodiments, the integrating vector is a retroviral vector. In some preferred embodiments, the host cells synthesize greater than about 1, preferably greater than about 10, and even more preferably, greater than about 50 picograms per cell per day of protein from the exogenous gene of interest.

The present application further discloses a method of producing a protein of interest comprising providing a host cell comprising a genome, the genome comprising at least one integrated copy of at least one integrating vector comprising an exogenous gene operably linked to a promoter, wherein the integrating vector lacks a gene encoding a selectable marker, and wherein the exogenous gene encodes a protein of interest, and culturing the host cells under conditions such that the protein of interest is produced. In some preferred embodiments, the integrated exogenous gene is stable in the absence of selection. In some embodiments, the integrating vector further comprises a secretion signal sequence operably linked to the exogenous gene. In some embodiments, the method further comprises the step of isolating the protein of interest. In some embodiments, the method further comprises the step of clonally selecting at least 10 colonies. In some embodiments, the clonally selecting comprising detecting the protein expressed by the exogenous gene. In some embodiments, detecting protein expressed by the exogenous gene comprises a detection assay selected from the group consisting of an immunoassay and a biochemical assay. In some embodiments, the immunoassay is selected from the group consisting of ELISA and Western blot. In some embodiments, the genome of the host cell comprises greater than 5, and preferably greater than 10, integrated copies of the integrating vector. In some embodiments, the integrating vector is a retroviral vector. In some embodiments, the host cells synthesize greater than about 1, preferably greater than 10, and even more preferably, greater than 50 picograms per cell per day of the protein of interest.

The present invention also provides a retroviral vector comprising a gene construct comprising an exogenous promoter operably linked to an exogenous gene, wherein the vector lacks a gene encoding a selectable marker. In accordance with the present invention, the retroviral vector is a pseudotyped retroviral vector. In some embodiments, the pseudotyped retroviral vector comprises a G glycoprotein selected from the group including, but not limited to, vesicular stomatitis virus, Piry virus, Chandipura virus, Spring viremia of carp virus and Mokola virus G glycoproteins. In some embodiments, the retroviral vector comprises long terminal repeats selected from the group including, but not limited to, MoMLV, MoMuSV, and MMTV long terminal repeats.

### DESCRIPTION OF THE FIGURES

Figure 1 is a western blot of a 15% SDS-PAGE gel run under denaturing conditions and probed with anti-human IgG (Fc) and anti-human IgG (Kappa).
Figure 2 is a graph of MN14 expression over time.
Figure 3 is a Western blot of a 15% PAGE run under non-denaturing conditions and probed with anti-human IgG (Fc) and anti-human IgG (Kappa).

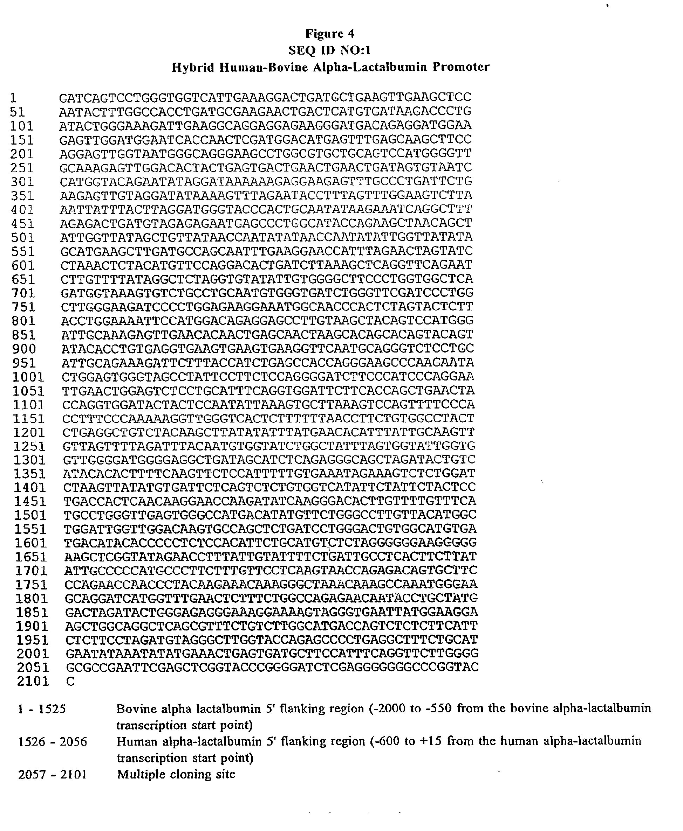

Figure 4 provides the sequence for the hybrid human-bovine alpha-lactalbumin promoter (SEQ ID NO:1).

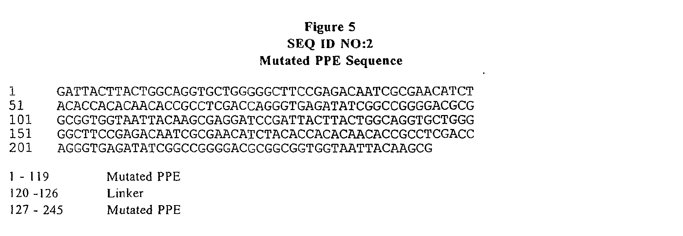

Figure 5 provides the sequence for the mutated PPE sequence (SEQ ID NO:2).

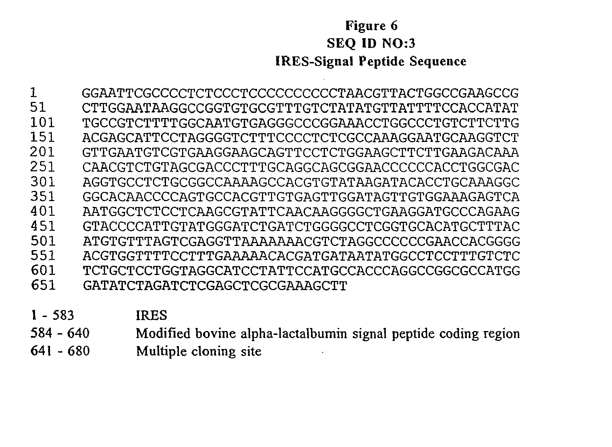

Figure 6 provides the sequence for the IRES-Signal peptide sequence (SEQ ID NO:3).

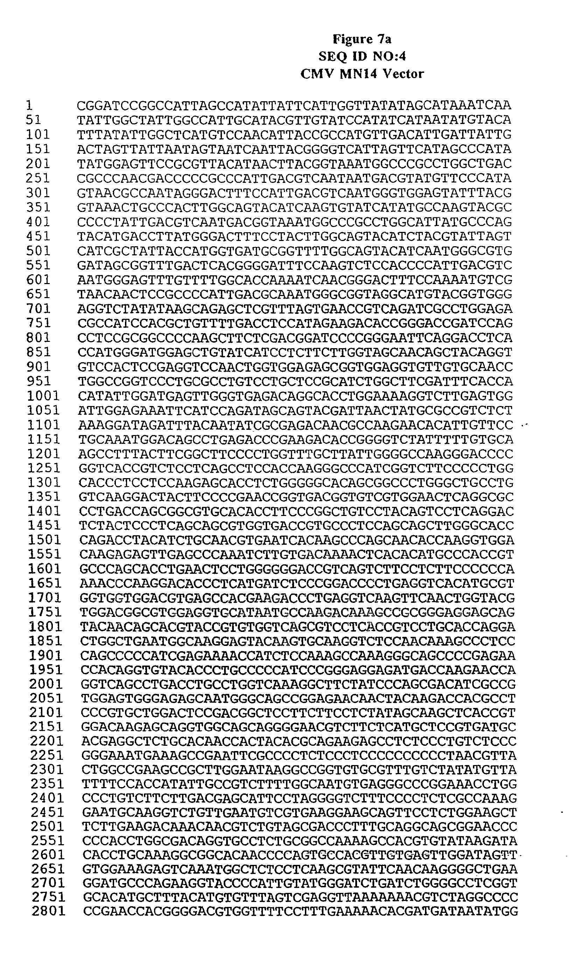

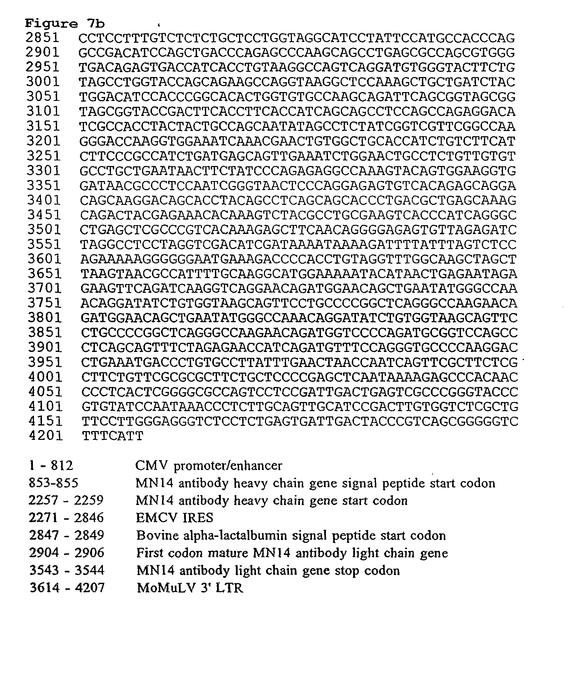

Figures 7a and 7b provide the sequence for CMV MN14 vector (SEQ ID NO:4).

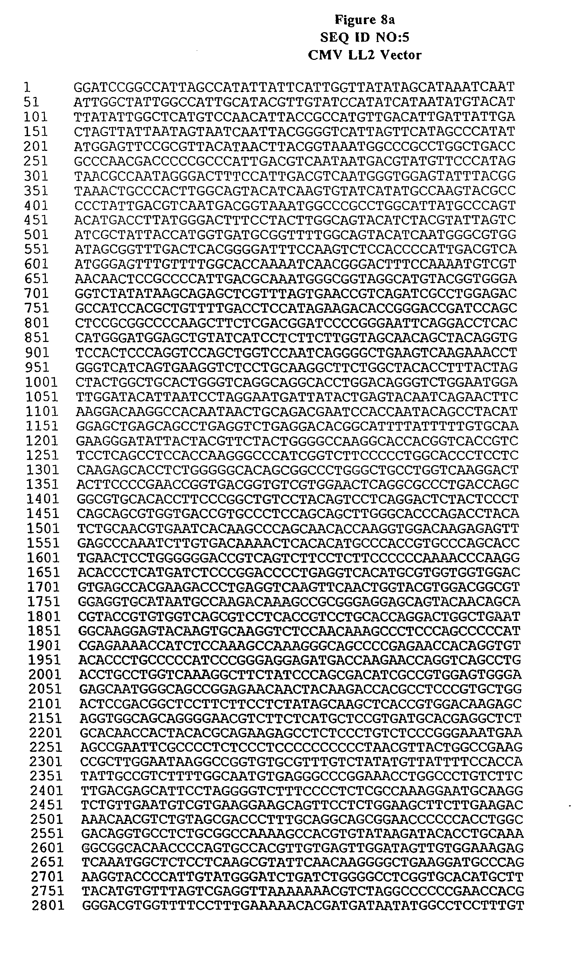

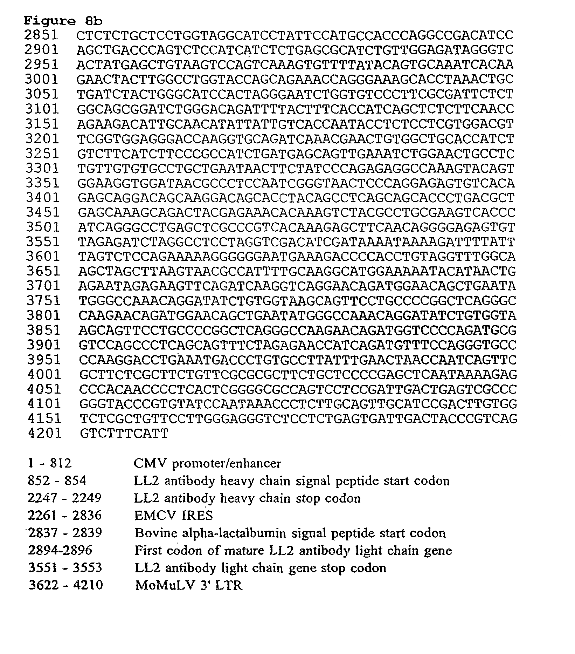

Figures 8a and 8b provide the sequence for the CMV LL2 vector (SEQ ID NO:5).

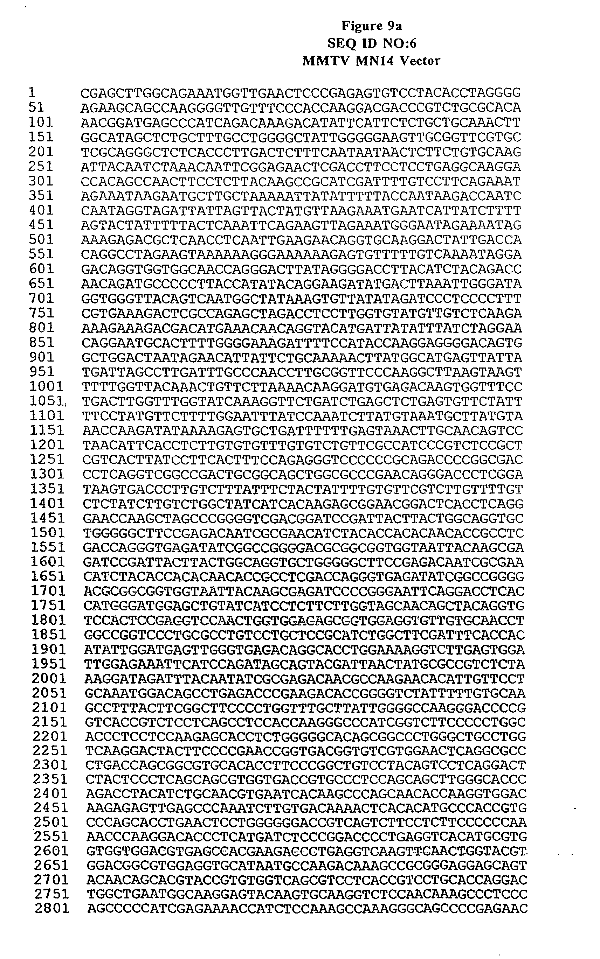

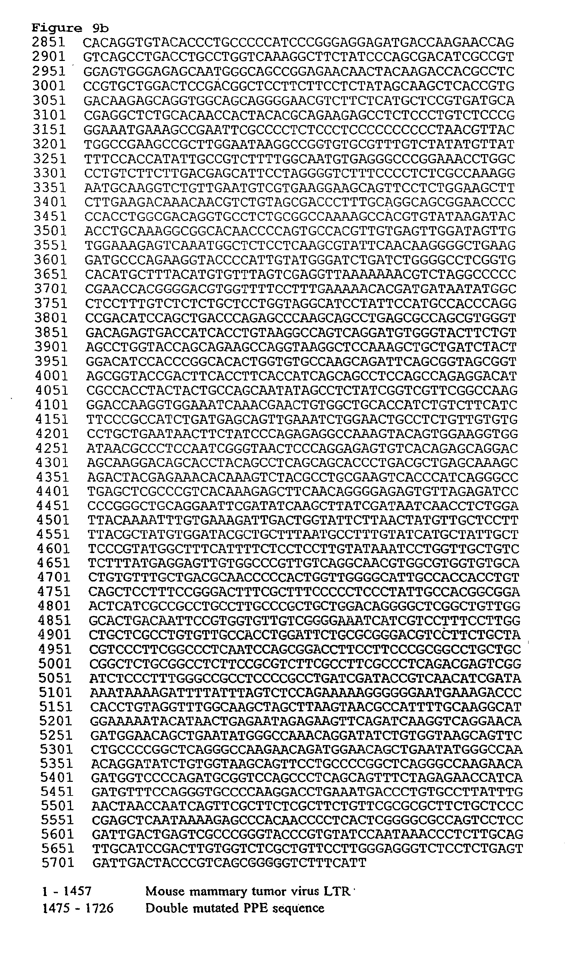

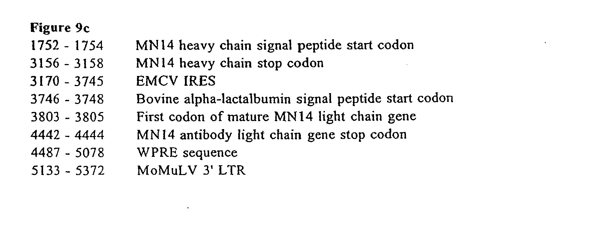

Figures 9a-c provide the sequence for the MMTV MN14 vector (SEQ ID NO:6).

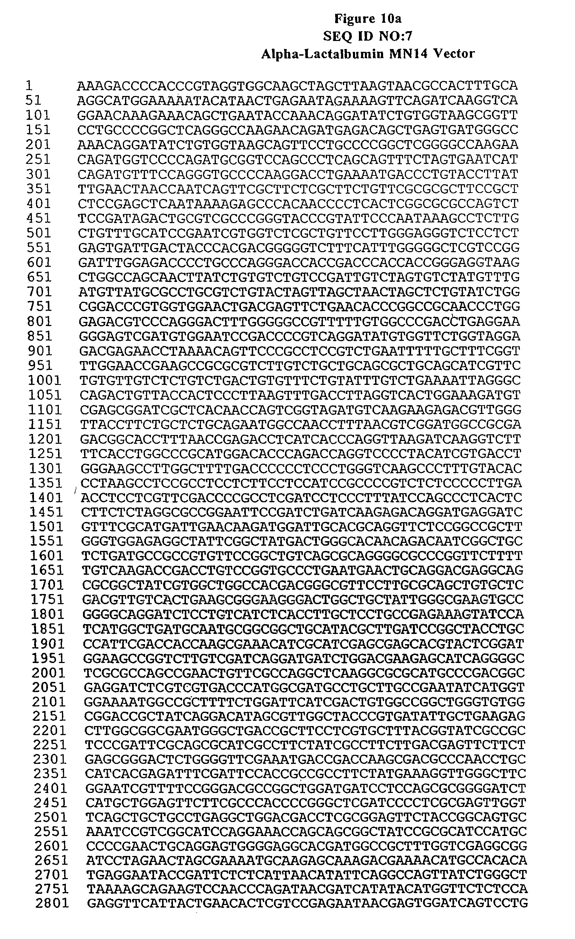

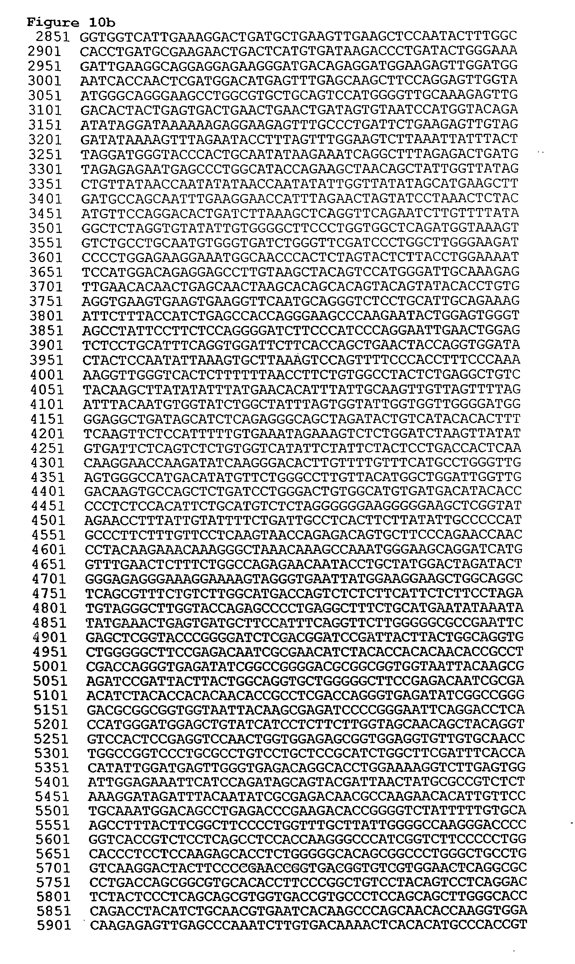

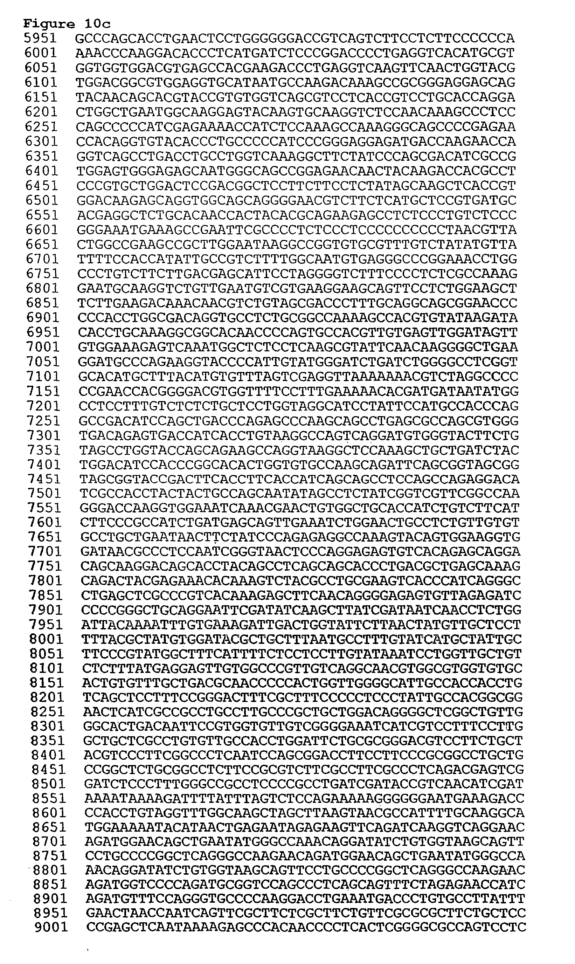

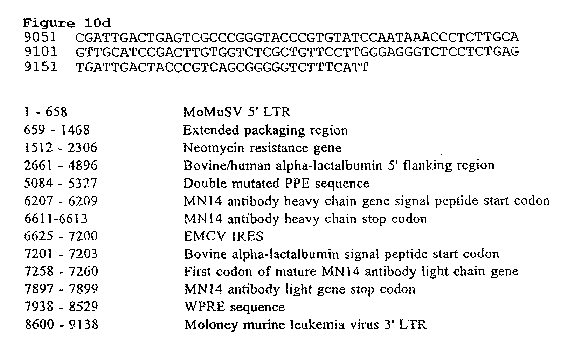

Figures 10a-d provide the sequence for the alpha-lactalbumin MN14 Vector (SEQ ID NO:7).

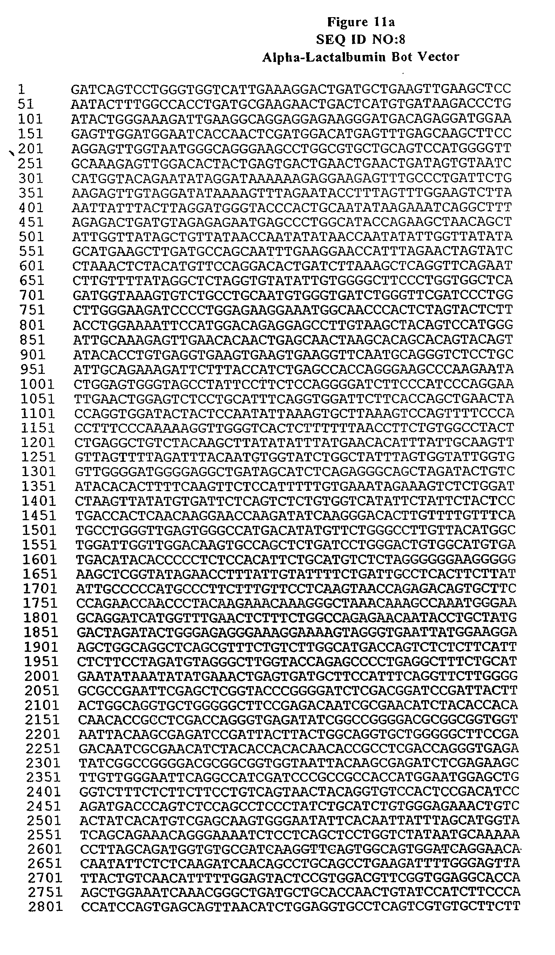

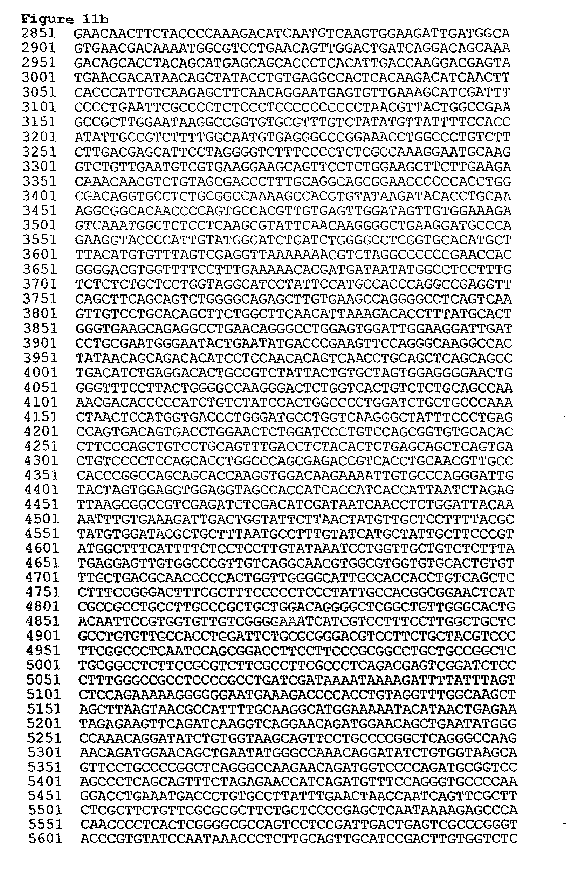

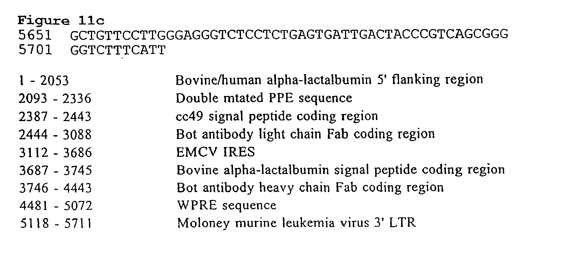

Figures 11a-c provide the sequence for the alpha-lactalbumin Bot vector (SEQ ID NO:8).

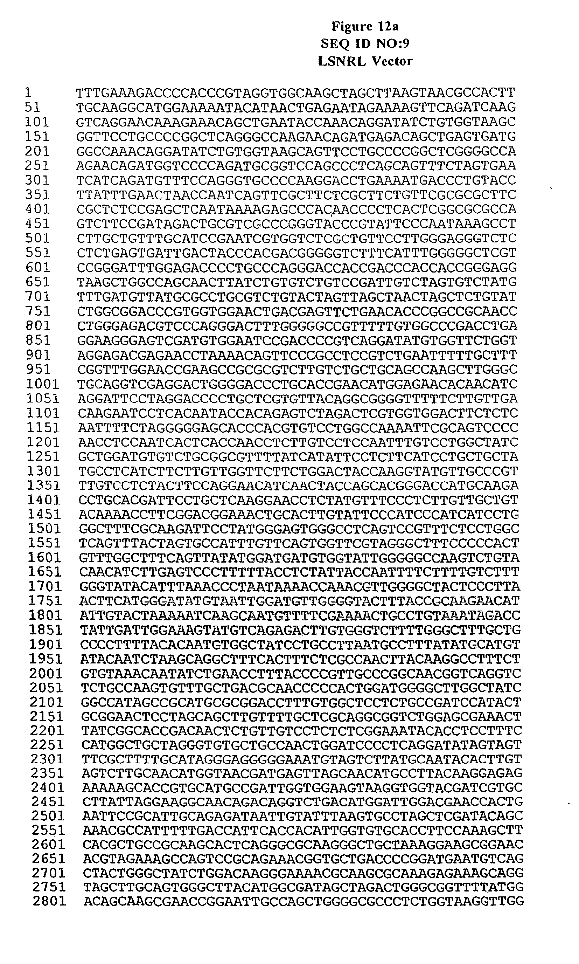

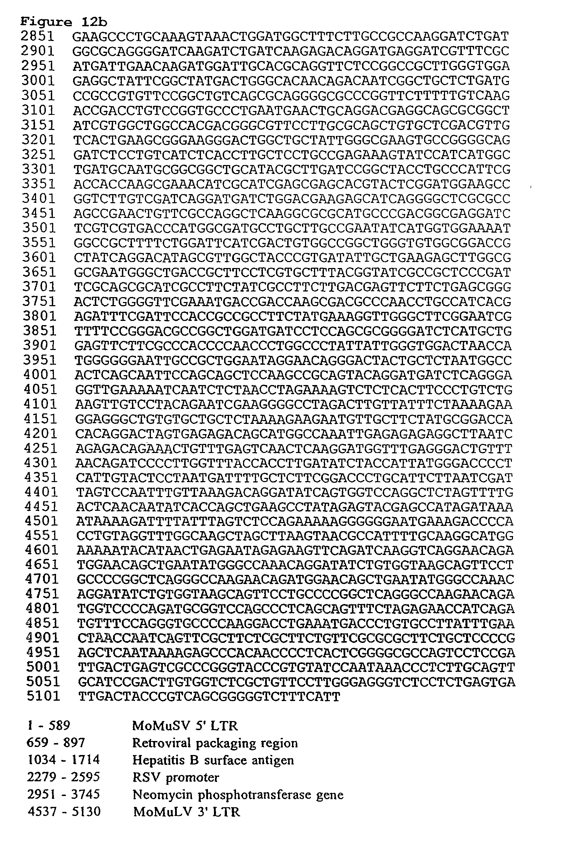

Figures 12a-b provide the sequence for the LSRNL vector (SEQ ID NO:9).

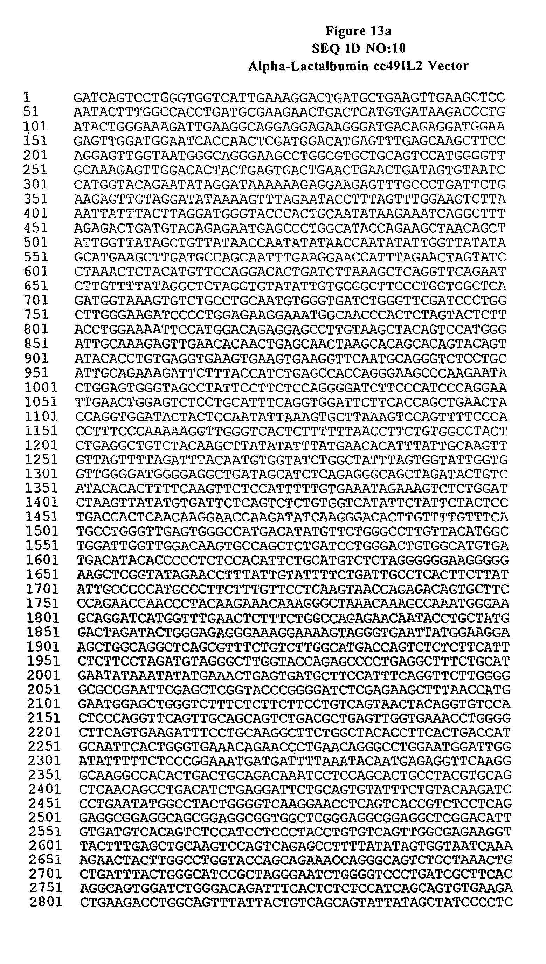

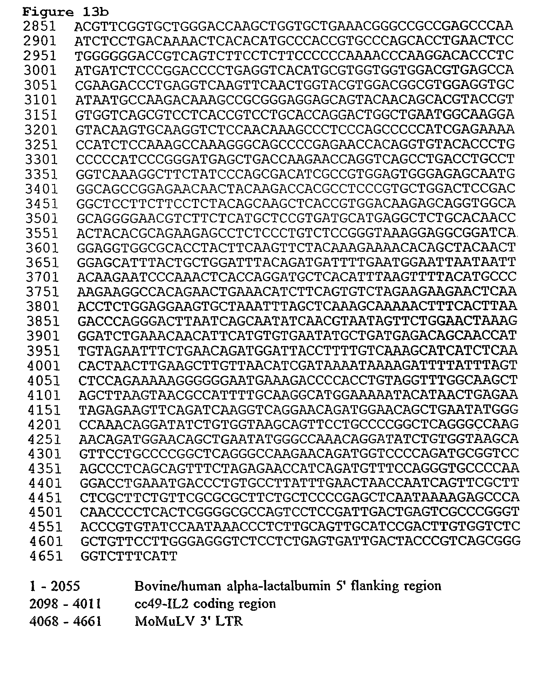

Figures 13a-b provide the sequence for the alpha-lactalbumin cc49IL2 vector (SEQ ID NO: 10).

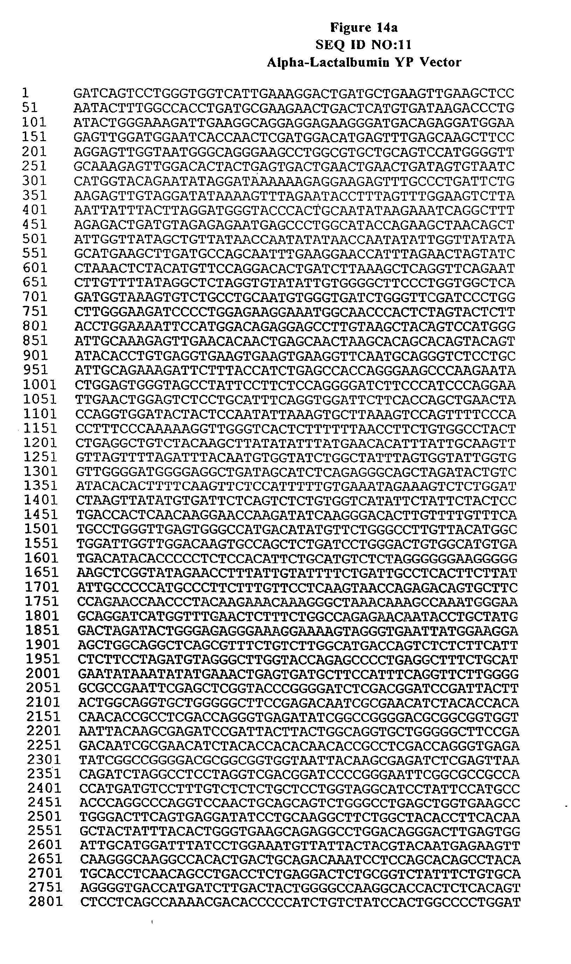

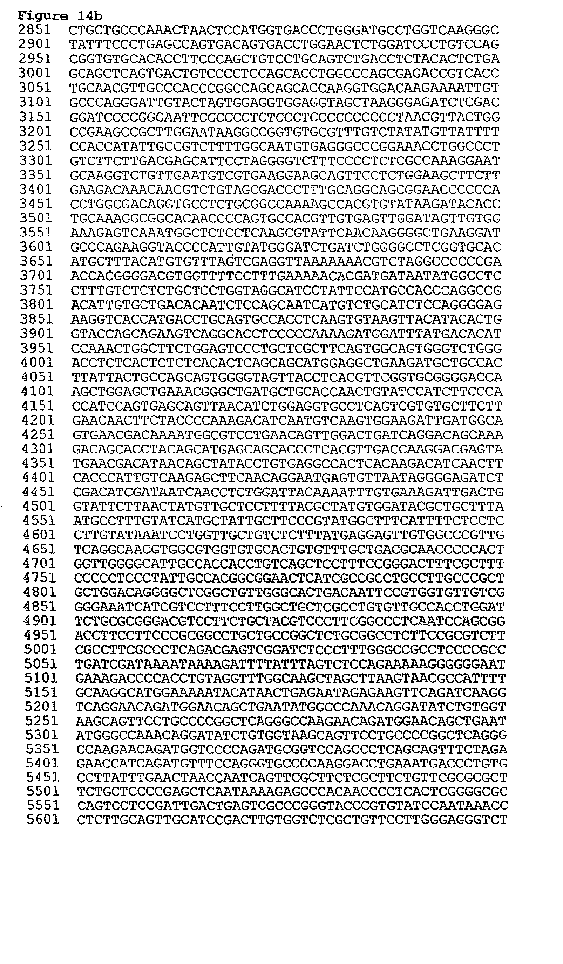

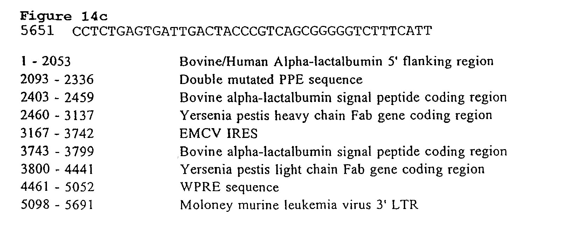

Figures 14a-c provides the sequence for the alpha-lactalbumin YP vector (SEQ ID NO:11).

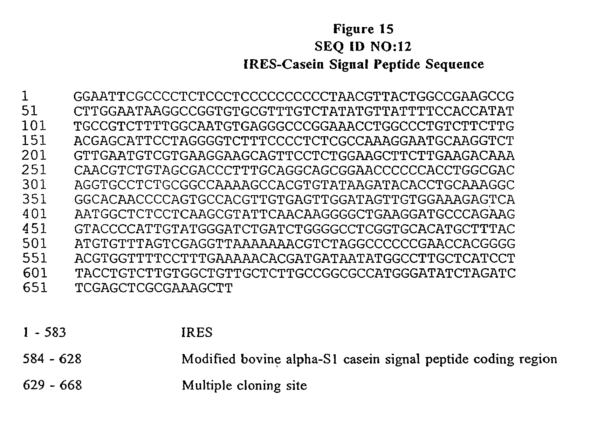

Figure 15 provides the sequence for the IRES-Casein signal peptide sequence (SEQ ID NO: 12).

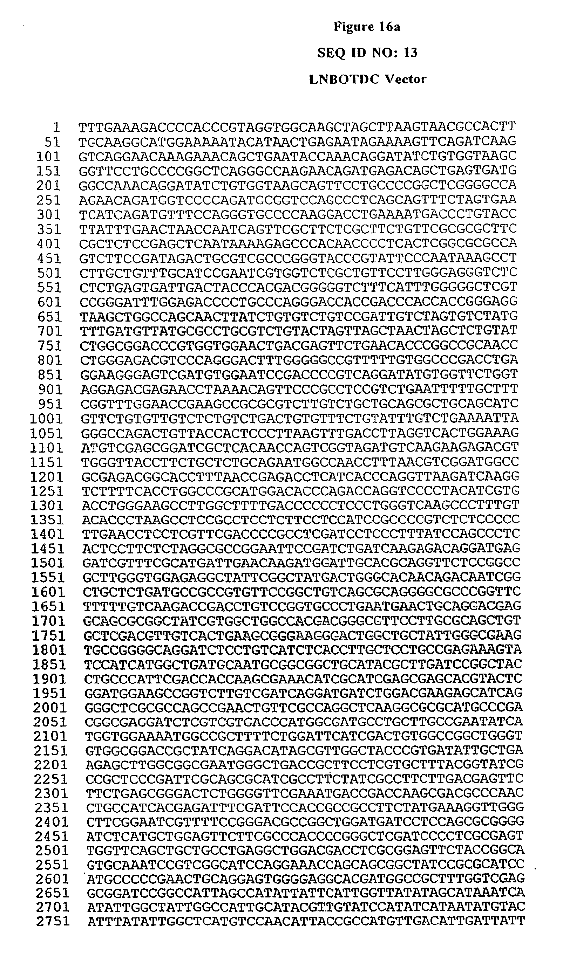

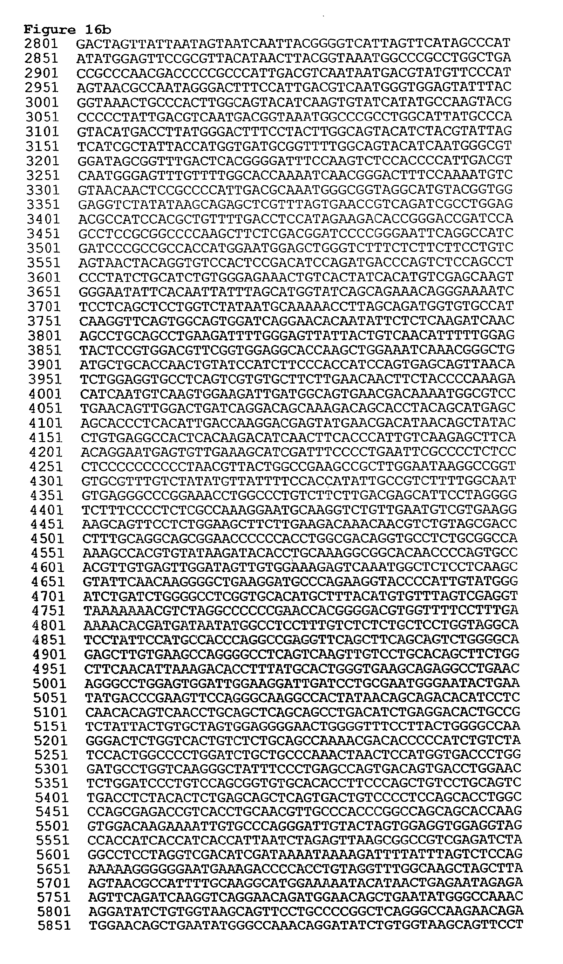

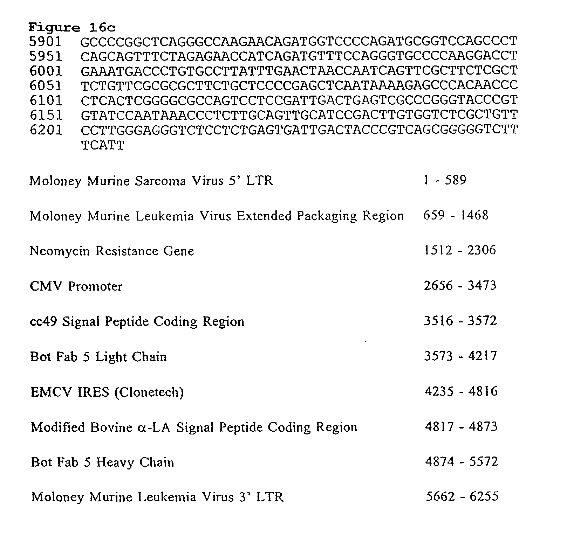

Figures 16a-c provide the sequence for the LNBOTDC vector (SEQ ID NO: 13).
Figure 17 provides a graph depicting the INVADER Assay gene ratio in CMV promoter cell lines.
Figure 18 provides a graph depicting the INVADER Assay gene ratio in α-lactalbumin promotor cell lines.
Figures 19a-d provides the sequence of a retroviral vector that expresses a G-Protein coupled receptor and antibody light chain.
Figure 20 shows a graph demonstrating increased expression of a gene of interest in the absence of a selectable marker.

### DEFINITIONS

To facilitate understanding of the invention, a number of terms are defined below.

As used herein, the term "host cell" refers to any eukaryotic cell (e.g., mammalian cells, avian cells, amphibian cells, plant cells, fish cells, and insect cells), whether located in vitro or in vivo.

As used herein, the term "cell culture" refers to any in vitro culture of cells. Included within this term are continuous cell lines (e.g., with an immortal phenotype), primary cell cultures, finite cell lines (*e.g.,* non-transformed cells), and any other cell population maintained in vitro, including oocytes and embryos.

As used herein, the term "vector" refers to any genetic element, such as a plasmid, phage, transposon, cosmid, chromosome, virus, virion, etc., which is capable of replication when associated with the proper control elements and which can transfer gene sequences between cells. Thus, the term includes cloning and expression vehicles, as well as viral vectors.

As used herein, the term "integrating vector" refers to a vector whose integration or insertion into a nucleic acid *(e.g.,* a chromosome) is accomplished via an integrase. Examples of "integrating vectors" include, but are not limited to, retroviral vectors, transposons, and adeno associated virus vectors.

As used herein, the term "integrated" refers to a vector that is stably inserted into the genome (*i.e.,* into a chromosome) of a host cell.

As used herein, the term "multiplicity of infection" or "MOI" refers to the ratio of integrating vectors:host cells used during transfection or transduction of host cells. For example, if 1,000,000 vectors are used to transduce 100,000 host cells, the multiplicity of infection is 10. The use of this term is not limited to events involving transduction, but instead encompasses introduction of a vector into a host by methods such as lipofection, microinjection, calcium phosphate precipitation, and electroporation.

As used herein, the term "genome" refers to the genetic material (*e.g.,* chomosomes) of an organism.

The term "nucleotide sequence of interest" refers to any nucleotide sequence (*e.g.,* RNA or DNA), the manipulation of which may be deemed desirable for any reason (*e.g.,* treat disease, confer improved qualities, expression of a protein of interest in a host cell, expression of a ribozyme, etc.), by one of ordinary skill in the art. Such nucleotide sequences include, but are not limited to, coding sequences of structural genes *(e.g.,* reporter genes, selection marker genes, oncogenes, drug resistance genes, growth factors, etc.), and non-coding regulatory sequences which do not encode an mRNA or protein product (*e.g.,* promoter sequence, polyadenylation sequence, termination sequence, enhancer sequence, etc.).

As used herein, the term "protein of interest" refers to a protein encoded by a nucleic acid of interest.

As used herein, the term "signal protein" refers to a protein that is co-expressed with a protein of interest and which, when detected by a suitable assay, provides indirect evidence of expression of the protein of interest. Examples of signal protein useful in the present invention include, but are not limited to, immunoglobulin heavy and light chains, beta-galactosidase, beta-lactamase, green fluorescent protein, and luciferase.

As used herein, the term "exogenous gene" refers to a gene that is not naturally present in a host organism or cell, or is artificially introduced into a host organism or cell.

The term "gene" refers to a nucleic acid (*e.g.,* DNA or RNA) sequence that comprises coding sequences necessary for the production of a polypeptide or precursor (*e.g.,* proinsulin). The polypeptide can be encoded by a full length coding sequence or by any portion of the coding sequence so long as the desired activity or functional properties (*e.g.,* enzymatic activity, ligand binding, signal transduction, etc.) of the full-length or fragment are retained. The term also encompasses the coding region of a structural gene and includes sequences located adjacent to the coding region on both the 5' and 3' ends for a distance of about 1 kb or more on either end such that the gene corresponds to the length of the full-length mRNA. The sequences that are located 5' of the coding region and which are present on the mRNA are referred to as 5' untranslated sequences. The sequences that are located 3' or downstream of the coding region and which are present on the mRNA are referred to as 3' untranslated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "gene expression" refers to the process of converting genetic information encoded in a gene into RNA (*e.g.,* mRNA, rRNA, tRNA, or snRNA) through "transcription" of the gene (*i.e*., via the enzymatic action of an RNA polymerase), and for protein encoding genes, into protein through "translation" of mRNA. Gene expression can be regulated at many stages in the process. "Up-regulation" or "activation" refers to regulation that increases the production of gene expression products (*i.e.,* RNA or protein), while "down-regulation" or "repression" refers to regulation that decrease production. Molecules (*e.g.,* transcription factors) that are involved in up-regulation or down-regulation are often called "activators" and "repressors," respectively.

Where "amino acid sequence" is recited herein to refer to an amino acid sequence of a naturally occurring protein molecule, "amino acid sequence" and like terms, such as "polypeptide" or "protein" are not meant to limit the amino acid sequence to the complete, native amino acid sequence associated with the recited protein molecule.

As used herein, the terms "nucleic acid molecule encoding," "DNA sequence encoding," "DNA encoding," "RNA sequence encoding," and "RNA encoding" refer to the order or sequence of deoxyribonucleotides or ribonucleotides along a strand of deoxyribonucleic acid or ribonucleic acid. The order of these deoxyribonucleotides or ribonucleotides determines the order of amino acids along the polypeptide (protein) chain. The DNA or RNA sequence thus codes for the amino acid sequence.

As used herein, the term "variant," when used in reference to proteins, refers to proteins encoded by partially homologous nucleic acids so that the amino acid sequence of the proteins varies. As used herein, the term "variant" encompasses proteins encoded by homologous genes having both conservative and nonconservative amino acid substitutions that do not result in a change in protein function, as well as proteins encoded by homologous genes having amino acid substitutions that cause decreased (*e.g.,* null mutations) protein function or increased protein function.

As used herein, the terms "complementary" or "complementarity" are used in reference to polynucleotides (*i.e.,* a sequence of nucleotides) related by the base-pairing rules. For example, the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The terms "homology" and "percent identity" when used in relation to nucleic acids refers to a degree of complementarity. There may be partial homology (*i.e.,* partial identity) or complete homology (*i.e.,* complete identity). A partially complementary sequence is one that at least partially inhibits a completely complementary sequence from hybridizing to a target nucleic acid sequence and is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe (*i.e.,* an oligonucleotide which is capable of hybridizing to another oligonucleotide of interest) will compete for and inhibit the binding *(i.e.,* the hybridization) of a completely homologous sequence to a target sequence under conditions of low stringency. This is not to say that conditions of low stringency are such that non-specific binding is permitted; low stringency conditions require that the binding of two sequences to one another be a specific (*i.e.,* selective) interaction. The absence of non-specific binding may be tested by the use of a second target which lacks even a partial degree of complementarity (*e.g.,* less than about 30% identity); in the absence of non-specific binding the probe will not hybridize to the second non-complementary target.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (*e.g.,* the presence or absence of formamide, dextran sulfate, polyethylene glycol) are considered and the hybridization solution maybe varied to generate conditions of low stringency hybridization different from, but equivalent to, the above listed conditions. In addition, the art knows conditions that promote hybridization under conditions of high stringency (*e.g.,* increasing the temperature of the hybridization and/or wash steps, the use of formamide in the hybridization solution, etc.).

When used in reference to a double-stranded nucleic acid sequence such as a cDNA or genomic clone, the term "substantially homologous" refers to any probe that can hybridize to either or both strands of the double-stranded nucleic acid sequence under conditions of low stringency as described above.

When used in reference to a single-stranded nucleic acid sequence, the term "substantially homologous" refers to any probe that can hybridize (*i.e.,* it is the complement of) the single-stranded nucleic acid sequence under conditions of low stringency as described above.

As used herein, the term "hybridization" is used in reference to the pairing of complementary nucleic acids. Hybridization and the strength of hybridization (*i.e.,* the strength of the association between the nucleic acids) is impacted by such factors as the degree of complementary between the nucleic acids, stringency of the conditions involved, the Tₘ of the formed hybrid, and the G:C ratio within the nucleic acids. A single molecule that contains pairing of complementary nucleic acids within its structure is said to be "self-hybridized."

As used herein, the term "Tₘ" is used in reference to the "melting temperature" of a nucleic acid. The melting temperature is the temperature at which a population of double-stranded nucleic acid molecules becomes half dissociated into single strands. The equation for calculating the Tₘ of nucleic acids is well known in the art. As indicated by standard references, a simple estimate of the Tₘ value may be calculated by the equation: Tₘ = 81.5 + 0.41(% G + C), when a nucleic acid is in aqueous solution at 1 M NaCl (See *e.g.,* Anderson and Young, Quantitative Filter Hybridization, in Nucleic Acid Hybridization [1985]). Other references include more sophisticated computations that take structural as well as sequence characteristics into account for the calculation of Tₘ.

As used herein the term "stringency" is used in reference to the conditions of temperature, ionic strength, and the presence of other compounds such as organic solvents, under which nucleic acid hybridizations are conducted. With "high stringency" conditions, nucleic acid base pairing will occur only between nucleic acid fragments that have a high frequency of complementary base sequences. Thus, conditions of "weak" or "low" stringency are often required with nucleic acids that are derived from organisms that are genetically diverse, as the frequency of complementary sequences is usually less.

"High stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄•H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 0.1X SSPE, 1.0% SDS at 42°C when a probe of about 500 nucleotides in length is employed.

"Medium stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄•H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 1.0X SSPE, 1.0% SDS at 42°C when a probe of about 500 nucleotides in length is employed.

"Low stringency conditions" comprise conditions equivalent to binding or hybridization at 42°C in a solution consisting of 5X SSPE (43.8 g/l NaCl, 6.9 g/l NaH₂PO₄•H₂O and 1.85 g/l EDTA, pH adjusted to 7.4 with NaOH), 0.1% SDS, 5X Denhardt's reagent [50X Denhardt's contains per 500 ml: 5 g Ficoll (Type 400, Pharamcia), 5 g BSA (Fraction V; Sigma)] and 100 µg/ml denatured salmon sperm DNA followed by washing in a solution comprising 5X SSPE, 0.1% SDS at 42°C when a probe of about 500 nucleotides in length is employed.

A gene may produce multiple RNA species that are generated by differential splicing of the primary RNA transcript. cDNAs that are splice variants of the same gene will contain regions of sequence identity or complete homology (representing the presence of the same exon or portion of the same exon on both cDNAs) and regions of complete non-identity (for example, representing the presence of exon "A" on cDNA 1 wherein cDNA 2 contains exon "B" instead). Because the two cDNAs contain regions of sequence identity they will both hybridize to a probe derived from the entire gene or portions of the gene containing sequences found on both cDNAs; the two splice variants are therefore substantially homologous to such a probe and to each other.

The terms "in operable combination," "in operable order," and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

As used herein, the term "selectable marker" refers to a gene that encodes an enzymatic activity that confers the ability to grow in medium lacking what would otherwise be an essential nutrient (*e.g.* the HIS3 gene in yeast cells); in addition, a selectable marker may confer resistance to an antibiotic or drug upon the cell in which the selectable marker is expressed. Selectable markers may be "dominant"; a dominant selectable marker encodes an enzymatic activity that can be detected in any eukaryotic cell line. Examples of dominant selectable markers include the bacterial aminoglycoside 3' phosphotransferase gene (also referred to as the neo gene) that confers resistance to the drug G418 in mammalian cells, the bacterial hygromycin G phosphotransferase (hyg) gene that confers resistance to the antibiotic hygromycin and the bacterial xanthine-guanine phosphoribosyl transferase gene (also referred to as the gpt gene) that confers the ability to grow in the presence of mycophenolic acid. Other selectable markers are not dominant in that their use must be in conjunction with a cell line that lacks the relevant enzyme activity. Examples of non-dominant selectable markers include the thymidine kinase (tk) gene that is used in conjunction with tk- cell lines, the CAD gene, which is used in conjunction with CAD-deficient cells, and the mammalian hypoxanthine-guanine phosphoribosyl transferase (hprt) gene, which is used in conjunction with hprt- cell lines. A review of the use of selectable markers in mammalian cell lines is provided in Sambrook, J. et al., Molecular Cloning: A Laboratory Manual, 2nd ed., Cold Spring Harbor Laboratory Press, New York (1989) pp.16.9-16.15.

As used herein, the term "lacking a selectable marker" as in integrating vectors that lack a gene encoding a selectable marker refers to integrating vectors that do not contain a gene encoding a selectable marker.

As used herein, the term "selection free growth" refers to growth in the absence of selective conditions required for a given selectable marker (*e.g.,* antibiotics or the deficiency of a nutrient of enzymatic activity). In some preferred embodiments, host cells comprising integrating vectors that "lack a selectable marker" are also subjected to selection free growth.

As used herein, the term "regulatory element" refers to a genetic element that controls some aspect of the expression of nucleic acid sequences. For example, a promoter is a regulatory element that facilitates the initiation of transcription of an operably linked coding region. Other regulatory elements are splicing signals, polyadenylation signals, termination signals, RNA export elements, internal ribosome entry sites, etc. (defined infra).

Transcriptional control signals in eukaryotes comprise "promoter" and "enhancer" elements. Promoters and enhancers consist of short arrays of DNA sequences that interact specifically with cellular proteins involved in transcription (Maniatis et al., Science 236:1237 [1987]). Promoter and enhancer elements have been isolated from a variety of eukaryotic sources including genes in yeast, insect and mammalian cells, and viruses (analogous control elements, *i.e.,* promoters, are also found in prokaryotes). The selection of a particular promoter and enhancer depends on what cell type is to be used to express the protein of interest. Some eukaryotic promoters and enhancers have a broad host range while others are functional in a limited subset of cell types (for review see, Voss et al., Trends Biochem. Sci., 11:287 [1986]; and Maniatis *et al.,* supra). For example, the SV40 early gene enhancer is very active in a wide variety of cell types from many mammalian species and has been widely used for the expression of proteins in mammalian cells (Dijkema et al., EMBO J. 4:761 [1985]). Two other examples of promoter/enhancer elements active in a broad range of mammalian cell types are those from the human elongation factor 1α gene (Uetsuki et al., J. Biol. Chem., 264:5791 [1989]; Kim et al., Gene 91:217 [1990]; and Mizushima and Nagata, Nuc. Acids. Res., 18:5322 [1990]) and the long terminal repeats of the Rous sarcoma virus (German et al., Proc. Natl. Acad. Sci. USA 79:6777 [1982]) and the human cytomegalovirus (Boshart et al., Cell 41:521 [1985]).

As used herein, the term "promoter/enhancer" denotes a segment of DNA which contains sequences capable of providing both promoter and enhancer functions (*i.e.,* the functions provided by a promoter element and an enhancer element, see above for a discussion of these functions). For example, the long terminal repeats of retroviruses contain both promoter and enhancer functions. The enhancer/promoter may be "endogenous" or "exogenous" or "heterologous." An "endogenous" enhancer/promoter is one that is naturally linked with a given gene in the genome. An "exogenous" or "heterologous" enhancer/promoter is one that is placed in juxtaposition to a gene by means of genetic manipulation (*i.e.,* molecular biological techniques such as cloning and recombination) such that transcription of that gene is directed by the linked enhancer/promoter.

Regulatory elements may be tissue specific or cell specific. The term "tissue specific" as it applies to a regulatory element refers to a regulatory element that is capable of directing selective expression of a nucleotide sequence of interest to a specific type of tissue (*e.g.,* liver) in the relative absence of expression of the same nucleotide sequence of interest in a different type of tissue (*e.g.,* lung).

Tissue specificity of a regulatory element may be evaluated by, for example, operably linking a reporter gene to a promoter sequence (which is not tissue-specific) and to the regulatory element to generate a reporter construct, introducing the reporter construct into the genome of an animal such that the reporter construct is integrated into every tissue of the resulting transgenic animal, and detecting the expression of the reporter gene (*e.g.,* detecting mRNA, protein, or the activity of a protein encoded by the reporter gene) in different tissues of the transgenic animal. The detection of a greater level of expression of the reporter gene in one or more tissues relative to the level of expression of the reporter gene in other tissues shows that the regulatory element is "specific" for the tissues in which greater levels of expression are detected. Thus, the term "tissue-specific" (*e.g.,* liver-specific) as used herein is a relative term that does not require absolute specificity of expression. In other words, the term "tissue-specific" does not require that one tissue have extremely high levels of expression and another tissue have no expression. It is sufficient that expression is greater in one tissue than another. By contrast, "strict" or "absolute" tissue-specific expression is meant to indicate expression in a single tissue type (*e.g.,* liver) with no detectable expression in other tissues.

The term "cell type specific" as applied to a regulatory element refers to a regulatory element that is capable of directing selective expression of a nucleotide sequence of interest in a specific type of cell in the relative absence of expression of the same nucleotide sequence of interest in a different type of cell within the same tissue. The term "cell type specific" when applied to a regulatory element also means a regulatory element capable of promoting selective expression of a nucleotide sequence of interest in a region within a single tissue.

Cell type specificity of a regulatory element may be assessed using methods well known in the art (*e.g.,* immunohistochemical staining and/or Northern blot analysis). Briefly, for immunohistochemical staining, tissue sections are embedded in paraffin, and paraffin sections are reacted with a primary antibody specific for the polypeptide product encoded by the nucleotide sequence of interest whose expression is regulated by the regulatory element. A labeled (*e.g.,* peroxidase conjugated) secondary antibody specific for the primary antibody is allowed to bind to the sectioned tissue and specific binding detected (*e.g.,* with avidin/biotin) by microscopy. Briefly, for Northern blot analysis, RNA is isolated from cells and electrophoresed on agarose gels to fractionate the RNA according to size followed by transfer of the RNA from the gel to a solid support (*e.g.,* nitrocellulose or a nylon membrane). The immobilized RNA is then probed with a labeled oligo-deoxyribonucleotide probe or DNA probe to detect RNA species complementary to the probe used. Northern blots are a standard tool of molecular biologists.

The term "promoter," "promoter element," or "promoter sequence" as used herein, refers to a DNA sequence which when ligated to a nucleotide sequence of interest is capable of controlling the transcription of the nucleotide sequence of interest into mRNA. A promoter is typically, though not necessarily, located 5' (*i.e.,* upstream) of a nucleotide sequence of interest whose transcription into mRNA it controls, and provides a site for specific binding by RNA polymerase and other transcription factors for initiation of transcription.

Promoters may be constitutive or regulatable. The term "constitutive" when made in reference to a promoter means that the promoter is capable of directing transcription of an operably linked nucleic acid sequence in the absence of a stimulus (*e.g.,* heat shock, chemicals, etc.). In contrast, a "regulatable" promoter is one that is capable of directing a level of transcription of an operably linked nucleic acid sequence in the presence of a stimulus (*e.g.,* heat shock, chemicals, etc.), which is different from the level of transcription of the operably linked nucleic acid sequence in the absence of the stimulus.

The presence of "splicing signals" on an expression vector often results in higher levels of expression of the recombinant transcript. Splicing signals mediate the removal of introns from the primary RNA transcript and consist of a splice donor and acceptor site (Sambrook et al., Molecular Cloning: A Laboratory Manual, 2nd ed., Cold Spring Harbor Laboratory Press, New York [1989], pp. 16.7-16.8). A commonly used splice donor and acceptor site is the splice junction from the 16S RNA of SV40.

Efficient expression of recombinant DNA sequences in eukaryotic cells requires expression of signals directing the efficient termination and polyadenylation of the resulting transcript. Transcription termination signals are generally found downstream of the polyadenylation signal and are a few hundred nucleotides in length. The term "poly A site" or "poly A sequence" as used herein denotes a DNA sequence that directs both the termination and polyadenylation of the nascent RNA transcript. Efficient polyadenylation of the recombinant transcript is desirable as transcripts lacking a poly A tail are unstable and are rapidly degraded. The poly A signal utilized in an expression vector may be "heterologous" or "endogenous." An endogenous poly A signal is one that is found naturally at the 3' end of the coding region of a given gene in the genome. A heterologous poly A signal is one that is isolated from one gene and placed 3' of another gene. A commonly used heterologous poly A signal is the SV40 poly A signal. The SV40 poly A signal is contained on a 237 bp BamHI/BclI restriction fragment and directs both termination and polyadenylation (Sambrook, *supra,* at 16.6-16.7).

Eukaryotic expression vectors may also contain "viral replicons "or "viral origins of replication." Viral replicons are viral DNA sequences that allow for the extrachromosomal replication of a vector in a host cell expressing the appropriate replication factors. Vectors that contain either the SV40 or polyoma virus origin of replication replicate to high "copy number" (up to 10⁴ copies/cell) in cells that express the appropriate viral T antigen. Vectors that contain the replicons from bovine papillomavirus or Epstein-Barr virus replicate extrachromosomally at "low copy number" (∼100 copies/cell). However, it is not intended that expression vectors be limited to any particular viral origin of replication.

As used herein, the term "long terminal repeat" of "LTR" refers to transcriptional control elements located in or isolated from the U3 region 5' and 3' of a retroviral genome. As is known in the art, long terminal repeats may be used as control elements in retroviral vectors, or isolated from the retroviral genome and used to control expression from other types of vectors.

As used herein, the term "secretion signal" refers to any DNA sequence which, when operably linked to a recombinant DNA sequence, encodes a signal peptide which is capable of causing the secretion of the recombinant polypeptide. In general, the signal peptides comprise a series of about 15 to 30 hydrophobic amino acid residues (*See, e.g.,* Zwizinski et al., J. Biol. Chem. 255(16): 7973-77 [1980], Gray et al., Gene 39(2): 247-54 [1985], and Martial et al, Science 205: 602-607 [1979]). Such secretion signal sequences are preferably derived from genes encoding polypeptides secreted from the cell type targeted for tissue-specific expression (*e.g.,* secreted milk proteins for expression in and secretion from mammary secretory cells). Secretory DNA sequences, however, are not limited to such sequences. Secretory DNA sequences from proteins secreted from many cell types and organisms may also be used (e.g., the secretion signals for t-PA, serum albumin, lactoferrin, and growth hormone, and secretion signals from microbial genes encoding secreted polypeptides such as from yeast, filamentous fungi, and bacteria).

As used herein, the terms "RNA export element" or "Pre-mRNA Processing Enhancer (PPE)" refer to 3' and 5' cis-acting post-transcriptional regulatory elements that enhance export of RNA from the nucleus. "PPE" elements include, but are not limited to Mertz sequences (described in U.S. Pat. Nos. 5,914,267 and 5,686,120) and woodchuck mRNA processing enhancer (WPRE; WO99/14310 and U.S. Pat. No. 6,136,597).

As used herein, the term "polycistronic" refers to an mRNA encoding more than polypeptide chain (See, *e.g.,* WO 93/03143, WO 88/05486, and European Pat. No.). Likewise, the term "arranged in polycistronic sequence" refers to the arrangement of genes encoding two different polypeptide chains in a single mRNA.

As used herein, the term "internal ribosome entry site" or "IRES" refers to a sequence located between polycistronic genes that permits the production of the expression product originating from the second gene by internal initiation of the translation of the dicistronic mRNA. Examples of internal ribosome entry sites include, but are not limited to, those derived from foot and mouth disease virus (FDV), encephalomyocarditis virus, poliovirus and RDV (Scheper et al., Biochem. 76: 801-809 [1994]; Meyer et al., J. Virol. 69: 2819-2824 [1995]; Jang et al., 1988, J. Virol. 62: 2636-2643 [1998]; Haller et al., J. Virol. 66: 5075-5086 [1995]). Vectors incorporating IRES's may be assembled as is known in the art. For example, a retroviral vector containing a polycistronic sequence may contain the following elements in operable association: nucleotide polylinker, gene of interest, an internal ribosome entry site and a mammalian selectable marker or another gene of interest. The polycistronic cassette is situated within the retroviral vector between the 5' LTR and the 3' LTR at a position such that transcription from the 5' LTR promoter transcribes the polycistronic message cassette. The transcription of the polycistronic message cassette may also be driven by an internal promoter (*e.g.,* cytomegalovirus promoter) or an inducible promoter, which maybe preferable depending on the use. The polycistronic message cassette can further comprise a cDNA or genomic DNA (gDNA) sequence operatively associated within the polylinker. Any mammalian selectable marker can be utilized as the polycistronic message cassette mammalian selectable marker. Such mammalian selectable markers are well known to those of skill in the art and can include, but are not limited to, kanamycin/G418, hygromycin B or mycophenolic acid resistance markers.

As used herein, the term "retrovirus" refers to a retroviral particle which is capable of entering a cell (*i.e.,* the particle contains a membrane-associated protein such as an envelope protein or a viral G glycoprotein which can bind to the host cell surface and facilitate entry of the viral particle into the cytoplasm of the host cell) and integrating the retroviral genome (as a double-stranded provirus) into the genome of the host cell. The term "retrovirus" encompasses Oncovirinae (*e.g.,* Moloney murine leukemia virus (MoMOLV), Moloney murine sarcoma virus (MoMSV), and Mouse mammary tumor virus (MMTV), Spumavirinae, amd Lentivirinae (*e.g.,* Human immunodeficiency virus, Simian immunodeficiency virus, Equine infection anemia virus, and Caprine arthritis-encephalitis virus; *See, e.g.,* U.S. Pat. Nos. 5,994,136 and 6,013,516).

As used herein, the term "retroviral vector" refers to a retrovirus that has been modified to express a gene of interest. Retroviral vectors can be used to transfer genes efficiently into host cells by exploiting the viral infectious process. Foreign or heterologous genes cloned (*i.e.,* inserted using molecular biological techniques) into the retroviral genome can be delivered efficiently to host cells that are susceptible to infection by the retrovirus. Through well known genetic manipulations, the replicative capacity of the retroviral genome can be destroyed. The resulting replication-defective vectors can be used to introduce new genetic material to a cell but they are unable to replicate. A helper virus or packaging cell line can be used to permit vector particle assembly and egress from the cell. Such retroviral vectors comprise a replication-deficient retroviral genome containing a nucleic acid sequence encoding at least one gene of interest (*i.e.,* a polycistronic nucleic acid sequence can encode more than one gene of interest), a 5' retroviral long terminal repeat (5' LTR); and a 3' retroviral long terminal repeat (3' LTR).

The term "pseudotyped retroviral vector" refers to a retroviral vector containing a heterologous membrane protein. The term "membrane-associated protein" refers to a protein (*e.g.,* a viral envelope glycoprotein or the G proteins of viruses in the Rhabdoviridae family such as VSV, Piry, Chandipura and Mokola) that are associated with the membrane surrounding a viral particle; these membrane-associated proteins mediate the entry of the viral particle into the host cell. The membrane associated protein may bind to specific cell surface protein receptors, as is the case for retroviral envelope proteins or the membrane-associated protein may interact with a phospholipid component of the plasma membrane of the host cell, as is the case for the G proteins derived from members of the Rhabdoviridae family.

The term "heterologous membrane-associated protein" refers to a membrane-associated protein which is derived from a virus that is not a member of the same viral class or family as that from which the nucleocapsid protein of the vector particle is derived. "Viral class or family" refers to the taxonomic rank of class or family, as assigned by the International Committee on Taxonomy of Viruses.

The term "Rhabdoviridae" refers to a family of enveloped RNA viruses that infect animals, including humans, and plants. The Rhabdoviridae family encompasses the genus Vesiculovirus that includes vesicular stomatitis virus (VSV), Cocal virus, Piry virus, Chandipura virus, and Spring viremia of carp virus (sequences encoding the Spring viremia of carp virus are available under GenBank accession number U18101). The G proteins of viruses in the Vesiculovirus genera are virally-encoded integral membrane proteins that form externally projecting homotrimeric spike glycoproteins complexes that are required for receptor binding and membrane fusion. The G proteins of viruses in the Vesiculovirus genera have a covalently bound palmititic acid (C₁₆) moiety. The amino acid sequences of the G proteins from the Vesiculoviruses are fairly well conserved. For example, the Piry virus G protein share about 38% identity and about 55% similarity with the VSV G proteins (several strains of VSV are known, *e.g*., Indiana, New Jersey, Orsay, San Juan, etc., and their G proteins are highly homologous). The Chandipura virus G protein and the VSV G proteins share about 37% identity and 52% similarity. Given the high degree of conservation (amino acid sequence) and the related functional characteristics (*e.g.,* binding of the virus to the host cell and fusion of membranes, including syncytia formation) of the G proteins of the Vesiculoviruses, the G proteins from non-VSV Vesiculoviruses may be used in place of the VSV G protein for the pseudotyping of viral particles. The G proteins of the Lyssa viruses (another genera within the Rhabdoviridae family) also share a fair degree of conservation with the VSV G proteins and function in a similar manner (*e.g.,* mediate fusion of membranes) and therefore may be used in place of the VSV G protein for the pseudotyping of viral particles. The Lyssa viruses include the Mokola virus and the Rabies viruses (several strains of Rabies virus are known and their G proteins have been cloned and sequenced). The Mokola virus G protein shares stretches of homology (particularly over the extracellular and transmembrane domains) with the VSV G proteins which show about 31% identity and 48% similarity with the VSV G proteins. Preferred G proteins share at least 25% identity, preferably at least 30% identity and most preferably at least 35% identity with the VSV G proteins. The VSV G protein from which New Jersey strain (the sequence of this G protein is provided in GenBank accession numbers M27165 and M21557) is employed as the reference VSV G protein.

As used herein, the term "lentivirus vector" refers to retroviral vectors derived from the Lentiviridae family (e.g., human immunodeficiency virus, simian immunodeficiency virus, equine infectious anemia virus, and caprine arthritis-encephalitis virus) that are capable of integrating into non-dividing cells (*See, e.g.,* U.S. Pat. Nos. 5,994,136 and 6,013,516).

The term "pseudotyped lentivirus vector" refers to lentivirus vector containing a heterologous membrane protein (*e.g.,* a viral envelope glycoprotein or the G proteins of viruses in the Rhabdoviridae family such as VSV, Piry, Chandipura and Mokola).

As used herein, the term "transposon" refers to transposable elements (*e.g.,* Tn5, Tn7, and Tn10) that can move or transpose from one position to another in a genome. In general, the transposition is controlled by a transposase. The term "transposon vector," as used herein, refers to a vector encoding a nucleic acid of interest flanked by the terminal ends of transposon. Examples of transposon vectors include, but are not limited to, those described in U.S. Pat. Nos. 6,027,722; 5,958,775; 5,968,785; 5,965,443; and 5,719,055 reference.

As used herein, the term "adeno-associated virus (AAV) vector" refers to a vector derived from an adeno-associated virus serotype, including without limitation, AAV-1, AAV-2, AAV-3, AAV-4, AAV-5, AAVX7, etc. AAV vectors can have one or more of the AAV wild-type genes deleted in whole or part, preferably the rep and/or cap genes, but retain functional flanking ITR sequences.

AAV vectors can be constructed using recombinant techniques that are known in the art to include one or more heterologous nucleotide sequences flanked on both ends (5' and 3') with functional AAV ITRs. In the practice of the invention, an AAV vector can include at least one AAV ITR and a suitable promoter sequence positioned upstream of the heterologous nucleotide sequence and at least one AAV ITR positioned downstream of the heterologous sequence. A "recombinant AAV vector plasmid" refers to one type of recombinant AAV vector wherein the vector comprises a plasmid. As with AAV vectors in general, 5' and 3' ITRs flank the selected heterologous nucleotide sequence.

AAV vectors can also include transcription sequences such as polyadenylation sites, as well as selectable markers or reporter genes, enhancer sequences, and other control elements that allow for the induction of transcription. Such control elements are described above.

As used herein, the term "AAV virion" refers to a complete virus particle. An AAV virion may be a wild type AAV virus particle (comprising a linear, single-stranded AAV nucleic acid genome associated with an AAV capsid, *i.e.,* a protein coat), or a recombinant AAV virus particle (described below). In this regard, single-stranded AAV nucleic acid molecules (either the sense/coding strand or the antisense/anticoding strand as those terms are generally defined) can be packaged into an AAV virion; both the sense and the antisense strands are equally infectious.

As used herein, the term "recombinant AAV virion" or "rAAV" is defined as an infectious, replication-defective virus composed of an AAV protein shell encapsidating (*i.e.,* surrounding with a protein coat) a heterologous nucleotide sequence, which in turn is flanked 5' and 3' by AAV ITRs. A number of techniques for constructing recombinant AAV virions are known in the art (*See, e.g.,* U.S. Patent No. 5,173,414; WO 92/01070; WO 93/03769; Lebkowski et al., Molec. Cell. Biol. 8:3988-3996 [1988]; Vincent et al., Vaccines 90 [1990] (Cold Spring Harbor Laboratory Press); Carter, Current Opinion in Biotechnology 3:533-539 [1992]; Muzyczka, Current Topics in Microbiol. and Immunol. 158:97-129 [1992]; Kotin, Human Gene Therapy 5:793-801 [1994]; Shelling and Smith, Gene Therapy 1:165-169 [1994]; and Zhou et al., J. Exp. Med. 179:1867-1875 [1994]).

Suitable nucleotide sequences for use in AAV vectors (and, indeed, any of the vectors described herein) include any functionally relevant nucleotide sequence. Thus, the AAV vectors of the present invention can comprise any desired gene that encodes a protein that is defective or missing from a target cell genome or that encodes a non-native protein having a desired biological or therapeutic effect (*e.g.,* an antiviral function), or the sequence can correspond to a molecule having an antisense or ribozyme function. Suitable genes include those used for the treatment of inflammatory diseases, autoimmune, chronic and infectious diseases, including such disorders as AIDS, cancer, neurological diseases, cardiovascular disease, hypercholestemia; various blood disorders including various anemias, thalasemias and hemophilia; genetic defects such as cystic fibrosis, Gaucher's Disease, adenosine deaminase (ADA) deficiency, emphysema, etc. A number of antisense oligonucleotides (*e.g.,* short oligonucleotides complementary to sequences around the translational initiation site (AUG codon) of an mRNA) that are useful in antisense therapy for cancer and for viral diseases have been described in the art. (*See, e.g.,* Han et al., Proc. Natl. Acad. Sci. USA 88:4313-4317 [1991]; Uhlmann et al., Chem. Rev. 90:543-584 [1990]; Helene et al., Biochim. Biophys. Acta. 1049:99-125 [1990]; Agarwal et al., Proc. Natl. Acad. Sci. USA 85:7079-7083 [1989]; and Heikkila et al., Nature 328:445-449 [1987]). For a discussion of suitable ribozymes, *see, e.g.,* Cech et al. (1992) J. Biol. Chem. 267:17479-17482 and U.S. Patent No. 5,225,347.

By "adeno-associated virus inverted terminal repeats" or "AAV ITRs" is meant the art-recognized palindromic regions found at each end of the AAV genome which function together in cis as origins of DNA replication and as packaging signals for the virus. For use with the present invention, flanking AAV ITRs are positioned 5' and 3' of one or more selected heterologous nucleotide sequences and, together with the rep coding region or the Rep expression product, provide for the integration of the selected sequences into the genome of a target cell.

The nucleotide sequences of AAV ITR regions are known (*See, e.g.,* Kotin, Human Gene Therapy 5:793-801 [1994]; Berns, K.I. "Parvoviridae and their Replication" in Fundamental Virology, 2nd Edition, (B.N. Fields and D.M. Knipe, eds.) for the AAV-2 sequence. As used herein, an "AAV ITR" need not have the wild-type nucleotide sequence depicted, but may be altered, *e.g.,* by the insertion, deletion or substitution of nucleotides. Additionally, the AAV ITR may be derived from any of several AAV serotypes, including without limitation, AAV-1, AAV-2, AAV-3, AAV-4, AAV-5, AAVX7, etc. The 5' and 3' ITRs which flank a selected heterologous nucleotide sequence need not necessarily be identical or derived from the same AAV serotype or isolate, so long as they function as intended, *i.e.,* to allow for the integration of the associated heterologous sequence into the target cell genome when the rep gene is present (either on the same or on a different vector), or when the Rep expression product is present in the target cell.

As used herein the term, the term "*in vitro*" refers to an artificial environment and to processes or reactions that occur within an artificial environment. In vitro environments can consist of, but are not limited to, test tubes and cell cultures. The term "*in vivo*" refers to the natural environment (*e.g.,* an animal or a cell) and to processes or reaction that occur within a natural environment.

As used herein, the term "clonally derived" refers to a cell line that it derived from a single cell.

As used herein, the term "clonally selecting" refers to selecting (*e.g.,* selecting for the presence of a integrated vector) cell lines derived from a single cell.

As used herein, the term "non-clonally derived" refers to a cell line that is derived from more than one cell.

As used herein, the term "passage" refers to the process of diluting a culture of cells that has grown to a particular density or confluency (*e.g.,* 70% or 80% confluent), and then allowing the diluted cells to regrow to the particular density or confluency desired (*e.g*., by replating the cells or establishing a new roller bottle culture with the cells.

As used herein, the term "stable," when used in reference to genome, refers to the stable maintenance of the information content of the genome from one generation to the next, or, in the particular case of a cell line, from one passage to the next. Accordingly, a genome is considered to be stable if no gross changes occur in the genome (*e.g.,* a gene is deleted or a chromosomal translocation occurs). The term "stable" does not exclude subtle changes that may occur to the genome such as point mutations.

As used herein, the term "response," when used in reference to an assay, refers to the generation of a detectable signal (*e.g.,* accumulation of reporter protein, increase in ion concentration, accumulation of a detectable chemical product).

As used herein, the term "membrane receptor protein" refers to membrane spanning proteins that bind a ligand (*e.g.,* a hormone or neurotransmitter). As is known in the art, protein phosphorylation is a common regulatory mechanism used by cells to selectively modify proteins carrying regulatory signals from outside the cell to the nucleus. The proteins that execute these biochemical modifications are a group of enzymes known as protein kinases. They may further be defined by the substrate residue that they target for phosphorylation. One group of protein kinases are the tyrosine kinases (TKs) which selectively phosphorylate a target protein on its tyrosine residues. Some tyrosine kinases are membrane-bound receptors (RTKs), and, upon activation by a ligand, can autophosphorylate as well as modify substrates. The initiation of sequential phosphorylation by ligand stimulation is a paradigm that underlies the action of such effectors as, for example, epidermal growth factor (EGF), insulin, platelet-derived growth factor (PDGF), and fibroblast growth factor (FGF). The receptors for these ligands are tyrosine kinases and provide the interface between the binding of a ligand (hormone, growth factor) to a target cell and the transmission of a signal into the cell by the activation of one or more biochemical pathways. Ligand binding to a receptor tyrosine kinase activates its intrinsic enzymatic activity (*See, e.g.,* Ullrich and Schlessinger, Cell 61:203-212 [1990]). Tyrosine kinases can also be cytoplasmic, non-receptor-type enzymes and act as a downstream component of a signal transduction pathway.

As used herein, the term "signal transduction protein" refers to a protein that is activated or otherwise affected by ligand binding to a membrane receptor protein or some other stimulus. Examples of signal transduction protein include adenyl cyclase, phospholipase C, and G-proteins. Many membrane receptor proteins are coupled to G-proteins (*i.e*., G-protein coupled receptors (GPCRs); for a review, see Neer, 1995, Cell 80:249-257 [1995]). Typically, GPCRs contain seven transmembrane domains. Putative GPCRs can be identified on the basis of sequence homology to known GPCRs.

GPCRs mediate signal transduction across a cell membrane upon the binding of a ligand to an extracellular portion of a GPCR. The intracellular portion of a GPCR interacts with a G-protein to modulate signal transduction from outside to inside a cell. A GPCR is therefore said to be "coupled" to a G-protein. G-proteins are composed of three polypeptide subunits: an α subunit, which binds and hydrolyses GTP, and a dimeric βγ subunit. In the basal, inactive state, the G-protein exists as a heterotrimer of the α and βγ subunits. When the G-protein is inactive, guanosine diphosphate (GDP) is associated with the α subunit of the G-protein. When a GPCR is bound and activated by a ligand, the GPCR binds to the G-protein heterotrimer and decreases the affinity of the Gα subunit for GDP. In its active state, the G subunit exchanges GDP for guanine triphosphate (GTP) and active Gα subunit disassociates from both the receptor and the dimeric βγ subunit. The disassociated, active Gα subunit transduces signals to effectors that are "downstream" in the G-protein signalling pathway within the cell. Eventually, the G-protein's endogenous GTPase activity returns active G subunit to its inactive state, in which it is associated with GDP and the dimeric βγ subunit.

Numerous members of the heterotrimeric G-protein family have been cloned, including more than 20 genes encoding various Gα subunits. The various G subunits have been categorized into four families, on the basis of amino acid sequences and functional homology. These four families are termed Gaₛ, Gαᵢ, Gα_{q}, and Gα₁₂. Functionally, these four families differ with respect to the intracellular signaling pathways that they activate and the GPCR to which they couple.

For example, certain GPCRs normally couple with Gas and, through Gaₛ, these GPCRs stimulate adenylyl cyclase activity. Other GPCRs normally couple with GGα_{q}, and through GGα_{q}, these GPCRs can activate phospholipase C (PLC), such as the β isoform of phospholipase C (*i.e.,* PLCβ, Stermweis and Smrcka, Trends in Biochem. Sci. 17:502-506 [1992]).

As used herein, the term "nucleic acid binding protein" refers to proteins that bind to nucleic acid, and in particular to proteins that cause increased (*i.e.,* activators or transcription factors) or decreased (*i.e.,* inhibitors) transcription from a gene.

As used herein, the term "ion channel protein" refers to proteins that control the ingress or egress of ions across cell membranes. Examples of ion channel proteins include, but are not limited to, the Na⁺-K⁺ ATPase pump, the Ca²⁺ pump, and the K⁺ leak channel.

As used herein, the term "protein kinase" refers to proteins that catalyze the addition of a phosphate group from a nucleoside triphosphate to an amino acid side chain in a protein. Kinases comprise the largest known enzyme superfamily and vary widely in their target proteins. Kinases may be categorized as protein tyrosine kinases (PTKs), which phosphorylate tyrosine residues, and protein serine/threonine kinases (STKs), which phosphorylate serine and/or threonine residues. Some kinases have dual specificity for both serine/threonine and tyrosine residues. Almost all kinases contain a conserved 250-300 amino acid catalytic domain. This domain can be further divided into 11 subdomains. N-terminal subdomains I-IV fold into a two-lobed structure that binds and orients the ATP donor molecule, and subdomain V spans the two lobes. C-terminal subdomains VI-XI bind the protein substrate and transfer the gamma phosphate from ATP to the hydroxyl group of a serine, threonine, or tyrosine residue. Each of the 11 subdomains contains specific catalytic residues or amino acid motifs characteristic of that subdomain. For example, subdomain I contains an 8-amino acid glycine-rich ATP binding consensus motif, subdomain II contains a critical lysine residue required for maximal catalytic activity, and subdomains VI through IX comprise the highly conserved catalytic core. STKs and PTKs also contain distinct sequence motifs in subdomains VI and VIII, which may confer hydroxyamino acid specificity. Some STKs and PTKs possess structural characteristics of both families. In addition, kinases may also be classified by additional amino acid sequences, generally between 5 and 100 residues, which either flank or occur within the kinase domain.

Non-transmembrane PTKs form signaling complexes with the cytosolic domains of plasma membrane receptors. Receptors that signal through non-transmembrane PTKs include cytokine, hormone, and antigen-specific lymphocytic receptors. Many PTKs were first identified as oncogene products in cancer cells in which PTK activation was no longer subject to normal cellular controls. In fact, about one third of the known oncogenes encode PTKs. Furthermore, cellular transformation (oncogenesis) is often accompanied by increased tyrosine phosphorylation activity *(See, e.g.,* Carbonneau, H. and Tonks, Annu. Rev. Cell Biol. 8:463-93 [1992]). Regulation of PTK activity may therefore be an important strategy in controlling some types of cancer.

Examples of protein kinases include, but are not limited to, cAMP-dependent protein kinase, protein kinase C, and cyclin-dependent protein kinases *(See, e.g.,* U.S. Pat. Nos. 6,034,228; 6,030,822; 6,030,788; 6,020,306; 6,013,455; 6,013,464; and 6,015,807).

As used herein, the term "protein phosphatase" refers to proteins that remove a phosphate group from a protein. Protein phosphatases are generally divided into two groups, receptor and non-receptor type proteins. Most receptor-type protein tyrosine phosphatases contain two conserved catalytic domains, each of which encompasses a segment of 240 amino acid residues *(See, e.g.,* Saito et al., Cell Growth and Diff. 2:59-65 [1991]). Receptor protein tyrosine phosphatases can be subclassified further based upon the amino acid sequence diversity of their extracellular domains (*See, e.g.,* Krueger et al., Proc. Natl. Acad. Sci. USA 89:7417-7421 [1992]). Examples of protein phosphatases include, but are not limited to, cdc25 a, b, and c, PTP20, PTP1D, and PTPλ (*See, e.g.,* U.S. Pat. Nos. 5,976,853; 5,994,074; 6,004,791; 5,981,251; 5,976,852; 5,958,719; 5,955,592; and 5,952,212).

As used herein, the term "protein encoded by an oncogene" refers to proteins that cause, either directly or indirectly, the neoplastic transformation of a host cell. Examples of oncogenes include, but are not limited to, the following genes: *src, fps, fes, fgr, ros, H-ras, abl, ski, erbA, erbB, fins, fos, mos, sis, myc, myb, rel, kits raf, K-ras,* and *ets.*

As used herein, the term "immunoglobulin" refers to proteins that bind a specific antigen. Immunoglobulins include, but are not limited to, polyclonal, monoclonal, chimeric, and humanized antibodies, Fab fragments, F(ab')2 fragments, and includes immunoglobulins of the following classes: IgG, IgA, IgM, IgD, IbE, and secreted immunoglobulins (sIg). Immunoglobulins generally comprise two identical heavy chains (y, α, µ, δ, or ε) and two light chains (κ or λ).

As used herein, the term "antigen binding protein" refers to proteins that bind to a specific antigen. "Antigen binding proteins" include, but are not limited to, immunoglobulins, including polyclonal, monoclonal, chimeric, and humanized antibodies; Fab fragments, F(ab')2 fragments, and Fab expression libraries; and single chain antibodies. Various procedures known in the art are used for the production of polyclonal antibodies. For the production of an antibody, various host animals can be immunized by injection with the peptide corresponding to the desired epitope including but not limited to rabbits, mice, rats, sheep, goats, etc. In a preferred embodiment, the peptide is conjugated to an immunogenic carrier (*e.g.,* diphtheria toxoid, bovine serum albumin (BSA), or keyhole limpet hemocyanin (KLH)). Various adjuvants are used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and Corynebacterium parvum.

For preparation of monoclonal antibodies, any technique that provides for the production of antibody molecules by continuous cell lines in culture may be used (*See, e.g.,* Harlow and Lane, Antibodies: A Laboratory Manual, Cold Spring Harbor Laboratory Press, Cold Spring Harbor, NY). These include, but are not limited to, the hybridoma technique originally developed by Köhler and Milstein (Köhler and Milstein, Nature 256:495-497 [1975]), as well as the trioma technique, the human B-cell hybridoma technique (*See e.g.,* Kozbor et al. Immunol. Today 4:72 [1983]), and the EBV-hybridoma technique to produce human monoclonal antibodies (Cole et al., in Monoclonal Antibodies and Cancer Therapy, Alan R. Liss, Inc., pp. 77-96 [1985]).

According to the invention, techniques described for the production of single chain antibodies (U.S. Patent 4,946,778) can be adapted to produce specific single chain antibodies as desired. An additional embodiment of the invention utilizes the techniques known in the art for the construction of Fab expression libraries (Huse et al., Science 246:1275-1281 [1989]) to allow rapid and easy identification of monoclonal Fab fragments with the desired specificity.

Antibody fragments that contain the idiotype (antigen binding region) of the antibody molecule can be generated by known techniques. For example, such fragments include but are not limited to: the F(ab')2 fragment that can be produced by pepsin digestion of an antibody molecule; the Fab' fragments that can be generated by reducing the disulfide bridges of an F(ab')2 fragment, and the Fab fragments that can be generated by treating an antibody molecule with papain and a reducing agent.

Genes encoding antigen binding proteins can be isolated by methods known in the art. In the production of antibodies, screening for the desired antibody can be accomplished by techniques known in the art (*e.g*., radioimmunoassay, ELISA (enzyme-linked immunosorbant assay), "sandwich" immunoassays, immunoradiometric assays, gel diffusion precipitin reactions, immunodiffusion assays, in situ immunoassays (using colloidal gold, enzyme or radioisotope labels, for example), Western Blots, precipitation reactions, agglutination assays *(e.g.,* gel agglutination assays, hemagglutination assays, etc.), complement fixation assays, immunofluorescence assays, protein A assays, and immunoelectrophoresis assays, etc.) etc.

As used herein, the term "reporter gene" refers to a gene encoding a protein that may be assayed. Examples of reporter genes include, but are not limited to, luciferase (*See, e.g*., deWet et al., Mol. Cell. Biol. 7:725 [1987] and U.S. Pat Nos. 6,074,859; 5,976,796; 5,674,713; and 5,618,682), green fluorescent protein (*e.g.,* GenBank Accession Number U43284; a number of GFP variants are commercially available from CLONTECH Laboratories, Palo Alto, CA), chloramphenicol acetyltransferase, β-galactosidase, alkaline phosphatase, and horse radish peroxidase.

As used herein, the term "purified" refers to molecules, either nucleic or amino acid sequences, that are removed from their natural environment, isolated or separated. An "isolated nucleic acid sequence" is therefore a purified nucleic acid sequence. "Substantially purified" molecules are at least 60% free, preferably at least 75% free, and more preferably at least 90% free from other components with which they are naturally associated.

The term "test compound" refers to any chemical entity, pharmaceutical, drug, and the like contemplated to be useful in the treatment and/or prevention of a disease, illness, sickness, or disorder of bodily function, or otherwise alter the physiological or cellular status of a sample. Test compounds comprise both known and potential therapeutic compounds. A test compound can be determined to be therapeutic by screening using the screening methods of the present invention. A "known therapeutic compound" refers to a therapeutic compound that has been shown (*e.g.,* through animal trials or prior experience with administration to humans) to be effective in such treatment or prevention.

### DETAILED DESCRIPTION OF THE INVENTION

The present invention relates to the production of proteins in host cells, and more particularly to the use of host cells containing multiple integrated copies of an integrating vector. The present invention utilizes integrating vectors *(i.e.,* vectors that integrate via an integrase or transposase) to create cell lines containing a high copy number of a nucleic acid encoding a gene of interest. The transfected genomes of the high copy number cells are stable through repeated passages (*e.g.,* at least 10 passages, preferably at least 50 passages, and most preferably at least 100 passages). Furthermore, the host cells of the present invention are capable of producing high levels of protein (*e.g.,* more than 1 pg/cell/day, preferably more than 10 pg/cell/day, more preferably more than 50 pg/cell/day, and most preferably more than 100 pg/cell/day).

The genomic stability and high expression levels of the host cells of the present invention provide distinct advantages over previously described methods of cell culture. For example, mammalian cell lines containing multiple copies of genes are known in the art to be intrinsically unstable. Indeed, this instability is a recognized problem facing researchers desiring to use mammalian cell lines for various purposes, including high throughput screening assays (See, *e.g.,* Sittampalam et al., Curr. Opin. Chem. Biol. 1(3):384-91 [1997]).

It is not intended that the present invention be limited to particular mechanism of action. Indeed, an understanding of the mechanism is not necessary to make and use the present invention. However, the high genomic stability and protein expression levels of the host cells of the present invention are thought to be due to unique properties of the integrating vectors (*e.g.,* retroviral vectors). For example, it is known that retroviruses are inherited elements in the germ line of many organisms. Indeed, as much as 5-10% of the mammalian genome may consist of elements contributed by reverse transcription, indicating a high degree of stability. Likewise, many of these types of vectors target active (*e.g.,* DNase I hypersensitive sites) transcriptional sites in the genome.

Many investigations have focused on the deleterious effects of retroviral and transposon integration. The property of targeting active regions of the genome has led to the use of retroviral vectors and transposon vectors in promoter trap schemes and for saturation mutagenesis (See, *e.g.,* U.S. Pat. Nos. 5,627,058 and 5,922,601). In promoter trap schemes, the cells are infected with a promoterless reporter vector. If the promoterless vector integrates downstream of a promoter (*i.e.,* into a gene), the reporter gene encoded by the vector is activated. The promoter can then be cloned and further characterized.

As can be seen, these schemes rely on the disruption of an endogenous gene. Therefore, it is surprising that the methods of the present invention, which utilize integrating vectors at high multiplicities of infection that would normally be thought to lead to gene disruption, led to the development of stable cell lines that express high quantities of a protein of interest. The development of these cell lines is described more fully below. The description is divided into the following sections: I) Host Cells; II) Vectors and Methods of Transfection; and III) Uses of Transfected Host Cells.

### I. Host Cells

The present invention contemplates the transfection of a variety of host cells with integrating vectors. A number of mammalian host cell lines are known in the art. In general, these host cells are capable of growth and survival when placed in either monolayer culture or in suspension culture in a medium containing the appropriate nutrients and growth factors, as is described in more detail below. Typically, the cells are capable of expressing and secreting large quantities of a particular protein of interest into the culture medium. Examples of suitable mammalian host cells include, but are not limited to Chinese hamster ovary cells (CHO-K1, ATCC CC1-61); bovine mammary epithelial cells (ATCC CRL 10274; bovine mammary epithelial cells); monkey kidney CV1 line transformed by SV40 (COS-7, ATCC CRL 1651); human embryonic kidney line (293 or 293 cells subcloned for growth in suspension culture; see, *e.g.,* Graham et al., J. Gen Virol., 36:59 [1977]); baby hamster kidney cells (BHK, ATCC CCL 10); mouse sertoli cells (TM4, Mather, Biol. Reprod. 23:243-251 [1980]); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68 [1982]); MRC 5 cells; FS4 cells; rat fibroblasts (208F cells); MDBK cells (bovine kidney cells); and a human hepatoma line (Hep G2).

In addition to mammalian cell lines, the present invention also contemplates the transfection of plant protoplasts with integrating vectors at a low or high multiplicity of infection. For example, the present invention contemplates a plant cell or whole plant comprising at least ten integrated vectors pseudotyped retroviral vector. All plants that can be produced by regeneration from protoplasts can also be transfected using the process according to the invention (*e.g.,* cultivated plants of the genera Solanum, Nicotiana, Brassica, Beta, Pisum, Phaseolus, Glycine, Helianthus, Allium, Avena, Hordeum, Oryzae, Setaria, Secale, Sorghum, Triticum, Zea, Musa, Cocos, Cydonia, Pyrus, Malus, Phoenix, Elaeis, Rubus, Fragaria, Prunus, Arachis, Panicum, Saccharum, Coffea, Camellia, Ananas, Vitis or Citrus). In general, protoplasts are produced in accordance with conventional methods *(See, e.g.,* U.S. Pat. Nos. 4,743,548; 4,677,066, 5,149,645; and 5,508,184). Plant tissue may be dispersed in an appropriate medium having an appropriate osmotic potential (*e.g.,* 3 to 8 wt. % of a sugar polyol) and one or more polysaccharide hydrolases (*e.g*., pectinase, cellulase, etc.), and the cell wall degradation allowed to proceed for a sufficient time to provide protoplasts. After filtration the protoplasts may be isolated by centrifugation and may then be resuspended for subsequent treatment or use. Regeneration of protoplasts kept in culture to whole plants is performed by methods known in the art (See, *e.g.,* Evans et al., Handbook of Plant Cell Culture, 1: 124-176, MacMillan Publishing Co., New York [1983]; Binding, Plant Protoplasts, p. 21-37, CRC Press, Boca Raton [1985],) and Potrykus and Shillito, Methods in Enzymology, Vol. 118, Plant Molecular Biology, A. and H. Weissbach eds., Academic Press, Orlando [1986]).

The present invention also contemplates the use of amphibian and insect host cell lines. Examples of suitable insect host cell lines include, but are not limited to, mosquito cell lines (*e.g.,* ATCC CRL-1660). Examples of suitable amphibian host cell lines include, but are not limited to, toad cell lines (*e.g.,* ATCC CCL-102).

### II. Vectors and Methods for Transfection

According to the present invention, host cells such as those described above are transduced or transfected with integrating vectors. Examples of integrating vectors include, but are not limited to, retroviral vectors, lentiviral vectors, adeno-associated viral vectors, and transposon vectors. The design, production, and use of these vectors in the present invention is described below.

### A. Retroviral Vectors

Retroviruses (family Retroviridae) are divided into three groups: the spumaviruses (*e.g.,* human foamy virus); the lentiviruses (*e.g.,* human immunodeficiency virus and sheep visna virus) and the oncoviruses (*e.g.,* MLV, Rous sarcoma virus).

Retroviruses are enveloped (*i.e.,* surrounded by a host cell-derived lipid bilayer membrane) single-stranded RNA viruses which infect animal cells. When a retrovirus infects a cell, its RNA genome is converted into a double-stranded linear DNA form *(i.e.,* it is reverse transcribed). The DNA form of the virus is then integrated into the host cell genome as a provirus. The provirus serves as a template for the production of additional viral genomes and viral mRNAs. Mature viral particles containing two copies of genomic RNA bud from the surface of the infected cell. The viral particle comprises the genomic RNA, reverse transcriptase and other pol gene products inside the viral capsid (which contains the viral gag gene products), which is surrounded by a lipid bilayer membrane derived from the host cell containing the viral envelope glycoproteins (also referred to as membrane-associated proteins).

The organization of the genomes of numerous retroviruses is well known to the art and this has allowed the adaptation of the retroviral genome to produce retroviral vectors. The production of a recombinant retroviral vector carrying a gene of interest is typically achieved in two stages.

First, the gene of interest is inserted into a retroviral vector which contains the sequences necessary for the efficient expression of the gene of interest (including promoter and/or enhancer elements which may be provided by the viral long terminal repeats (LTRs) or by an internal promoter/enhancer and relevant splicing signals), sequences required for the efficient packaging of the viral RNA into infectious virions (*e.g.,* the packaging signal (Psi), the tRNA primer binding site (-PBS), the 3' regulatory sequences required for reverse transcription (+PBS)) and the viral LTRs. The LTRs contain sequences required for the association of viral genomic RNA, reverse transcriptase and integrase functions, and sequences involved in directing the expression of the genomic RNA to be packaged in viral particles. For safety reasons, many recombinant retroviral vectors lack functional copies of the genes that are essential for viral replication (these essential genes are either deleted or disabled); therefore, the resulting virus is said to be replication defective.

Second, following the construction of the recombinant vector, the vector DNA is introduced into a packaging cell line. Packaging cell lines provide proteins required in trans for the packaging of the viral genomic RNA into viral particles having the desired host range (*i.e.,* the viral-encoded gag, pol and env proteins). The host range is controlled, in part, by the type of envelope gene product expressed on the surface of the viral particle. Packaging cell lines may express ecotrophic, amphotropic or xenotropic envelope gene products. Alternatively, the packaging cell line may lack sequences encoding a viral envelope (env) protein. In this case the packaging cell line will package the viral genome into particles that lack a membrane-associated protein (e.g., an env protein). In order to produce viral particles containing a membrane associated protein that will permit entry of the virus into a cell, the packaging cell line containing the retroviral sequences is transfected with sequences encoding a membrane-associated protein (*e.g.,* the G protein of vesicular stomatitis virus (VSV)). The transfected packaging cell will then produce viral particles, which contain the membrane-associated protein expressed by the transfected packaging cell line; these viral particles, which contain viral genomic RNA derived from one virus encapsidated by the envelope proteins of another virus are said to be pseudotyped virus particles.

The retroviral vectors of the present invention can be further modified to include additional regulatory sequences. As described above, the retroviral vectors of the present invention include the following elements in operable association: a) a 5' LTR; b) a packaging signal; c) a 3' LTR and d) a nucleic acid encoding a protein of interest located between the 5' and 3' LTRs. In some embodiments of the present invention, the nucleic acid of interest may be arranged in opposite orientation to the 5' LTR when transcription from an internal promoter is desired. Suitable internal promoters include, but are not limited to, the alpha-lactalbumin promoter, the CMV promoter (human or ape), and the thymidine kinase promoter.

In other embodiments of the present invention, where secretion of the protein of interest is desired, the vectors are modified by including a signal peptide sequence in operable association with the protein of interest. The sequences of several suitable signal peptides are known to those in the art, including, but not limited to, those derived from tissue plasminogen activator, human growth hormone, lactoferrin, alpha-casein, and alpha-lactalbumin.

In other embodiments of the present invention, the vectors are modified by incorporating an RNA export element (*See, e.g.,* U.S. Pat. Nos. 5,914,267; 6,136,597; and 5,686,120; and WO99/14310) either 3' or 5' to the nucleic acid sequence encoding the protein of interest. It is contemplated that the use of RNA export elements allows high levels of expression of the protein of interest without incorporating splice signals or introns in the nucleic acid sequence encoding the protein of interest.

In still other embodiments, the vector further comprises at least one internal ribosome entry site (IRES) sequence. The sequences of several suitable IRES's are available, including, but not limited to, those derived from foot and mouth disease virus (FDV), encephalomyocarditis virus, and poliovirus. The IRES sequence can be interposed between two transcriptional units (e.g., nucleic acids encoding different proteins of interest or subunits of a multisubunit protein such as an antibody) to form a polycistronic sequence so that the two transcriptional units are transcribed from the same promoter.

The retroviral vectors of the present invention may also further comprise a selectable marker allowing selection of transformed cells. A number of selectable markers find use in the present invention, including, but not limited to the bacterial aminoglycoside 3' phosphotransferase gene (also referred to as the neo gene) that confers resistance to the drug G418 in mammalian cells, the bacterial hygromycin G phosphotransferase (hyg) gene that confers resistance to the antibiotic hygromycin and the bacterial xanthine-guanine phosphoribosyl transferase gene (also referred to as the gpt gene) that confers the ability to grow in the presence of mycophenolic acid. In some embodiments, the selectable marker gene is provided as part of polycistronic sequence that also encodes the protein of interest.

In still other embodiments of the present invention, the retroviral vectors may comprise recombination elements recognized by a recombination system (*e.g.,* the cre/loxP or flp recombinase systems, see, *e.g.,* Hoess et al., Nucleic Acids Res. 14:2287-2300 [1986], O'Gorman et al., Science 251:1351-55 [1991], van Deursen et al., Proc. Natl. Acad. Sci. USA 92:7376-80 [1995], and U.S. Pat. No. 6,025,192). After integration of the vectors into the genome of the host cell, the host cell can be transiently transfected (e.g., by electroporation, lipofection, or microinjection) with either a recombinase enzyme (*e.g.,* Cre recombinase) or a nucleic acid sequence encoding the recombinase enzyme and one or more nucleic acid sequences encoding a protein of interest flanked by sequences recognized by the recombination enzyme so that the nucleic acid sequence is inserted into the integrated vector.

Viral vectors, including recombinant retroviral vectors, provide a more efficient means of transferring genes into cells as compared to other techniques such as calcium phosphate-DNA co-precipitation or DEAE-dextran-mediated transfection, electroporation or microinjection of nucleic acids. It is believed that the efficiency of viral transfer is due in part to the fact that the transfer of nucleic acid is a receptor-mediated process *(i.e.,* the virus binds to a specific receptor protein on the surface of the cell to be infected). In addition, the virally transferred nucleic acid once inside a cell integrates in controlled manner in contrast to the integration of nucleic acids which are not virally transferred; nucleic acids transferred by other means such as calcium phosphate-DNA co-precipitation are subject to rearrangement and degradation.

The most commonly used recombinant retroviral vectors are derived from the amphotropic Moloney murine leukemia virus (MoMLV) (See *e.g*., Miller and Baltimore Mol. Cell. Biol. 6:2895 [1986]). The MoMLV system has several advantages: 1) this specific retrovirus can infect many different cell types, 2) established packaging cell lines are available for the production of recombinant MoMLV viral particles and 3) the transferred genes are permanently integrated into the target cell chromosome. The established MoMLV vector systems comprise a DNA vector containing a small portion of the retroviral sequence (*e.g.,* the viral long terminal repeat or "LTR" and the packaging or "psi" signal) and a packaging cell line. The gene to be transferred is inserted into the DNA vector. The viral sequences present on the DNA vector provide the signals necessary for the insertion or packaging of the vector RNA into the viral particle and for the expression of the inserted gene. The packaging cell line provides the proteins required for particle assembly (Markowitz et al., J. Virol. 62:1120 [1988]).

Despite these advantages, existing retroviral vectors based upon MoMLV are limited by several intrinsic problems: 1) they do not infect non-dividing cells (Miller et al., Mol. Cell. Biol 10:4239 [1990]), except, perhaps, oocytes; 2) they produce low titers of the recombinant virus (Miller and Rosman, BioTechniques 7: 980 [1980] and Miller, Nature 357: 455 [1990]); and 3) they infect certain cell types (*e.g.,* human lymphocytes) with low efficiency (Adams et al., Proc. Natl. Acad. Sci. USA 89:8981 [1992]). The low titers associated with MoMLV-based vectors have been attributed, at least in part, to the instability of the virus-encoded envelope protein. Concentration of retrovirus stocks by physical means (e.g., ultracentrifugation and ultrafiltration) leads to a severe loss of infectious virus.

The low titer and inefficient infection of certain cell types by MoMLV-based vectors has been overcome by the use of pseudotyped retroviral vectors, which contain the G protein of VSV as the membrane associated protein. Unlike retroviral envelope proteins that bind to a specific cell surface protein receptor to gain entry into a cell, the VSV G protein interacts with a phospholipid component of the plasma membrane (Mastromarino et al., J. Gen. Virol. 68:2359 [1977]). Because entry of VSV into a cell is not dependent upon the presence of specific protein receptors, VSV has an extremely broad host range. Pseudotyped retroviral vectors bearing the VSV G protein have an altered host range characteristic of VSV (*i.e.,* they can infect almost all species of vertebrate, invertebrate and insect cells). Importantly, VSV G-pseudotyped retroviral vectors can be concentrated 2000-fold or more by ultracentrifugation without significant loss of infectivity (Burns et al. Proc. Natl. Acad. Sci. USA 90:8033 [1993]).

The present invention is not limited to the use of the VSV G protein when a viral G protein is employed as the heterologous membrane-associated protein within a viral particle (See, *e.g.,* U.S. Pat. No. 5,512,421). The G proteins of viruses in the Vesiculovirus genera other than VSV, such as the Piry and Chandipura viruses, that are highly homologous to the VSV G protein and, like the VSV G protein, contain covalently linked palmitic acid (Brun et al. Intervirol. 38:274 [1995] and Masters et al., Virol. 171:285 (1990]). Thus, the G protein of the Piry and Chandipura viruses can be used in place of the VSV G protein for the pseudotyping of viral particles. In addition, the VSV G proteins of viruses within the Lyssa virus genera such as Rabies and Mokola viruses show a high degree of conservation (amino acid sequence as well as functional conservation) with the VSV G proteins. For example, the Mokola virus G protein has been shown to function in a manner similar to the VSV G protein (*i.e.,* to mediate membrane fusion) and therefore may be used in place of the VSV G protein for the pseudotyping of viral particles (Mebatsion et al., J. Virol. 69:1444 [1995]). Viral particles may be pseudotyped using either the Piry, Chandipura or Mokola G protein as described in Example 2, with the exception that a plasmid containing sequences encoding either the Piry, Chandipura or Mokola G protein under the transcriptional control of a suitable promoter element (*e*.*g*., the CMV intermediate-early promoter; numerous expression vectors containing the CMV IE promoter are available, such as the pcDNA3.1 vectors (Invitrogen)) is used in place of pHCMV-G. Sequences encoding other G proteins derived from other members of the Rhabdoviridae family may be used; sequences encoding numerous rhabdoviral G proteins are available from the GenBank database.

The majority of retroviruses can transfer or integrate a double-stranded linear form of the virus (the provirus) into the genome of the recipient cell only if the recipient cell is cycling (*i.e.,* dividing) at the time of infection. Retroviruses that have been shown to infect dividing cells exclusively, or more efficiently, include MLV, spleen necrosis virus, Rous sarcoma virus and human immunodeficiency virus (HIV; while HIV infects dividing cells more efficiently, HIV can infect non-dividing cells).

It has been shown that the integration of MLV virus DNA depends upon the host cell's progression through mitosis and it has been postulated that the dependence upon mitosis reflects a requirement for the breakdown of the nuclear envelope in order for the viral integration complex to gain entry into the nucleus (Roe et al., EMBO J. 12:2099 [1993]). However, as integration does not occur in cells arrested in metaphase, the breakdown of the nuclear envelope alone may not be sufficient to permit viral integration; there may be additional requirements such as the state of condensation of the genomic DNA (Roe *et al.,* supra).

### B. Lentiviral Vectors

The present invention also contemplates the use of lentiviral vectors to generate high copy number cell lines. The lentiviruses (*e.g*.*,* equine infectious anemia virus, caprine arthritis-encephalitis virus, human immunodeficiency virus) are a subfamily of retroviruses that are able to integrate into non-dividing cells. The lentiviral genome and the proviral DNA have the three genes found in all retroviruses: gag, pol, and env, which are flanked by two LTR sequences. The gag gene encodes the internal structural proteins (*e.g.,* matrix, capsid, and nucleocapsid proteins); the pol gene encodes the reverse transcriptase, protease, and integrase proteins; and the pol gene encodes the viral envelope glycoproteins. The 5' and 3' LTRs control transcription and polyadenylation of the viral RNAs. Additional genes in the lentiviral genome include the vif, vpr, tat, rev, vpu, nef, and vpx genes.

A variety of lentiviral vectors and packaging cell lines are known in the art and find use in the present invention (See, *e.g.,* U.S. Pat. Nos. 5,994,136 and 6,013,516). Furthermore, the VSV G protein has also been used to pseudotype retroviral vectors based upon the human immunodeficiency virus (HIV) (Naldini et al., Science 272:263 [1996]). Thus, the VSV G protein may be used to generate a variety of pseudotyped retroviral vectors and is not limited to vectors based on MoMLV. The lentiviral vectors may also be modified as described above to contain various regulatory sequences (*e.g.,* signal peptide sequences, RNA export elements, and IRES's). After the lentiviral vectors are produced, they may be used to transfect host cells as described above for retroviral vectors.

### C. Adeno-Associated Viral Vectors

The present invention also contemplates the use of adeno associated virus (AAV) vectors to generate high copy number cell lines. AAV is a human DNA parvovirus, which belongs to the genus Dependovirus. The AAV genome is composed of a linear, single-stranded DNA molecule that contains approximately 4680 bases. The genome includes inverted terminal repeats (ITRs) at each end that function in cis as origins of DNA replication and as packaging signals for the virus. The internal nonrepeated portion of the genome includes two large open reading frames, known as the AAV rep and cap regions, respectively. These regions code for the viral proteins involved in replication and packaging of the virion. A family of at least four viral proteins are synthesized from the AAV rep region, Rep 78, Rep 68, Rep 52 and Rep 40, named according to their apparent molecular weight. The AAV cap region encodes at least three proteins, VP1, VP2 and VP3 (for a detailed description of the AAV genome, see *e.g*., Muzyczka, Current Topics Microbiol. Immunol. 158:97-129 [1992]; Kotin, Human Gene Therapy 5:793-801 [1994]).

AAV requires coinfection with an unrelated helper virus, such as adenovirus, a herpesvirus or vaccinia, in order for a productive infection to occur. In the absence of such coinfection, AAV establishes a latent state by insertion of its genome into a host cell chromosome. Subsequent infection by a helper virus rescues the integrated copy, which can then replicate to produce infectious viral progeny. Unlike the non-pseudotyped retroviruses, AAV has a wide host range and is able to replicate in cells from any species so long as there is coinfection with a helper virus that will also multiply in that species. Thus, for example, human AAV will replicate in canine cells coinfected with a canine adenovirus. Furthermore, unlike the retroviruses, AAV is not associated with any human or animal disease, does not appear to alter the biological properties of the host cell upon integration and is able to integrate into nondividing cells. It has also recently been found that AAV is capable of site-specific integration into a host cell genome.

In light of the above-described properties, a number of recombinant AAV vectors have been developed for gene delivery (*See, e.g.,* U.S. Patent Nos. 5,173,414; 5,139,941; WO 92/01070 and WO 93/03769; Lebkowski et al., Molec. Cell. Biol. 8:3988-3996 [1988]; Carter, Current Opinion in Biotechnology 3:533-539 [1992]; Muzyczka, Current Topics in Microbiol. and Immunol. 158:97-129 [1992]; Kotin, (1994) Human Gene Therapy 5:793-801; Shelling and Smith, Gene Therapy 1:165-169 [1994]; and Zhou et al., J. Exp. Med. 179:1867-1875 [1994]).

Recombinant AAV virions can be produced in a suitable host cell that has been transfected with both an AAV helper plasmid and an AAV vector. An AAV helper plasmid generally includes AAV rep and cap coding regions, but lacks AAV ITRs. Accordingly, the helper plasmid can neither replicate nor package itself. An AAV vector generally includes a selected gene of interest bounded by AAV ITRs that provide for viral replication and packaging functions. Both the helper plasmid and the AAV vector bearing the selected gene are introduced into a suitable host cell by transient transfection. The transfected cell is then infected with a helper virus, such as an adenovirus, which transactivates the AAV promoters present on the helper plasmid that direct the transcription and translation of AAV rep and cap regions. Recombinant AAV virions harboring the selected gene are formed and can be purified from the preparation. Once the AAV vectors are produced, they may be used to transfect (*See, e.g.,* U.S. Pat. 5,843,742) host cells at the desired multiplicity of infection to produce high copy number host cells. As will be understood by those skilled in the art, the AAV vectors may also be modified as described above to contain various regulatory sequences *(e.g.,* signal peptide sequences, RNA export elements, and IRES's).

### D. Transposon Vectors

The present invention also contemplates the use of transposon vectors to generate high copy number cell lines. Transposons are mobile genetic elements that can move or transpose from one location another in the genome. Transposition within the genome is controlled by a transposase enzyme that is encoded by the transposon. Many examples of transposons are known in the art, including, but not limited to, Tn5 (*See e.g.,* de la Cruz et al., J. Bact. 175: 6932-38 [1993], Tn7 *(See e.g.*, Craig, Curr. Topics Microbiol. Immunol. 204: 27-48 [1996]), and Tn10 *(See e.g.,* Morisato and Kleckner, Cell 51:101-111 [1987]). The ability of transposons to integrate into genomes has been utilized to create transposon vectors *(See, e.g.,* U.S. Pat. Nos. 5,719,055; 5,968,785; 5,958,775; and 6,027,722). Because transposons are not infectious, transposon vectors are introduced into host cells via methods known in the art (*e.g.,* electroporation, lipofection, or microinjection). Therefore, the ratio of transposon vectors to host cells may be adjusted to provide the desired multiplicity of infection to produce the high copy number host cells of the present invention.

Transposon vectors suitable for use in the present invention generally comprise a nucleic acid encoding a protein of interest interposed between two transposon insertion sequences. Some vectors also comprise a nucleic acid sequence encoding a transposase enzyme. In these vectors, the one of the insertion sequences is positioned between the transposase enzyme and the nucleic acid encoding the protein of interest so that it is not incorporated into the genome of the host cell during recombination. Alternatively, the transposase enzyme may be provided by a suitable method (*e.g.,* lipofection or microinjection). As will be understood by those skilled in the art, the transposon vectors may also be modified as described above to contain various regulatory sequences (*e.g.,* signal peptide sequences, RNA export elements, and IRES's).

### E. Transfection at High Multiplicities of Infection

Once integrating vectors (*e.g.,* retroviral vectors) encoding a protein of interest have been produced, they may be used to transfect or transduce host cells (examples of which are described above in Section I). Preferably, host cells are transfected or transduced with 10 integrating vectors at a multiplicity of infection sufficient to result in the integration of at least 10 pseudotyped retroviral vectors. In some embodiments, multiplicities of infection of from 10 to 1,000,000 maybe utilized, so that the genomes of the infected host cells contain from 10 to 100 copies of the integrated vectors, and preferably from 10 to 50 copies of the integrated vectors. In other embodiments, a multiplicity of infection of from 10 to 10,000 is utilized. The pseudotyped retroviral vectors are concentrated to the appropriate titer by ultracentrifugation and then added to the host cell culture. Alternatively, the concentrated vectors can be diluted in a culture medium appropriate for the cell type. Additionally, when expression of more than one protein of interest by the host cell is desired, the host cells can be transfected with multiple vectors each containing a nucleic acid encoding a different protein of interest.

In each case, the host cells are exposed to medium containing the infectious retroviral vectors for a sufficient period of time to allow infection and subsequent integration of the vectors. In general, the amount of medium used to overlay the cells should be kept to as small a volume as possible so as to encourage the maximum amount of integration events per cell. As a general guideline, the number of colony forming units (cfu) per milliliter should be about 10⁵ to 10⁷ cfu/ml, depending upon the number of integration events desired.

The present invention is not limited to any particular mechanism of action. Indeed, an understanding of the mechanism of action is not necessary for practicing the present invention. However, the diffusion rate of the vectors is known to be very limited (*See, e.g.,* U.S. Pat. No. 5,866,400, for a discussion of diffusion rates). Therefore, it is expected that the actual integration rate will be lower (and in some cases much lower) than the multiplicity of infection. Applying the equations from U.S Pat. No. 5,866,400, a titer of 10⁶ cfu/ml has an average vector-vector spacing of 1 micron. The diffusion time of a MMLV vector across 100 microns is approximately 20 minutes. Accordingly, the vector can travel approximately 300 microns in one hour. If 1000 cells are plated in a T25 flask, the cells are spaced 2.5 mm apart on average. Using these values, the only 56 viral particles would be expected to contact a given cell within an hour. The Table below provides the expected contact rate for a given number of cells in a T25 flask with a particular vector titer. However, as shown below in the examples, the actual number of integrations obtained is much lower than may be predicted by these equations.

| **Vector Contact Frequency As A Function of Time and Cell Spacing** | | | |
|---|---|---|---|
| **Vector** Titer | **Cells/T25 Flask** | **MOI** | **Contacts/Hour** |
| 10⁶ | 1000 | 1,000 | 56 |
| 10⁶ | 100 | 10,000 | <56 |
| 10⁵ | 1000 | 100 | 5.6 |
| 10⁴ | 1000 | 10 | 0.6 |

Accordingly, it is contemplated that the actual integration rate is dependent not only on the multiplicity of infection, but also on the contact time (*i.e.,* the length of time the host cells are exposed to infectious vector), the confluency or geometry of the host cells being transfected, and the volume of media that the vectors are contained in. It is contemplated that these conditions can be varied as taught herein to produce host cell lines containing multiple integrated copies of integrating vectors. As demonstrated in Examples 8 and 9, MOI can be varied by either holding the number of cells constant and varying CFU's (Example 9), or by holding CFU's constant and varying cell number (Example 8).

In some embodiments, after transfection or transduction, the cells are allowed to multiply, and are then trypsinized and replated. Individual colonies are then selected to provide clonally selected cell lines. In still further embodiments, the clonally selected cell lines are screened by Southern blotting or INVADER assay to verify that the desired number of integration events has occurred. It is also contemplated that clonal selection allows the identification of superior protein producing cell lines. In other embodiments, the cells are not clonally selected following transfection.

In some embodiments, the host cells are transfected with vectors encoding different proteins of interest. The vectors encoding different proteins of interest can be used to transfect the cells at the same time (*e.g.,* the host cells are exposed to a solution containing vectors encoding different proteins of interest) or the transfection can be serial (*e.g.,* the host cells are first transfected with a vector encoding a first protein of interest, a period of time is allowed to pass, and the host cells are then transfected with a vector encoding a second protein of interest). In some preferred embodiments, the host cells are transfected with an integrating vector encoding a first protein of interest, high expressing cell lines containing multiple integrated copies of the integrating vector are selected (*e.g.,* clonally selected), and the selected cell line is transfected with an integrating vector encoding a second protein of interest. This process may be repeated to introduce multiple proteins of interest. In some embodiments, the multiplicities of infection may be manipulated (*e.g.,* increased or decreased) to increase or decrease the expression of the protein of interest. Likewise, the different promoters may be utilized to vary the expression of the proteins of interest. It is contemplated that these transfection methods can be used to construct host cell lines containing an entire exogenous metabolic pathway or to provide host cells with an increased capability to process proteins (*e.g.,* the host cells can be provided with enzymes necessary for post-translational modification).

In still further embodiments, cell lines are serially transfected with vectors encoding the same gene. In some preferred embodiments, the host cells are transfected (*e.g.,* at an MOI of about 10 to 100,000, preferably 100 to 10,000) with an integrating vector encoding a protein of interest, cell lines containing single or multiple integrated copies of the integrating vector or expressing high levels of the desired protein are selected (*e.g.,* clonally selected), and the selected cell line is retransfected with the vector (*e.g.,* at an MOI of about 10 to 100,000, preferably 100 to 10,000). In some embodiments, cell lines comprising at least two integrated copies of the vector are identified and selected. This process may be repeated multiple times until the desired level of protein expression is obtained and may also be repeated to introduce vectors encoding multiple proteins of interest. Unexpectedly, serial transfection with the same gene results in increases in protein production from the resulting cells that are not merely dditive.

### F. Transfection in the Absence of Selectable Markers

In some embodiments, the present invention discloses methods of transfecting host cells with integrating vectors lacking selectable markers. Experiments conducted during the course of development of the present invention (Example 26) demonstrated that vectors lacking selectable markers and grown in selection-free media resulted in higher levels of protein expression at the same vector copy number than vectors comprising selectable markers. In some embodiments, host cells comprising integrated vectors comprising an exogenous gene and lacking a selectable marker express at least 20%, preferably at least 30%, even more preferably, at least 50%, and still more preferably, at least 60% more protein than a host cell with the same number of integrated vectors than contain selectable markers.

In some embodiments, host cell lines derived from integrating vectors comprising an exogenous gene and lacking a selectable marker are clonally selected for the presence of the exogenous gene of interest. In preferred embodiments, selection is performed via clonal analysis of individual cells. In preferred embodiments, expression of a protein of interest is detected directly. For example, in some embodiments, selection is performed via an immunoassay (*e.g.,* an ELISA assay) with an antibody specific for the protein of interest. In other embodiments (e.g., those where the protein of interest is an enzyme) proteins are detected via a biochemical assay (e.g., via the altering of the substrate of an enzyme).

In other embodiments, nucleic acid encoding the protein of interest is detected. For example, in some embodiments, a PCR assay is performed using primers specific for the protein of interest. In other embodiments, nucleic acid is detected via a hybridization assay (*e.g*., including, but not limited to, Southern Blot, Northern Blot, INVADER Assay (Third Wave Technologies, Madison, WI), TaqMan assay (Applied Biosystems, Foster City, CA), and SNP-IT primer extension assay (Orchid Biosciences, Princeton, NJ).

### III. Uses of Transfected Host Cells

The host cells transfected at a high multiplicity of infection can be used for a variety of purposes. First, the host cells find use in the production of proteins for pharmaceutical, industrial, diagnostic, and other purposes. Second, host cells expressing a particular protein or proteins find use in screening assays (*e.g.,* high throughput screening). Third, the host cells find use in the production of multiple variants of proteins, followed by analysis of the activity of the protein variants. Each of these uses is explained in more detail below.

### A. Production of Proteins

It is contemplated that the host cells of the present invention find use in the production of proteins for pharmaceutical, industrial, diagnostic, and other uses. The present invention is not limited to the production of any particular protein. Indeed, the production of a wide variety of proteins is contemplated, including, but not limited to, erythropoietin, alpha-interferon, alpha-1 proteinase inhibitor, angiogenin, antithrombin III, beta-acid decarboxylase, human growth hormone, bovine growth hormone, porcine growth hormone, human serum albumin, beta-interferon, calf intestine alkaline phosphatase, cystic fibrosis transmembrane regulator, Factor VIII, Factor IX, Factor X, insulin, lactoferrin, tissue plasminogen activator, myelin basic protein, insulin, proinsulin, prolactin, hepatitis B antigen, immunoglobulins, monoclonal antibody CTLA4 Ig, Tag 72 monoclonal antibody, Tag 72 single chain antigen binding protein, protein C, cytokines and their receptors, including, for instance tumor necrosis factors alpha and beta, their receptors and their derivatives; renin; growth hormone releasing factor; parathyroid hormone; thyroid stimulating hormone; lipoproteins; alpha-1-antitrypsin; follicle stimulating hormone; calcitonin; luteinizing hormone; glucagon; von Willebrands factor; atrial natriuretic factor; lung surfactant; urokinase; bombesin; thrombin; hemopoietic growth factor; enkephalinase; human macrophage inflammatory protein (MIP-1-alpha); a serum albumin such mullerian-inhibiting substance; relaxin A-chain; relaxin B-chain; prorelaxin; mouse gonadotropin-associated peptide; beta-lactamase; DNase; inhibin; activin; vascular endothelial growth factor (VEGF); receptors for hormones or growth factors; integrin; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-beta; platelet-derived growth factor (PDGF); fibroblast growth factor such as aFGF and bFGF; epidermal growth factor (EGF); transforming growth factor (TGF) such as TGF-alpha and TGF-beta, including TGF-β1, TGF-β 2, TGF-β3, TGF- β4, or TGF- β5; insulin-like growth factor-I and -II (IGF-I and IGF-II); des(1-3)-IGF-I (brain IGF-I), insulinslike growth factor binding proteins; CD proteins such as CD-3, CD-4, CD-8, and CD-19; osteoinductive factors; immunotoxins; a bone morphogenetic protein (BMP); an interferon such as interferon-alpha, -beta, and -gamma; colony stimulating factors (CSFs), *e.g.,* M-CSF, GM-CSF and G-CSF; interleukins (ILs) *e.g.,* IL-1 to IL-10; superoxide dismutase; T-cell receptors; surface membrane proteins; decay accelerating factor; viral antigen such as, for example, a portion of the AIDS envelope; transport proteins; homing receptors; addressins; regulatory proteins; antibodies; chimeric proteins, such as immunoadhesins, and fragments of any of the above-listed polypeptides. Nucleic acid and protein sequences for these proteins are available in public databases such as GenBank.

In some embodiments, the host cells express more than one exogenous protein. For example, the host cells may be transfected vectors encoding different proteins of interest (*e.g.,* cotransfection or infection at a multiplicity of infection of 1000 with one vector encoding a first protein of interest and a second vector encoding a second protein of interest or serial transfection or infection) so that the host cell contains at least one integrated copy of a first vector encoding a first protein of interest and at least one integrated copy of second integrating vector encoding a second protein of interest. In other embodiments, more than one protein is expressed by arranging the nucleic acids encoding the different proteins of interest in a polycistronic sequence (*e.g.,* bicistronic or tricistronic sequences). This arrangement is especially useful when expression of the different proteins of interest in about a 1:1 molar ratio is desired (*e.g.,* expressing the light and heavy chains of an antibody molecule).

In still further embodiments, ribozymes are expressed in the host cells. It is contemplated that the ribozyme can be utilized for down-regulating expression of a particular gene or used in conjunction with gene switches such as TET, ecdysone, glucocorticoid enhancer, etc. to provide host cells with various phenotypes.

The transfected host cells are cultured according to methods known in the art. Suitable culture conditions for mammalian cells are well known in the art (See *e.g.,* J. Immunol. Methods (1983) 56:221-234 [1983], Animal Cell Culture: A Practical Approach 2nd Ed., Rickwood, D. and Hames, B. D., eds. Oxford University Press, New York [1992]).

The host cell cultures of the present invention are prepared in a media suitable for the particular cell being cultured. Commercially available media such as Ham's F10 (Sigma, St. Louis, MO), Minimal Essential Medium (MEM, Sigma), RPMI-1640 (Sigma), and Dulbecco's Modified Eagle's Medium (DMEM, Sigma) are exemplary nutrient solutions. Suitable media are also described in U.S. Pat. Nos. 4,767,704; 4,657,866; 4,927,762; 5,122,469; 4,560,655; and WO 90/03430 and WO 87/00195. Any of these media may be supplemented as necessary with serum, hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as gentamycin (gentamicin), trace elements (defined as inorganic compounds usually present at final concentrations in the micromolar range) lipids (such as linoleic or other fatty acids) and their suitable carriers, and glucose or an equivalent energy source. Any other necessary supplements may also be included at appropriate concentrations that would be known to those skilled in the art. For mammalian cell culture, the osmolality of the culture medium is generally about 290-330 mOsm.

The present invention also contemplates the use of a variety of culture systems *(e.g.,* petri dishes, 96 well plates, roller bottles, and bioreactors) for the transfected host cells. For example, the transfected host cells can be cultured in a perfusion system. Perfusion culture refers to providing a continuous flow of culture medium through a culture maintained at high cell density. The cells are suspended and do not require a solid support to grow on. Generally, fresh nutrients must be supplied continuously with concomitant removal of toxic metabolites and, ideally, selective removal of dead cells. Filtering, entrapment and micro-capsulation methods are all suitable for refreshing the culture environment at sufficient rates.

As another example, in some embodiments a fed batch culture procedure can be employed. In the preferred fed batch culture the mammalian host, cells and culture medium are supplied to a culturing vessel initially and additional culture nutrients are fed, continuously or in discrete increments, to the culture during culturing, with or without periodic cell and/or product harvest before termination of culture. The fed batch culture can include, for example, a semi-continuous fed batch culture, wherein periodically whole culture (including cells and medium) is removed and replaced by fresh medium. Fed batch culture is distinguished from simple batch culture in which all components for cell culturing (including the cells and all culture nutrients) are supplied to the culturing vessel at the start of the culturing process. Fed batch culture can be further distinguished from perfusion culturing insofar as the supernate is not removed from the culturing vessel during the process (in perfusion culturing, the cells are restrained in the culture by, *e.g*., filtration, encapsulation, anchoring to microcarriers etc. and the culture medium is continuously or intermittently introduced and removed from the culturing vessel). In some particularly preferred embodiments, the batch cultures are performed in roller bottles.

Further, the cells of the culture may be propagated according to any scheme or routine that may be suitable for the particular host cell and the particular production plan contemplated. Therefore, the present invention contemplates a single step or multiple step culture procedure. In a single step culture the host cells are inoculated into a culture environment and the processes of the instant invention are employed during a single production phase of the cell culture. Alternatively, a multi-stage culture is envisioned. In the multi-stage culture cells may be cultivated in a number of steps or phases. For instance, cells may be grown in a first step or growth phase culture wherein cells, possibly removed from storage, are inoculated into a medium suitable for promoting growth and high viability. The cells may be maintained in the growth phase for a suitable period of time by the addition of fresh medium to the host cell culture.

Fed batch or continuous cell culture conditions are devised to enhance growth of the mammalian cells in the growth phase of the cell culture. In the growth phase cells are grown under conditions and for a period of time that is maximized for growth. Culture conditions, such as temperature, pH, dissolved oxygen (dO₂) and the like, are those used with the particular host and will be apparent to the ordinarily skilled artisan. Generally, the pH is adjusted to a level between about 6.5 and 7.5 using either an acid (*e.g.,* CO₂) or a base (*e.g.,* Na₂CO₃ or NaOH). A suitable temperature range for culturing mammalian cells such as CHO cells is between about 30° to 38° C and a suitable dO₂ is between 5-90% of air saturation.

Following the polypeptide production phase, the polypeptide of interest is recovered from the culture medium using techniques that are well established in the art. The protein of interest preferably is recovered from the culture medium as a secreted polypeptide (*e.g.,* the secretion of the protein of interest is directed by a signal peptide sequence), although it also may be recovered from host cell lysates. As a first step, the culture medium or lysate is centrifuged to remove particulate cell debris. The polypeptide thereafter is purified from contaminant soluble proteins and polypeptides, with the following procedures being exemplary of suitable purification procedures: by fractionation on immunoaffinity or ion-exchange columns; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; and protein A Sepharose columns to remove contaminants such as IgG. A protease inhibitor such as phenyl methyl sulfonyl fluoride (PMSF) also may be useful to inhibit proteolytic degradation during purification. Additionally, the protein of interest can be fused in frame to a marker sequence that allows for purification of the protein of interest. Non-limiting examples of marker sequences include a hexahistidine tag, which may be supplied by a vector, preferably a pQE-9 vector, and a hemagglutinin (HA) tag. The HA tag corresponds to an epitope derived from the influenza hemagglutinin protein *(See e.g.,* Wilson et al., Cell, 37:767 [1984]). One skilled in the art will appreciate that purification methods suitable for the polypeptide of interest may require modification to account for changes in the character of the polypeptide upon expression in recombinant cell culture.

The host cells of the present invention are also useful for expressing G-protein coupled receptors (GPCRs) and other transmembrane proteins. It is contemplated that when these proteins are expressed, they are correctly inserted into the membrane in their native conformation. Thus, GPCRs and other transmembrane proteins may be purified as part of a membrane fraction or purified from the membranes by methods known in the art.

Furthermore, the vectors of the present invention are useful for co-expressing a protein of interest for which there is no assay or for which assays are difficult. In this system, a protein of interest and a signal protein are arranged in a polycistronic sequence. Preferably, an IRES sequence separates the signal protein and protein of interest (*e.g.,* a GPCR) and the genes encoding the signal protein and protein of interest are expressed as a single transcriptional unit. The present invention is not limited to any particular signal protein. Indeed, the use of a variety of signal proteins for which easy assays exist is contemplated. These signal proteins include, but are not limited to, green fluorescent protein, luciferase, beta-galactosidase, and antibody heavy or light chains. It is contemplated that when the signal protein and protein of interest are co-expressed from a polycistronic sequence, the presence of the signal protein is indicative of the presence of the protein of interest. Accordingly, in some embodiments, the present invention provides methods for indirectly detecting the expression of a protein of interest comprising providing a host cell transfected with a vector encoding a polycistronic sequence, wherein the polycistronic sequence comprises a signal protein and a protein of interest operably linked by an IRES, and culturing the host cells under conditions such that the signal protein and protein of interest are produced, wherein the presence of the signal protein indicates the presence of the protein of interest.

### B. Screening Compounds for Activity

The present invention contemplates the use of the high copy number cell lines for screening compounds for activity, and in particular to high throughput screening of compounds from combinatorial libraries (e.g., libraries containing greater than 10⁴ compounds). The high copy number cell lines of the present invention can be used in a variety of screening methods. In some embodiments, the cells can be used in second messenger assays that monitor signal transduction following activation of cell-surface receptors. In other embodiments, the cells can be used in reporter gene assays that monitor cellular responses at the transcription/translation level. In still further embodiments, the cells can be used in cell proliferation assays to monitor the overall growth/no growth response of cells to external stimuli.

In second messenger assays, the host cells are preferably transfected as described above with vectors encoding cell surface receptors, ion channels, cytoplasmic receptors, or other proteins involved in signal transduction (*e.g.,* G proteins, protein kinases, or protein phosphatases) *(See, e.g.,* U.S Pat. Nos. 5,670,113; 5,807,689; 5,876,946; and 6,027,875). The host cells are then treated with a compound or plurality of compounds (*e.g.,* from a combinatorial library) and assayed for the presence or absence of a response. It is contemplated that at least some of the compounds in the combinatorial library can serve as agonists, antagonists, activators, or inhibitors of the protein or proteins encoded by the vectors. It is also contemplated that at least some of the compounds in the combinatorial library can serve as agonists, antagonists, activators, or inhibitors of protein acting upstream or downstream of the protein encoded by the vector in a signal transduction pathway.

By way of non-limiting example, it is known that agonist engaged transmembrane receptors are functionally linked to the modulation of several well characterized promoter/enhancer elements (*e.g.,* AP1, cAMP response element (CRE), serum response element (SRE), and nuclear factor of activated T-cells (NF-AT)). Upon activation of a Gas coupling receptor, adenylyl cyclase is stimulated, producing increased concentrations of intracellular cAMP, stimulation of protein kinase A, phosphorylation of the CRE binding protein (CREB) and induction of promoters with CRE elements. Gαᵢ coupling receptors dampen CRE activity by inhibition of the same signal transduction components. Gα_{q} and some βγ pairs stimulate phospholipase C (PLC), and the generation of inositol triphosphate (IP3) and diacylglycerol (DAG). A transient flux in intracellular calcium promotes induction of calcineurin and NA-FT, as well as calmodulin (CaM)-dependent kinase and CREB. Increased DAG concentrations stimulate protein kinase C (PKC) and endosomal/lysosomal acidic sphingomyelinase (aSMase); while the aSMase pathway is dominant, both induce degradation of the NFκB inhibitor IκB as well as NFκB activation. In an alternative pathway, a receptor such as growth factor receptor is activated and recruits Sos to the plasma membrane, resulting in the stimulation of Ras, which in turn recruits the serine/threonine kinase Raf to the plasma membrane. Once activated, Raf phosphorylates MEK kinase, which phosphorylates and activates MAPK and the transcription factor ELK. ELK drives transcription from promoters with SRE elements, leading the synthesis of the transcription factors Fos and Jun, thus forming a transcription factor complex capable of activating AP1 sites. It is contemplated that the proteins forming the described pathways, as well as other receptors, kinases, phosphatases, and nucleic binding proteins, are targets for compounds in the combinatorial library, as well as candidates for expression in the host cells of the present invention.

In some embodiments, the second messenger assays measure fluorescent signals from reporter molecules that respond to intracellular changes (*e.g.,* Ca²⁺ concentration, membrane potential, pH, IP₃, cAMP, arachidonic acid release) due to stimulation of membrane receptors and ion channels (*e.g.,* ligand gated ion channels; see Denyer et al., Drug Discov. Today 3:323-32 [1998]; and Gonzales et al., Drug. Discov. Today 4:431-39 [1999]). Examples of reporter molecules include, but are not limited to, FRET (florescence resonance energy transfer) systems (*e.g.,* Cuo-lipids and oxonols, EDAN/DABCYL), calcium sensitive indicators (*e.g.*, Fluo-3, FURA 2, INDO 1, and FLUO3/AM, BAPTA AM), chloride-sensitive indicators (*e.g.*, SPQ, SPA), potassium-sensitive indicators (*e.g.,* PBFI), sodium-sensitive indicators (*e.g.,* SBFI), and pH sensitive indicators (*e.g.*, BCECF).

In general, the host cells are loaded with the indicator prior to exposure to the compound. Responses of the host cells to treatment with the compounds can be detected by methods known in the art, including, but not limited to, fluorescence microscopy, confocal microscopy (*e.g.*, FCS systems), flow cytometry, microfluidic devices, FLIPR systems (*See, e.g.,* Schroeder and Neagle, J. Biomol. Screening 1:75-80 [1996]), and plate-reading systems. In some preferred embodiments, the response (*e.g.*, increase in fluorescent intensity) caused by compound of unknown activity is compared to the response generated by a known agonist and expressed as a percentage of the maximal response of the known agonist. The maximum response caused by a known agonist is defined as a 100% response. Likewise, the maximal response recorded after addition of an agonist to a sample containing a known or test antagonist is detectably lower than the 100% response.

The cells are also useful in reporter gene assays. Reporter gene assays involve the use of host cells transfected with vectors encoding a nucleic acid comprising transcriptional control elements of a target gene (*i.e.,* a gene that controls the biological expression and function of a disease target) spliced to a coding sequence for a reporter gene. Therefore, activation of the target gene results in activation of the reporter gene product. Examples of reporter genes finding use in the present invention include, but are not limited to, chloramphenicol transferase, alkaline phosphatase, firefly and bacterial luciferases, β-galactosidase, α-lactamase, and green fluorescent protein. The production of these proteins, with the exception of green fluorescent protein, is detected through the use of chemiluminescent, colorimetric, or bioluminecent products of specific substrates (*e.g.*, X-gal and luciferin). Comparsions between compounds of known and unknown activities may be conducted as described above.

### C. Comparison of Variant Protein Activity

The present invention also contemplates the use of the high copy number host cells to produce variants of proteins so that the activity of the variants can be compared. In some embodiments, the variants differ by a single nucleotide polymorphism (SNP) causing a single amino acid difference. In other embodiments, the variants contain multiple amino acid substitutions. In some embodiments, the activity of the variant proteins are assayed in vivo or in cell extracts. In other embodiments, the proteins are purified and assayed in vitro. It is also contemplated that in some embodiments the variant proteins are fused to a sequence that allows easy purification (*e.g.,* a his-tag sequence) or to a reporter gene (*e.g.,* green fluorescent protein). Activity of the proteins may be assayed by appropriate methods known in the art (*e.g.*, conversion of a substrate to a product). In some preferred embodiments, the activity of a wild-type protein is determined, and the activity of variant versions of the wild-type proteins are expressed as a percentage of the activity of the wild-type protein. Furthermore, the intracellular activity of variant proteins may be compared by constructing a plurality of host cells lines, each of which expresses a different variant of the wild-type protein. The activity of the variant proteins (*e.g.*, variants of proteins involved in signal transduction pathways) may then be compared using the reporter systems for second messenger assays described above. Therefore, in some embodiments, the direct or indirect response (*e.g.*, through downstream or upstream activation of signal transduction pathway) of variant proteins to stimulation or binding by agonists or antagonists is compared. In some preferred embodiments, the response of a wild-type protein is determined, and the responses of variant versions of the wild-type proteins are expressed as a percentage of the response of the wild-type protein.

### EXPERIMENTAL

The following examples serve to illustrate certain preferred embodiments and aspects of the present invention and are not to be construed as limiting the scope thereof.

In the experimental disclosure which follows, the following abbreviations apply: M (molar); mM (millimolar); µM (micromolar); nM (nanomolar); mol (moles); mmol (millimoles); µmol (micromoles); nmol (nanomoles); gm (grams); mg (milligrams); µg (micrograms);pg (picograms); L (liters); ml (milliliters); µl (microliters); cm (centimeters); mm (millimeters); µm (micrometers); nm (nanometers); °C (degrees Centigrade); AMP (adenosine 5'-monophosphate); BSA (bovine serum albumin); cDNA (copy or complimentary DNA); CS (calf serum); DNA (deoxyribonucleic acid); ssDNA (single stranded DNA); dsDNA (double stranded DNA); dNTP (deoxyribonucleotide triphosphate); LH (luteinizing hormone); NIH (National Institues of Health, Besthesda, MD); RNA (ribonucleic acid); PBS (phosphate buffered saline); g (gravity); OD (optical density); HEPES (N-[2-Hydroxyethyl]piperazine-N-[2-ethanesulfonic acid]); HBS (HEPES buffered saline); PBS (phosphate buffered saline); SDS (sodium dodecylsulfate); Tris-HCl (tris[Hydroxymethyl]aminomethane-hydrochloride); Klenow (DNA polymerase I large (Klenow) fragment); rpm (revolutions per minute); EGTA (ethylene glycol-bis(β-aminoethyl ether) N, N, N', N'-tetraacetic acid); EDTA (ethylenediaminetetracetic acid); bla (β-lactamase or ampicillin-resistance gene); ORI (plasmid origin of replication); lacI (lac repressor); X-gal (5-bromo-4-chloro-3-indolyl-β-D-galactoside); ATCC (American Type Culture Collection, Rockville, MD); GIBCO/BRL (GIBCO/BRL, Grand Island, NY); Perkin-Ehner (Perkin-Elmer, Norwalk, CT); and Sigma (Sigma Chemical Company, St. Louis, MO).

### Example 1

### Vector Construction

The following Example describes the construction of vectors used in the experiments below.

### A. CMV MN14

The CMV MN14 vector (SEQ ID NO:4; MN14 antibody is described in U.S. Pat. No. 5,874,540) comprises the following elements, arranged in 5' to 3' order: CMV promoter; MN14 heavy chain signal peptide, MN14 antibody heavy chain; IRES from encephalomyocarditis virus; bovine α-lactalbumin signal peptide; MN 14 antibody light chain; and 3' MoMuLV LTR. In addition to sequences described in SEQ ID NO: 4, the CMV MN14 vector further comprises a 5' MoMuLV LTR, a MoMuLV extended viral packaging signal, and a neomycin phosphotransferase gene (these additional elements are provided in SEQ ID NO:7; the 5' LTR is derived from Moloney Murine Sarcoma Virus in each of the constructs described herein, but is converted to the MoMuLV 5' LTR when integrated).

This construct uses the 5' MoMuLV LTR to control production of the neomycin phosphotransferase gene. The expression of MN14 antibody is controlled by the CMV promoter. The MN14 heavy chain gene and light chain gene are attached together by an IRES sequence. The CMV promoter drives production of a mRNA containing the heavy chain gene and the light chain gene attached by the IRES. Ribosomes attach to the mRNA at the CAP site and at the IRES sequence. This allows both heavy and light chain protein to be produced from a single mRNA. The mRNA expression from the LTR as well as from the CMV promoter is terminated and poly adenylated in the 3'LTR. The construct was cloned by similar methods as described in section B below.

The IRES sequence (SEQ ID NO:3) comprises a fusion of the IRES from the plasmid pLXIN (Clontech) and the bovine α -lactalbumin signal peptide. The initial ATG of the signal peptide was attached to the IRES to allow the most efficient translation initiation from the IRES. The 3' end of the signal peptide provides a multiple cloning site allowing easy attachment of any protein of interest to create a fusion protein with the signal peptide. The IRES sequence can serve as a translational enhancer as well as creating a second translation initiation site that allows two proteins to be produced from a single mRNA.

The IRES-bovine α -lactalbumin signal peptide was constructed as follows. The portion of the plasmid pLXIN (Clontech, Palo Alto, CA) containing the ECMV IRES was PCR amplified using the following primers.
Primer 1 (SEQ ID NO: 35):
5'GATCCACTAGTAACGGCCGCCAGAATTCGC 3'
Primer 2 (SEQ ID NO: 36):
5' CAGAGAGACAAAGGAGGCCATATTATCATCGTGTTTTTCAAAG 3'

Primer 2 attaches a tail corresponding to the start of the bovine α -lactalbumin signal peptide coding region to the IRES sequence. In addition, the second triplet codon of the α-lactalbumin signal peptide was mutated from ATG to GCC to allow efficient translation from the IRES sequence. This mutation results in a methionine to alanine change in the protein sequence. This mutation was performed because the IRES prefers an alanine as the second amino acid in the protein chain. The resulting IRES PCR product contains an EcoRI site on the 5' end of the fragment (just downstream of Primer 1 above).

Next, the α-lactalbumin signal peptide containing sequence was PCR amplified from the α-LA Signal Peptide vector construct using the following primers.
Primer 3 (SEQ ID NO: 14):
5' CTTTGAAAAACACGATGATAATATGGCCTCCTTTGTCTCTCTG 3'
Primer 4 (SEQ ID NO: 15):
5'TTCGCGAGCTCGAGATCTAGATATCCCATG 3'

Primer 3 attaches a tail corresponding to the 3' end of the IRES sequence to the α-lactalbumin signal peptide coding region. As stated above, the second triplet codon of the bovine α-lactalbumin signal peptide was mutated to allow efficient translation from the IRES sequence. The resulting signal peptide PCR fragment contains NaeI, NcoI, EcoRV, XbaI, BglII and XhoI sites on the 3' end.

After the IRES and signal peptide were amplified individually using the primers shown above, the two reaction products were mixed and PCR was performed using primer 1 and primer 4. The resultant product of this reaction is a spliced fragment that contains the IRES attached to the full length α-lactalbumin signal peptide. The ATG encoding the start of the signal peptide is placed at the same location as the ATG encoding the start of the neomycin phosphotransferase gene found in the vector pLXIN. The fragment also contains the EcoRI site on the 5' end and NaeI, NcoI, EcoRV, XbaI, BglII and XhoI sites on the 3' end.

The spliced IRES/ α-lactalbumin signal peptide PCR fragment was digested with EcoRI and XhoI. The α-LA Signal Peptide vector construct was also digested with EcoRI and XhoI. These two fragments were ligated together to give the pIRES construct.

The IRES/ α-lactalbumin signal peptide portion of the pIRES vector was sequenced and found to contain mutations in the 5' end of the IRES. These mutations occur in a long stretch of C's and were found in all clones that were isolated.

To repair this problem, pLXIN DNA was digested with EcoRI and BsmFI. The 500bp band corresponding to a portion of the IRES sequence was isolated. The mutated IRES/ α-lactalbumin signal peptide construct was also digested with EcoRI and BsmFI and the mutated IRES fragment was removed. The IRES fragment from pLXIN was then substituted for the IRES fragment of the mutated IRES/ α-lactalbumin signal peptide construct. The IRES/ α-LA signal peptide portion of resulting plasmid was then verified by DNA sequencing.

The resulting construct was found to have a number of sequence differences when compared to the expected pLXIN sequence obtained from Clontech. The IRES portion of pLXIN purchased from Clontech was sequenced to verify its sequence. The differences from the expected sequence also appear to be present in the pLXIN plasmid obtained from Clontech. Four sequence differences were identified:
bp 347 T - was G in pLXIN sequence
bp 786-788 ACG - was GC in LXIN sequence.

### B. CMV LL2

The CMV LL2 (SEQ ID NO:5; LL2 antibody is described in U.S. Pat. No. 6,187,287) construct comprises the following elements, arranged in 5' to 3' order: 5' CMV promoter (Clonetech), LL2 heavy chain signal peptide, LL2 antibody heavy chain; IRES from encephalomyocarditis virus; bovine α-LA signal peptide; LL2 antibody light chain; and 3' MoMuLV LTR. In addition to sequences described in SEQ ID NO:5, the CMV LL2 vector further comprises a 5' MoMuLV LTR, a MoMuLV extended viral packaging signal, and a neomycin phosphotransferase gene (these additional elements are provided in SEQ ID NO:7).

This construct uses the 5' MoMuLV LTR to control production of the neomycin phosphotransferase gene. The expression of LL2 antibody is controlled by the CMV promoter (Clontech). The LL2 heavy chain gene and light chain gene are attached together by an IRES sequence. The CMV promoter drives production of a mRNA containing the heavy chain gene and the light chain gene attached by the IRES. Ribosomes attach to the mRNA at the CAP site and at the IRES sequence. This allows both heavy and light chain protein to be produced from a single mRNA. The mRNA expression from the LTR as well as from the CMV promoter is terminated and poly adenylated in the 3' LTR.

The IRES sequence (SEQ ID NO:3) comprises a fusion of the IRES from the plasmid pLXIN (Clontech) and the bovine alpha-lactalbumin signal peptide. The initial ATG of the signal peptide was attached to the IRES to allow the most efficient translation initiation from the IRES. The 3' end of the signal peptide provides a multiple cloning site allowing easy attachment of any protein of interest to create a fusion protein with the signal peptide. The IRES sequence can serve as a translational enhancer as well as creating a second translation initiation site that allows two proteins to be produced from a single mRNA.

The LL2 light chain gene was attached to the IRES α-lactalbumin signal peptide as follows. The LL2 light chain was PCR amplified from the vector pCRLL2 using the following primers.
Primer 1 (SEQ ID NO: 16):
5'CTACAGGTGTCCACGTCGACATCCAGCTGACCCAG3'
Primer 2 (SEQ ID NO: 17):
5'CTGCAGAATAGATCTCTAACACTCTCCCCTGTTG3'

These primers add a HincII site right at the start of the coding region for mature LL2 light chain. Digestion of the PCR product with HincII gives a blunt end fragment starting with the initial GAC encoding mature LL2 on the 5' end. Primer 2 adds a BglII site to the 3' end of the gene right after the stop codon. The resulting PCR product was digested with HincII and BglII and cloned directly into the IRES-Signal Peptide plasmid that was digested with NaeI and BglII.

The Kozak sequence of the LL2 heavy chain gene was then modified. The vector pCRMN14HC was digested with XhoI and AvrII to remove about a 400 bp fragment. PCR was then used to amplify the same portion of the LL2 heavy chain construct that was removed by the XhoI-AvrII digestion. This amplification also mutated the 5' end of the gene to add a better Kozak sequence to the clone. The Kozak sequence was modified to resemble the typical IgG Kozak sequence. The PCR primers are shown below.
Primer 1 (SEQ ID NO: 18):
5'CAGTGTGATCTCGAGAATTCAGGACCTCACCATGGGATGGAGCTGTATCAT 3'
Primer 2 (SEQ ID NO: 19):
5'AGGCTGTATTGGTGGATTCGTCT 3'

The PCR product was digested with XhoI and AvrII and inserted back into the previously digested plasmid backbone.

The "good" Kozak sequence was then added to the light chain gene. The "good" Kozak LL2 heavy chain gene construct was digested with EcoRI and the heavy chain gene containing fragment was isolated. The IRES α -Lactalbumin Signal Peptide LL2 light chain gene construct was also digested with EcoRI. The heavy chain gene was then cloned into the EcoRI site of IRES light chain construct. This resulted in the heavy chain gene being placed at the 5' end of the IRES sequence.

Next, a multiple cloning site was added into the LNCX retroviral backbone plasmid. The LNCX plasmid was digested with HindIII and ClaI. Two oligonucleotide primers were produced and annealed together to create an double stranded DNA multiple cloning site. The following primers were annealed together.
Primer 1 (SEQ ID NO: 20):
5'AGCTTCTCGAGTTAACAGATCTAGGCCTCCTAGGTCGACAT 3'
Primer 2 (SEQ ID NO: 21): 5'
CGATGTCGACCTAGGAGGCCTAGATCTGTTAACTCGAGA 3'
After annealing, the multiple cloning site was ligated into LNCX to create LNC-MCS.

Next, the double chain gene fragment was ligated into the retroviral backbone gene construct. The double chain gene construct created above was digested with SalI and BglII and the double chain-containing fragment was isolated. The retroviral expression plasmid LNC-MCS was digested with XhoI and BglII. The double chain fragment was then cloned into the LNC-MCS retroviral expression backbone.

Next, an RNA splicing problem in the construct was corrected. The construct was digested with NsiI. The resulting fragment was then partially digested with EcoRI. The fragments resulting from the partial digest that were approximately 9300 base pairs in size were gel purified. A linker was created to mutate the splice donor site at the 3' end of the LL2 heavy chain gene. The linker was again created by annealing two oligonucleotide primers together to form the double stranded DNA linker. The two primers used to create the linker are shown below.
Primer 1 (SEQ ID NO: 22): 
5’CGAGGCTCTGCACAACCACTACACGCAGAAGAGCCTCTCCCTGTCTCCCGG
GAAATGAAAGCCG 3’
 Primer 2 (SEQ ID NO: 23):

5’AATTCGGCTTTCATTTCCCGGAGACAGGGAGAGGCTCTTCTGCGTGTAGTGGTTG
TGCAGAGCCTCGTGCA 3’

After annealing the linker was substituted for the original NsiI/EcoRI fragment that was removed during the partial digestion.

### C. MMTV MN14

The MMTV MN14 (SEQ ID NO:6) construct comprises the following elements, arranged in 5' to 3' order: 5' MMTV promoter; double mutated PPE sequence; MN 14 antibody heavy chain; IRES from encephalomyocarditis virus; bovine α LA signal peptide MN 14 antibody light chain; WPRE sequence; and 3' MoMuLV LTR. In addition to the sequences described in SEQ ID NO:6, the MMTV MN14 vector further comprises a MoMuLV LTR, MoMuLV extended viral packaging signal; neomycin phosphotransferase gene located 5' of the MMTV promoter (these additional elements are provided in SEQ ID NO: 7).

This construct uses the 5' MoMuLV LTR to control production of the neomycin phosphotransferase gene. The expression of MN14 antibody is controlled by the MMTV promoter (Pharmacia). The MN14 heavy chain gene and light chain gene are attached together by an IRES/ bovine α-LA signal peptide sequence (SEQ ID NO: 3). The MMTV promoter drives production of a mRNA containing the heavy chain gene and the light chain gene attached by the IRES/bovine α-LA signal peptide sequence. Ribosomes attach to the mRNA at the CAP site and at the IRES/ bovine α-LA signal peptide sequence. This allows both heavy and light chain protein to be produced from a single mRNA. In addition, there are two genetic elements contained within the mRNA to aid in export of the mRNA from the nucleus to the cytoplasm and aid in poly-adenylation of the mRNA. The PPE sequence is contained between the RNA CAP site and the start of the MN14 protein coding region, the WPRE is contained between the end of MN14 protein coding and the poly-adenylation site. The mRNA expression from the LTR as well as from the MMTV promoter is terminated and poly-adenylated in the 3' LTR.

ATG sequences within the PPE element (SEQ ID NO:2) were mutated to prevent potential unwanted translation initiation. Two copies of this mutated sequence were used in a head to tail array. This sequence is placed just downstream of the promoter and upstream of the Kozak sequence and signal peptide-coding region. The WPRE is isolated from woodchuck hepatitis virus and also aids in the export of mRNA from the nucleus and creating stability in the mRNA. If this sequence is included in the 3' untranslated region of the RNA, level of protein expression from this RNA increases up to 10-fold.

### D. α-LA MN14

The α -LA MN14 (SEQ ID NO:7) construct comprises the following elements, arranged in 5' to 3' order: 5' MoMuLV LTR, MoMuLV extended viral packaging signal, neomycin phosphotransferase gene, bovine/human alpha-lactalbumin hybrid promoter, double mutated PPE element, MN14 heavy chain signal peptide, MN14 antibody heavy chain, IRES from encephalomyocarditis virus/bovine α LA signal peptide, MN14 antibody light chain, WPRE sequence; and 3' MoMuLV LTR.

This construct uses the 5' MoMuLV LTR to control production of the neomycin phosphotransferase gene. The expression of MN14 antibody is controlled by the hybrid α-LA promoter (SEQ ID NO:1). The MN14 heavy chain gene and light chain gene are attached together by an IRES sequence/ bovine α -LA signal peptide (SEQ ID NO:3). The α -LA promoter drives production of a mRNA containing the heavy chain gene and the light chain gene attached by the IRES. Ribosomes attach to the mRNA at the CAP site and at the IRES sequence. This allows both heavy and light chain protein to be produced from a single mRNA.

In addition, there are two genetic elements contained within the mRNA to aid in export of the mRNA from the nucleus to the cytoplasm and aid in poly-adenylation of the mRNA. The mutated PPE sequence (SEQ ID NO:2) is contained between the RNA CAP site and the start of the MN14 protein coding region. ATG sequences within the PPE element (SEQ ID NO:2) were mutated to prevent potential unwanted translation initiation. Two copies of this mutated sequence were used in a head to tail array. This sequence is placed just downstream of the promoter and upstream of the Kozak sequence and signal peptide-coding region. The WPRE was isolated from woodchuck hepatitis virus and also aids in the export of mRNA from the nucleus and creating stability in the mRNA. If this sequence is included in the 3' untranslated region of the RNA, level of protein expression from this RNA increases up to 10-fold. The WPRE is contained between the end of MN14 protein coding and the poly-adenylation site. The mRNA expression from the LTR as well as from the bovine/human alpha-lactalbumin hybrid promoter is terminated and poly adenylated in the 3' LTR.

The bovine/human alpha-lactalbumin hybrid promoter (SEQ ID NO: 1) is a modular promoter /enhancer element derived from human and bovine alpha-lactalbumin promoter sequences. The human portion of the promoter is from +15 relative to transcription start point (tsp) to -600 relative to the tsp. The bovine portion is then attached to the end of the human portion and corresponds to -550 to -2000 relative to the tsp. The hybrid was developed to remove poly-adenylation signals that were present in the bovine promoter and hinder retroviral RNA production. It was also developed to contain genetic control elements that are present in the human gene, but not the bovine.

For construction of the bovine/human α-lactalbumin promoter, human genomic DNA was isolated and purified. A portion of the human α-lactalbumin promoter was PCR amplified using the following two primers:
Primer 1 (SEQ ID NO: 24):
   5'AAAGCATATGTTCTGGGCCTTGTTACATGGCTGGATTGGTT3'
Primer 2 (SEQ ID NO: 25):

5'TGAATTCGGCGCCCCCAAGAACCTGAAATGGAAGCATCACTC
AGTTTCATATAT 3'

These two primers created a NdeI site on the 5' end of the PCR fragment and a EcoRI site on the 3' end of the PCR fragment.

The human PCR fragment created using the above primers was double digested with the restriction enzymes NdeI and EcoRI. The plasmid pKBaP-1 was also double digested with NdeI and EcoRI. The plasmid pKBaP-1 contains the bovine α -lactalbumin 5' flanking region attached to a multiple cloning site. This plasmid allows attachment of various genes to the bovine α -lactalbumin promoter.

Subsequently, the human fragment was ligated/substituted for the bovine fragment of the promoter that was removed from the pKBaP-1 plasmid during the double digestion. The resulting plasmid was confirmed by DNA sequencing to be a hybrid of the Bovine and Human α-lactalbumin promoter/regulatory regions.

Attachment of the MN14 light chain gene to the IRES α-lactalbumin signal peptide was accomplished as follows. The MN14 light chain was PCR amplified from the vector pCRMN14LC using the following primers.
Primer 1 (SEQ ID NO: 26): 5' CTACAGGTGTCCACGTCGACATCCAGCTGACCCAG 3'
Primer 2 (SEQ ID NO: 27): 5' CTGCAGAATAGATCTCTAACACTCTCCCCTGTTG 3'

These primers add a HincII site right at the start of the coding region for mature MN14 light chain. Digestion of the PCR product with HincII gives a blunt end fragment starting with the initial GAC encoding mature MN14 on the 5' end. Primer 2 adds a BglII site to the 3' end of the gene right after the stop codon. The resulting PCR product was digested with HincII and BglII and cloned directly into the IRES-Signal Peptide plasmid that was digested with NaeI and BglII.

Next, the vector pCRMN14HC was digested with XhoI and NruI to remove about a 500 bp fragment. PCR was then used to amplify the same portion of the MN14 heavy chain construct that was removed by the XhoI-NruI digestion. This amplification also mutated the 5' end of the gene to add a better Kozak sequence to the clone. The Kozak sequence was modified to resemble the typical IgG Kozak sequence. The PCR primers are shown below.
Primer 1 (SEQ ID NO: 28):
5'CAGTGTGATCTCGAGAATTCAGGACCTCACCATGGGATGGAGCTGTATCAT 3'
Primer 2 (SEQ ID NO: 29):
5'GTGTCTTCGGGTCTCAGGCTGT 3'

The PCR product was digested with XhoI and NruI and inserted back into the previously digested plasmid backbone.

Next, the "good" Kozak MN14 heavy chain gene construct was digested with EcoRI and the heavy chain gene containing fragment was isolated. The IRES α-Lactalbumin Signal Peptide MN14 light chain gene construct was also digested with EcoRI. The heavy chain gene was then cloned into the EcoRI site of IRES light chain construct. This resulted in the heavy chain gene being placed at the 5' end of the IRES sequence.

A multiple cloning site was then added to the LNCX retroviral backbone plasmid. The LNCX plasmid was digested with HindIII and ClaI. Two oligonucleotide primers were produced and annealed together to create an double stranded DNA multiple cloning site. The following primers were annealed together.
Primer 1 (SEQ ID NO: 30):
5'AGCTTCTCGAGTTAACAGATCTAGGCCTCCTAGGTCGACAT3'
Primer 2 (SEQ ID NO: 31):
5' CGATGTCGACCTAGGAGGCCTAGATCTGTTAACTCGAGA 3'
After annealing the multiple cloning site was ligated into LNCX to create LNC-MCS.

The double chain gene fragment was then inserted into a retroviral backbone gene construct. The double chain gene construct created in step 3 was digested with SalI and BglII and the double chain containing fragment was isolated. The retroviral expression plasmid LNC-MCS was digested with XhoI and BglII. The double chain fragment was then cloned into the LNC-MCS retroviral expression backbone.

Next, a RNA splicing problem in the construct was repaired. The construct was digested with NsiI. The resulting fragment was then partially digested with EcoRI. The fragments resulting from the partial digest that were approximately 9300 base pairs in size, were gel purified. A linker was created to mutate the splice donor site at the 3' end of the MN14 heavy chain gene. The linker was again created by annealing two oligonucleotide primers together to form the double stranded DNA linker. The two primers used to create the linker are shown below.
Primer 1 (SEQ ID NO: 32): 
5’CGAGGCTCTGCACAACCACTACACGCAGAAGAGCCTCTCCCTGTCTCCCGGGAAA
TGAAAGCCG 3’
 Primer 2 (SEQ ID NO: 33):

5’AATTCGGCTTTCATTTCCCGGGAGACAGGGAGAGGCTCTTCTGCGTGTAGTGGTTG
TGCAGAGCCTCGTGCA 3’

After annealing the linker was substituted for the original NsiI/EcoRI fragment that was removed during the partial digestion.

Next, the mutated double chain fragment was inserted into the α-Lactalbumin expression retroviral backbone LN α-LA-Mertz-MCS. The gene construct produced above was digested with BamHI and BglII and the mutated double chain gene containing fragment was isolated. The LN α-LA-Mertz-MCS retroviral backbone plasmid was digested with BglII. The BamHI/BglII fragment was then inserted into the retroviral backbone plasmid.

A WPRE element was then inserted into the gene construct. The plasmid BluescriptII SK+ WPRE-B11 was digested with BamHI and HincII to remove the WPRE element and the element was isolated. The vector created above was digested with BglII and HpaI. The WPRE fragment was ligated into the BglII and HpaI sites to create the final gene construct.

### E. α-LA Bot

The α-LA Bot (SEQ ID NO: 8, botulinum toxin antibody) construct comprises the following elements, arranged in 5' to 3' order: bovine/human alpha-lactalbumin hybrid promoter, mutated PPE element, cc49 signal peptide, botulinum toxin antibody light chain, IRES from encephalomyocarditis virus/ bovine α-LA signal peptide, botulinum toxin antibody heavy chain, WPRE sequence, and 3' MoMuLV LTR. In addition, the α-LA botulinum toxin antibody vector further comprises a 5'MoMuLV LTR, a MoMuLV extended viral packaging signal, and a neomycin phosphotransferase gene (these additional elements are provided in SEQ ID NO: 7).

This construct uses the 5' MoMuLV LTR to control production of the neomycin phosphotransferase gene. The expression of botulinum toxin antibody is controlled by the hybrid α-LA promoter. The botulinum toxin antibody light chain gene and heavy chain gene are attached together by an IRES/ bovine α-LA signal peptide sequence. The bovine/human alpha-lactalbumin hybrid promoter drives production of a mRNA containing the light chain gene and the heavy chain gene attached by the IRES. Ribosomes attach to the mRNA at the CAP site and at the IRES sequence. This allows both light and heavy chain protein to be produced from a single mRNA.

In addition, there are two genetic elements contained within the mRNA to aid in export of the mRNA from the nucleus to the cytoplasm and aid in poly-adenylation of the mRNA. The mutated PPE sequence (SEQ ID NO:2) is contained between the RNA CAP site and the start of the MN14 protein coding region. ATG sequences within the PPE element (SEQ ID NO:2) were mutated to prevent potential unwanted translation initiation. Two copies of this mutated sequence were used in a head to tail array. This sequence was placed just downstream of the promoter and upstream of the Kozak sequence and signal peptide-coding region. The WPRE was isolated from woodchuck hepatitis virus and also aids in the export of mRNA from the nucleus and creating stability in the mRNA. If this sequence is included in the 3' untranslated region of the RNA, level of protein expression from this RNA increases up to 10-fold. The WPRE is contained between the end of MN14 protein coding and the poly-adenylation site. The mRNA expression from the LTR as well as from the bovine/human alpha-lactalbumin hybrid promoter is terminated and poly adenylated in the 3' LTR.

The bovine/human α-lactalbumin hybrid promoter (SEQ ID NO:1) is a modular promoter/enhancer element derived from human and bovine α -lactalbumin promoter sequences. The human portion of the promoter is from +15 relative to transcription start point to -600 relative to the tsp. The bovine portion is then attached to the end of the human portion and corresponds to -550 to -2000 relative to the tsp. The hybrid was developed to remove poly-adenylation signals that were present in the bovine promoter and hinder retroviral RNA production. It was also developed to contain genetic control elements that are present in the human gene, but not the bovine. Likewise, the construct contains control elements present in the bovine but not in the human.

### F. LSRNL

The LSRNL (SEQ ID NO:9) construct comprises the following elements, arranged in 5' to 3' order: 5' MoMuLV LTR, MoMuLV viral packaging signal; hepatitis B surface antigen; RSV promoter; neomycin phosphotransferase gene; and 3' MoMuLV LTR.

This construct uses the 5' MoMuLV LTR to control production of the Hepatitis B surface antigen gene. The expression of the neomycin phosphotransferase gene is controlled by the RSV promoter. The mRNA expression from the LTR as well as from the RSV promoter is terminated and poly adenylated in the 3' LTR.

### G. α -LA cc49IL2

The α-LA cc49IL2 (SEQ ID NO:10; the cc49 antibody is described in U.S. Pat. Nos. 5,512,443; 5,993,813; and 5,892,019) construct comprises the following elements, arranged in 5' to 3' order: 5' bovine/human α-lactalbumin hybrid promoter; cc49-IL2 coding region; and 3' MoMuLV LTR. This gene construct expresses a fusion protein of the single chain antibody cc49 attached to Interleukin-2. Expression of the fusion protein is controlled by the bovine/human α-lactalbumin hybrid promoter.

The bovine/human α -lactalbumin hybrid promoter (SEQ ID NO:1) is a modular promoter/enhancer element derived from human and bovine alpha-lactalbumin promoter sequences. The human portion of the promoter is from +15 relative to transcription start point to -600 relative to the tsp. The bovine portion is then attached to the end of the human portion and corresponds to -550 to -2000 relative to the tsp. The hybrid was developed to remove poly-adenylation signals that were present in the bovine promoter and hinder retroviral RNA production. It was also developed to contain genetic control elements that are present in the human gene, but not the bovine. Likewise, the construct contains control elements present in the bovine but not in the human. The 3' viral LTR provide the poly-adenylation sequence for the mRNA.

### H. α -LA YP

The α-LA YP (SEQ ID NO: 11) construct comprises the following elements, arranged in 5' to 3' order: 5' bovine/human alpha-lactalbumin hybrid promoter; double mutated PPE sequence; bovine α-LA signal peptide; Yersenia pestis antibody heavy chain Fab coding region; EMCV IRES/ bovine α-LA signal peptide; Yersenia pestis antibody light chain Fab coding region; WPRE sequence; 3' MoMuLV LTR.

This gene construct will cause the expression of Yersenia pestis mouse Fab antibody. The expression of the gene construct is controlled by the bovine/human α-lactalbumin hybrid promoter. The PPE sequence and the WPRE sequence aid in moving the mRNA from the nucleus to the cytoplasm. The IRES sequence allows both the heavy and the light chain genes to be translated from the same mRNA. The 3' viral LTR provides the poly-adenylation sequence for the mRNA.

In addition, there are two genetic elements contained within the mRNA to aid in export of the mRNA from the nucleus to the cytoplasm and aid in poly-adenylation of the mRNA. The mutated PPE sequence (SEQ ID NO:2) is contained between the RNA CAP site and the start of the MN14 protein coding region. ATG sequences within the PPE element (SEQ ID NO:2) were mutated (bases 4, 112, 131, and 238 of SEQ ID NO: 2 were changed from a G to a T) to prevent potential unwanted translation initiation. Two copies of this mutated sequence were used in a head to tail array. This sequence was placed just downstream of the promoter and upstream of the Kozak sequence and signal peptide-coding region. The WPRE was isolated from woodchuck hepatitis virus and also aids in the export of mRNA from the nucleus and creating stability in the mRNA. If this sequence is included in the 3' untranslated region of the RNA, level of protein expression from this RNA increases up to 10-fold. The WPRE is contained between the end of MN14 protein coding and the poly-adenylation site. The mRNA expression from the LTR as well as from the bovine/human alpha-lactalbumin hybrid promoter is terminated and poly adenylated in the 3' LTR.

The bovine/human alpha-lactalbumin hybrid promoter (SEQ ID NO: 1) is a modular promoter /enhancer element derived from human and bovine alpha-lactalbumin promoter sequences. The human portion of the promoter is from +15 relative to transcription start point to -600 relative to the tsp. The bovine portion is then attached to the end of the human portion and corresponds to -550 to -2000 relative to the tsp. The hybrid was developed to remove polyadenylation signals that were present in the bovine promoter and hinder retroviral RNA production. It was also developed to contain genetic control elements that are present in the human gene, but not the bovine. Likewise, the construct contains control elements present in the bovine but not in the human.

### Example 2

### Generation of Cell Lines Stably Expressing the MoMLV gag and pol Proteins

Examples 2-5 describe the production of pseudotyped retroviral vectors. These methods are generally applicable to the production of the vectors described above. The expression of the fusogenic VSV G protein on the surface of cells results in syncytium formation and cell death. Therefore, in order to produce retroviral particles containing the VSV G protein as the membrane-associated protein a two-step approach was taken. First, stable cell lines expressing the gag and pol proteins from MoMLV at high levels were generated (e.g., 293GP^{SD} cells). The stable cell line which expresses the gag and pol proteins produces noninfectious viral particles lacking a membrane-associated protein (e.g., an envelope protein). The stable cell line was then co-transfected, using the calcium phosphate precipitation, with VSV-G and gene of interest plasmid DNAs. The pseudotyped vector generated was used to infect 293GP^{SD} cells to produce stably transformed cell lines. Stable cell lines can be transiently transfected with a plasmid capable of directing the high level expression of the VSV G protein (see below). The transiently transfected cells produce VSV G-pseudotyped retroviral vectors, which can be collected from the cells over a period of 3 to 4 days before the producing cells die as a result of syncytium formation.

The first step in the production of VSV G-pseudotyped retroviral vectors, the generation of stable cell lines expressing the MoMLV gag and pol proteins is described below. The human adenovirus Ad-5-transformed embryonal kidney cell line 293 (ATCC CRL 1573) was cotransfected with the pCMVgag-pol and the gene encoding for phleomycin. pCMV gag-pol contains the MoMLV gag and pol genes under the control of the CMV promoter (pCMV gag-pol is available from the ATCC).

The plasmid DNA was introduced into the 293 cells using calcium phosphate co-precipitation (Graham and Van der Eb, Virol. 52:456 [1973]). Approximately 5 x 10⁵ 293 cells were plated into a 100 mm tissue culture plate the day before the DNA co-precipitate was added. Stable transformants were selected by growth in DMEM-high glucose medium containing 10% FCS and 10 µg/ml phleomycin (selective medium). Colonies which grew in the selective medium were screened for extracellular reverse transcriptase activity (Goff et al., J. Virol. 38:239 [1981]) and intracellular p30gag expression. The presence of p30gag expression was determined by Western blotting using a goat-anti p30 antibody (NCI antiserum 77S000087). A clone which exhibited stable expression of the retroviral genes was selected. This clone was named 293GP^{SD} (293 gag-pol-San Diego). The 293GP^{SD} cell line, a derivative of the human Ad-5-transformed embryonal kidney cell line 293, was grown in DMEM-high glucose medium containing 10% FCS.

### Example 3

### Preparation of Pseudotyped Retroviral Vectors Bearing the G Glycoprotein of VSV

In order to produce VSV G protein pseudotyped retrovirus the following steps were taken. The 293GP^{SD} cell line was co-transfected with VSV-G plasmid and DNA plasmid of interest. This co-transfection generates the infectious particles used to infect 293GP^{SD} cells to generate the packaging cell lines. This Example describes the production of pseudotyped LNBOTDC virus. This general method may be used to produce any of the vectors described in Example 1.

### a) Cell Lines and Plasmids

The packaging cell line, 293GP^{SD} was grown in alpha-MEM-high glucose medium containing 10% FCS The titer of the pseudo-typed virus may be determined using either 208F cells (Quade, Virol. 98:461 [1979]) or NIH/3T3 cells (ATCC CRL 1658); 208F and NIH/3T3 cells are grown in DMEM-high glucose medium containing 10% CS.

The plasmid LNBOTDC contains the gene encoding neomycin phosphotransferase (Neo) under the transcriptional control of the LTR promoter followed by the gene encoding BOTD under the transcriptional control of cytomegalovirus intermediate-early promoter. The plasmid pHCMV-G contains the VSV G gene under the transcriptional control of the human cytomegalovirus intermediate-early promoter (Yee et al., Meth. Cell Biol. 43:99 [1994]).

### b) Production of stable packaging cell lines, pseudotyped vector and Titering of Pseudotyped LNBOTDC Vector

LNBOTDC DNA (SEQ ID NO: 13) was co-transfected with pHCMV-G DNA into the packaging line 293GP^{SD} to produce LNBOTDC virus. The resulting LNBOTDC virus was then used to infect 293GP^{SD} cells to transform the cells. The procedure for producing pseudotyped LNBOTDC virus was carried out as described (Yee et al., Meth. Cell Biol. 43:99 [1994].

This is a retroviral gene construct that upon creation of infectious replication defective retroviral vector will cause the insertion of the sequence described above into the cells of interest. Upon insertion the CMV regulatory sequences control the expression of the botulinum toxin antibody heavy and light chain genes. The IRES sequence allows both the heavy and the light chain genes to be translated from the same mRNA. The 3' viral LTR provides the poly-adenylation sequence for the mRNA.

Both heavy and light chain protein for botulinum toxin antibody are produced from this signal mRNA. The two proteins associated to form active botulinum toxin antibody. The heavy and light chain proteins also appear to be formed in an equal molar ratio to each other.

Briefly, on day 1, approximately 5 x 10⁴ 293GP^{SD} cells were placed in a 75 cm² tissue culture flask. On the following day (day 2), the 293GP^{SD} cells were transfected with 25 µg of pLNBOTDC plasmid DNA and 25 µg of VSV-G plasmid DNA using the standard calcium phosphate co-precipitation procedure (Graham and Van der Eb, Virol. 52:456 [1973]). A range of 10 to 40 µg of plasmid DNA may be used. Because 293GP^{SD} cells may take more than 24 hours to attach firmly to tissue culture plates, the 293GP^{SD} cells may be placed in 75 cm² flasks 48 hours prior to transfection. The transfected 293GP^{SD} cells provide pseudotyped LNBOTDC virus.

On day 3, approximately 1 x 10⁵ 293GP^{SD} cells were placed in a 75 cm² tissue culture flask 24 hours prior to the harvest of the pseudotyped virus from the transfected 293GP^{SD} cells. On day 4, culture medium was harvested from the transfected 2093GP^{SD} cells 48 hours after the application of the pLNBOTDC and VSV-G DNA. The culture medium was filtered through a 0.45 µm filter and polybrene was added to a final concentration of 8 µg/ml. The culture medium containing LNBOTDC virus was used to infect the 293GP^{SD} cells as follows. The culture medium was removed from the 293GP^{SD} cells and was replaced with the LNBOTDC virus containing culture medium. Polybrene was added to the medium following addition to cells. The virus containing medium was allowed to remain on the 293GP^{SD} cells for 24 hours. Following the 16 hour infection period (on day 5), the medium was removed from the 293GP^{SD} cells and was replaced with fresh medium containing 400 µg/ml G418 (GIBCO/BRL). The medium was changed approximately every 3 days until G418-resistant colonies appeared approximately two weeks later.

The G418-resistant 293 colonies were plated as single cells in 96 wells. Sixty to one hundred G418-resistant colonies were screened for the expression of the BOTDC antibody in order to identify high producing clones. The top 10 clones in 96-well plates were transferred 6-well plates and allowed to grow to confluency.

The top 10 clones were then expanded to screen for high titer production. Based on protein expression and titer production, 5 clonal cell lines were selected. One line was designated the master cell bank and the other 4 as backup cell lines. Pseudotyped vector was generated as follows. Approximately 1 x 10⁶ 293GP^{SD}/LNBOTDC cells were placed into a 75cm² tissue culture flask. Twenty-four hours later, the cells were transfected with 25 µg of pHCMV-G plasmid DNA using calcium phosphate co-precipitation. Six to eight hours after the calcium-DNA precipitate was applied to the cells, the DNA solution was replaced with fresh culture medium (lacking G418). Longer transfection times (overnight) were found to result in the detachment of the majority of the 293GP^{SD}/LNBOTDC cells from the plate and are therefore avoided. The transfected 293GP^{SD}/LNBOTDC cells produce pseudotyped LNBOTDC virus.

The pseudotyped LNBOTDC virus generated from the transfected 293GP^{SD}/LNBOTDC cells can be collected at least once a day between 24 and 96 hr after transfection. The highest virus titer was generated approximately 48 to 72 hr after initial pHCMV-G transfection. While syncytium formation became visible about 48 hr after transfection in the majority of the transfected cells, the cells continued to generate pseudotyped virus for at least an additional 48 hr as long as the cells remained attached to the tissue culture plate. The collected culture medium containing the VSV G-pseudotyped LNBOTDC virus was pooled, filtered through a 0.45 µm filter and stored at -80°C or concentrated immediately and then stored at -80°C.

The titer of the VSV G-pseudotyped LNBOTDC virus was then determined as follows. Approximately 5 x 10⁴ rat 208F fibroblasts cells were plated into 6 well plates. Twenty-fours hours after plating, the cells were infected with serial dilutions of the LNBOTDC virus-containing culture medium in the presence of 8 µg/ml polybrene. Twenty four hours after infection with virus, the medium was replaced with fresh medium containing 400 µg/ml G418 and selection was continued for 14 days until G418-resistant colonies became visible. Viral titers were typically about 0.5 to 5.0 x 10⁶ colony forming units (cfu)/ml. The titer of the virus stock could be concentrated to a titer of greater than 10⁹ cfu/ml as described below.

### Example 4

### Concentration of Pseudotyped Retroviral Vectors

The VSV G-pseudotyped LNBOTDC viruses were concentrated to a high titer by one cycle of ultracentrifugation. However, two cycles can be performed for further concentration. The frozen culture medium collected as described in Example 2 which contained pseudotyped LNBOTDC virus was thawed in a 37°C water bath and was then transferred to Oakridge centrifuge tubes (50 ml Oakridge tubes with sealing caps, Nalge Nunc International) previously sterilized by autoclaving. The virus was sedimented in a JA20 rotor (Beckman) at 48,000 x g (20,000 rpm) at 4°C for 120 min. The culture medium was then removed from the tubes in a biosafety hood and the media remaining in the tubes was aspirated to remove the supernatent. The virus pellet was resuspended to 0.5 to 1% of the original volume of culture medium DMEM. The resuspended virus pellet was incubated overnight at 4°C without swirling. The virus pellet could be dispersed with gentle pipetting after the overnight incubation without significant loss of infectious virus. The titer of the virus stock was routinely increased 100- to 300-fold after one round of ultracentrifugation. The efficiency of recovery of infectious virus varied between 30 and 100%.

The virus stock was then subjected to low speed centrifugation in a microfuge for 5 min at 4°C to remove any visible cell debris or aggregated virions that were not resuspended under the above conditions. It was noted that if the virus stock is not to be used for injection into oocytes or embryos, this centrifugation step may be omitted.

The virus stock can be subjected to another round of ultracentrifugation to further concentrate the virus stock. The resuspended virus from the first round of centrifugation is pooled and pelleted by a second round of ultracentrifugation which is performed as described above. Viral titers are increased approximately 2000-fold after the second round of ultracentrifugation (titers of the pseudotyped LNBOTDC virus are typically greater than or equal to 1 x 10⁹ cfu/ml after the second round of ultracentrifugation).

The titers of the pre- and post-centrifugation fluids were determined by infection of 208F cells (NIH 3T3 or bovine mammary epithelial cells can also be employed) followed by selection of G418-resistant colonies as described above in Example 2.

### Example 5

### Preparation of Pseudotyped Retrovirus For Infection of Host Cells

The concentrated pseudotyped retroviruses were resuspended in 0.1X HBS (2.5 mM HEPES, pH 7.12,14 mM NaCl, 75 _M Na₂HPO₄-H₂O) and 18 µl aliquots were placed in 0.5 ml vials (Eppendorf) and stored at -80°C until used. The titer of the concentrated vector was determined by diluting 1 µl of the concentrated virus 10⁻⁷- or 10⁻⁸-fold with 0.1X HBS. The diluted virus solution was then used to infect 208F and bovine mammary epithelial cells and viral titers were determined as described in Example 2.

### Example 6

### Expression of MN14 by Host Cells

This Example describes the production of antibody MN14 from cells transfected with a high number of integrating vectors. Pseudotyped vector were made from the packaging cell lines for the following vectors: CMV MN14, α-LA MN14, and MMTV MN14. Rat fibroblasts (208F cells), MDBK cells (bovine kidney cells), and bovine mammary epithelial cells were transfected at a multiplicity of infection of 1000. One thousand cells were plated in a T25 flask and 10⁶ colony forming units (CFU's) of vector in 3 ml media was incubated with the cells. The duration of the infection was 24 hr, followed by a media change. Following transfection, the cells were allowed to grow and become confluent.

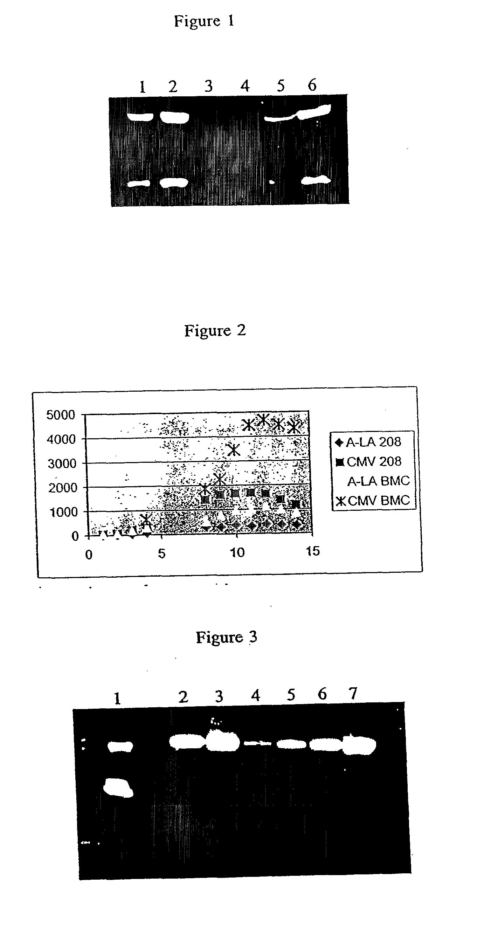

The cell lines were grown to confluency in T25 flasks and 5ml of media was changed daily. The media was assayed daily for the presence of MN14. All of the MN14 produced is active (an ELISA to detect human IgG gave the exact same values as the CEA binding ELISA) and Western blotting has shown that the heavy and light chains are produced at a ratio that appears to be a 1:1 ratio. In addition, a non-denaturing Western blot indicated that what appeared to be 100% of the antibody complexes were correctly formed (See Figure 1: Lane 1, 85 ng control Mn14; Lane 2, bovine mammary cell line, α -LA promoter; Lane 3, bovine mammary cell line, CMV promoter; Lane 4, bovine kidney cell line, α -LA promoter; Lane 5, bovine kidney cell line, CMV promoter; Lane 6, 208 cell line, α -LA promoter; Lane 7, 208 cell line, CMV promoter)).

Figure 2 is a graph showing the production of MN14 over time for four cell lines. The Y axis shows MN14 production in ng/ml of media. The X-axis shows the day of media collection for the experiment. Four sets of data are shown on the graph. The comparisons are between the CMV and α -LA promoter and between the 208 cells and the bovine mammary cells. The bovine mammary cell line exhibited the highest expression, followed by the 208F cells and MDBK cells. With respect to the constructs, the CMV driven construct demonstrated the highest level of expression, followed by the α -LA driven gene construct and the MMTV construct. At 2 weeks, the level of daily production of the CMV construct was 4.5 µg/ml of media (22.5 mg/day in a T25 flask). The level of expression subsequently increased slowly to 40 µg/day as the cells became very densely confluent over the subsequent week. 2.7 L of media from an α-lac-MN14 packaging cell line was processed by affinity chromatography to produce a purified stock of MN14.

Figure 3 is a western blot of a 15% SDS-PAGE gel run under denaturing conditions in order to separate the heavy and light chains of the MN14 antibody. Lane 1 shows MN14 from bovine mammary cell line, hybrid α-LA promoter; lane 2 shows MN14 from bovine mammary cell line, CMV promoter; lane 3 shows MN14 from bovine kidney cell line, hybrid α-LA promoter; lane 4 shows MN14 from bovine kidney cell line, CMV promoter; lane 5 shows MN14 from rat fibroblast cell line, hybrid α-LA promoter; lane 6 shows MN14 from rat fibroblast, CMV promoter. In agreement with Figure 1 above, the results show that the heavy and light chains are produced in a ratio of approximately 1:1.

### Example 7

### Quantitation of Protein Produced Per Cell

This Example describes the quantitation of the amount of protein produced per cell in cell cultures produced according to the invention. Various cells (208F cells, MDBK cells, and bovine mammary cells) were plated in 25 cm² culture dishes at 1000 cells/dish. Three different vectors were used to infect the three cells types (CMV-MN14, MMTV-MN14, and α-LA-MN14) at an MOI of 1000 (titers: 2.8 X 10⁶, 4.9 X 10⁶, and 4.3 X 10⁶, respectively). Media was collected approximately every 24 hours from all cells. Following one month of media collection, the 208F and MDBK cells were discarded due to poor health and low MN14 expression. The cells were passaged to T25 flasks and collection of media from the bovine mammary cells was continued for approximately 2 months with continued expression of MN14. After two months in T25 flasks, the cells with CMV promoters were producing 22.5 pg/cell/day and the cells with α-LA promoters were producing 2.5 pg MN14/cell/day.

After 2 months in T25 flasks, roller bottles (850 cm²) were seeded to scale-up production and to determine if MN14 expression was stable following multiple passages. Two roller bottles were seeded with bovine mammary cells expressing MN14 from a CMV promoter and two roller bottles were seeded with bovine mammary cells expressing MN14 from the α-LA promoter. The cultures reached confluency after approximately two weeks and continue to express MN14. Roller bottle expression is shown in Table 1 below.

| **Table 1** | | | |
|---|---|---|---|
| **Production of MN14 in Roller Bottles** | | | |
| Cell Line | Promoter | MN14 Production/Week (µg/ml) | MN14 Production/Week - Total (µg/ml) |
| Bovine mammary | CMV | 2.6 | 1-520 |
| Bovine mammary | CMV | 10.6 | 2-2120 |
| Bovine mammary | CMV | 8.7 | 3 - 1740 |
| Bovine mammary | CMV | 7.8 | 4 - 1560 |
| Bovine mammary | _-LA | 0.272 | 1 - 54.4 |
| Bovine mammary | _-LA | 2.8 | 2-560 |
| Bovine mammary | _-LA | 2.2 | 3-440 |
| Bovine mammary | _-LA | 2.3 | 4-460 |

### Example 8

### Transfection at Varied Multiplicities of Infection

This Example describes the effect of transfection at varied multiplicities of infection on protein expression. 208F rat fibroblast and bovine mammary epithelial cells (BMEC) were plated in a 25 cm² plates at varied cell numbers/25 cm². Cells were infected with either the CMV MN14 vector or the α LA MN14 vector at a MOI of 1,10, 1000, and 10,000 by keeping the number of CFUs kept constant and varying the number of cells infected.

Following infection, medium was changed daily and collected approximately every 24 hours from all cells for approximately 2 months. The results of both of the vectors in bovine mammary epithelial cells are shown in Table 2 below. Cells without data indicate cultures that became infected prior to the completion of the experiment. The "# cells" column represents the number of cells at the conclusion of the experiment. The results indicate that a higher MOI results in increased MN14 production, both in terms of the amount of protein produced per day, and the total accumulation.

| **Table 2** | | | | | | |
|---|---|---|---|---|---|---|
| **MOI vs. Protein Production** | | | | | | |
| Cell Line | Promoter | MOI | % cell Confluency | MN14 (ng/ml) | # Cells | MN14 Production /day (pg/cell) |
| BMEC | CMV | 10000 | 100% | 4228 | 4.5E5 | 47 |
| BMEC | CMV | 1000 | 100% | 2832 | 2.0E6 | 7.1 |
| BMEC | CMV | 100 | | | | |
| BMEC | CMV | 10 | 100% | 1873 | 2.5E6 | 3.75 |
| BMEC | CMV | 1 | | | | |
| BMEC | _LA | 10000 | 100% | 1024 | 1.5E6 | 3.4 |
| BMEC | _LA | 1000 | | | | |
| BMEC | _LA | 100 | 100% | 722 | 1.8E6 | 1.9 |
| BMEC | _LA | 10 | 100% | 421234 | 2.3E6 | .925 |
| BMEC | _LA | 1 | 100% | | 1.9E6 | .325 |

### Example 9

### Transfection at Varied Multiplicities of Infection

This experiment describes protein production from the CMV MN14 vector at a variety of MOI values. Bovine mammary cells, CHO cells, and human embryo kidney cells (293 cells) were plated in 24 well plates (2 cm²) at 100 cells/2 cm² well. Cells were infected at various dilutions with CMV MN14 to obtain MOI values of 1, 10, 100, 1000, and 10000. The CHO cells reached confluency at all MOI within 11 days of infection. However, the cells infected at a MOI of 10,000 grew more slowly. The bovine mammary and 293 cells grew slower, especially at the highest MOI of 10,000. The cells were then passaged into T25 flasks to disperse cells. Following dispersion, cells reached confluence within 1 week. The medium was collected after one week and analyzed for MN14 production. The CHO and human 293 cells did not exhibit good growth in extended culture. Thus, data were not collected from these cells. Data for bovine mammary epithelial cells are shown in Table 3 below. The results indicate that production of MN14 increased with higher MOI.

| **Table 3** | | | | |
|---|---|---|---|---|
| **MOI vs. Protein Production** | | | | |
| Cell Line | Promoter | MOI | % confluency | MN14 Production (ng/ml) |
| BMEC | CMV | 10000 | 100% | 1312 |
| BMEC | CMV | 1000 | 100% | 100 |
| BMEC | CMV | 100 | 100% | 7.23 |
| BMEC | CMV | 10 | 100% | 0 |
| BMEC | CMV | 1 | 100% | 0 |

### Example 10

### Expression of LL2 Antibody by Bovine Mammary Cells

This Example describes the expression of antibody LL2 by bovine mammary cells. Bovine mammary cells were infected with vector CMV LL2 (7.85 x 10⁷ CFU/ml) at MOI's of 1000 and 10,000 and plated in 25cm² culture dishes. None of the cells survived transfection at the MOI of 10,000. At 20% confluency, 250 ng/ml of LL2 was present in the media.

### Example 11

### Expression of Botulinum Toxin Antibody by Bovine Mammary Cells

This Example describes the expression of Botulinum toxin antibody in bovine mammary cells. Bovine mammary cells were infected with vector α-LA Bot (2.2 X 10² CFU/ml) and plated in 25cm² culture dishes. At 100% confluency, 6 ng/ml of Botulinum toxin antibody was present in the media.

### Example 12

### Expression of Hepatitis B Surface Antigen by Bovine Mammary Cells

This Example describes the expression of hepatitis B surface antigen (HBSAg) in bovine mammary cells. Bovine mammary cells were infected with vector LSRNL (350 CFU/ml) and plated in 25cm² culture dishes. At 100% confluency, 20 ng/ml of HBSAg was present in the media.

### Example 13

### Expression of cc49IL2 Antigen Binding Protein by Bovine Mammary Cells

This Example describes the expression of cc49IL2 in bovine mammary cells. Bovine mammary cells were infected with vector cc49IL2 (3.1 X 10⁵ CFU/ml) at a MOI of 1000 and plated in 25cm² culture dishes. At 100% confluency, 10 µg/ml of cc49IL2 was present in the media.

### Example 14

### Expression of Multiple Proteins by Bovine Mammary Cells

This Example describes the expression of multiple proteins in bovine mammary cells. Mammary cells producing MN14 (infected with CMV-MN14 vector) were infected with cc49IL2 vector (3.1 X 10⁵ CFU/ml) at an MOI of 1000, and 1000 cells were plated in 25cm² culture plates. At 100% confluency, the cells expressed MN14 at 2.5 µg/ml and cc49IL2 at 5 µg/ml.

### Example 15

### Expression of Multiple Proteins by Bovine Mammary Cells

This Example describes the expression of multiple proteins in bovine mammary cells. Mammary cells producing MN14 (infected with CMV-MN14 vector) were infected with LSNRL vector (100 CFU/ml) at an MOI of 1000, and 1000 cells were plated in 25cm² culture plates. At 100% confluency, the cells expressed MN14 at 2.5 µg/ml and hepatitis surface antigen at 150 ng/ml.

### Example 16

### Expression of Multiple Proteins by Bovine Mammary Cells

This Example describes the expression of multiple proteins in bovine mammary cells. Mammary cells producing hepatitis B surface antigen (infected with LSRNL vector) were infected with cc49IL2 vector at an MOI of 1000, and 1000 cells were plated in 25cm² culture plates. At 100% confluency, the cells expressed MN14 at 2.4 µg/ml and hepatitis B surface antigen at 13 ng/ml. It will be understood that multiple proteins may be expressed in the other cell lines described above.

### Example 17

### Expression of Hepatitis B Surface Antigen and Botulinum Toxin Antibody in Bovine Mammary Cells

This Example describes the culture of transfected cells in roller bottle cultures. 208F cells and bovine mammary cells were plated in 25cm² culture dishes at 1000 cells/ 25cm². LSRNL or α-LA Bot vectors were used to infect each cell line at a MOI of 1000. Following one month of culture and media collection, the 208F cells were discarded due to poor growth and plating. Likewise, the bovine mammary cells infected with α -LA Bot were discarded due to low protein expression. The bovine mammary cells infected with LSRNL were passaged to seed roller bottles (850 cm²). Approximately 20 ng/ml hepatitis type B surface antigen was produced in the roller bottle cultures.

### Example 18

### Expression in Clonally Selected Cell Lines

This experiment describes expression of MN14 from clonally selected cell lines. Cell lines were grown to confluency in T25 flasks and 5ml of media were collected daily. The media was assayed daily for the presence of MN14. All the MN14 produced was active and Western blotting indicated that the heavy and light chains were produce at a ratio that appears to be almost exactly 1:1. In addition, a non-denaturing western blot indicated that approximately 100% of the antibody complexes were correctly formed. After being in culture for about two months, the cells were expanded into roller bottles or plated as single cell clones in 96 well plates.

The production of MN14 in the roller bottles was analyzed for a 24 hour period to determine if additional medium changing would increase production over what was obtained with weekly medium changes. Three 24 hour periods were examined. The CMV promoter cells in 850 cm² roller bottles produced 909 ng/ml the first day, 1160 ng/ml the second day and 1112 ng/ml the third day. The α -LA promoter cells produced 401 ng/ml the first day, 477 ng/ml the second day and 463 ng/ml the third day. These values correspond well to the 8-10 mg/ml/week that were obtained for the CMV cells and the 2-3 mg/ml that were obtained for the α -LA cells. It does not appear that more frequent media changing would increase MN14 production in roller bottles.

Single cell lines were established in 96 well plates and then passaged into the same wells to allow the cells to grow to confluency. Once the cells reached confluency, they were assayed for MN14 production over a 24 hour period. The clonal production of MN14 from CMV cell lines ranged from 19 ng/ml/day to 5500 ng/ml/day. The average production of all cell clones was 1984 ng/ml/day. The α-LA cell clones yielded similar results. The clonal production of MN14 from α-LA cell lines ranged from 1 ng/ml/day to 2800 ng/ml/day. The average production of these cell clones was 622 ng/ml/day. The results are provided in Table 4 below.

| **Table 4** | | | |
|---|---|---|---|
| **Expression in Clonal Cell Lines** | | | |
| CMV Clonal Cell Line Number | MN14 Production (ng/ml) | Alpha-lactalbumin Clonal Cell Line Number | MN14 Production (ng/ml) |
| 22 | 19 | 27 | 0 |
| 6 | 88 | 29 | 0 |
| 29 | 134 | 12 | 0.7 |
| 34 | 151 | 50 | 8 |
| 32 | 221 | 28 | 55 |
| 23 | 343 | 43 | 57 |
| 27 | 423 | 8 | 81 |
| 4 | 536 | 13 | 154 |
| 41 | 682 | 48 | 159 |
| 45 | 685 | 7 | 186 |
| 40 | 696 | 36 | 228 |
| 11 | 1042 | 39 | 239 |
| 8 | 1044 | 51 | 275 |
| 5 | 1066 | 31 | 283 |
| 19 | 1104 | 54 | 311 |
| 48 | 1142 | 38 | 317 |
| 12 | 1224 | 21 | 318 |
| 26 | 1315 | 16 | 322 |
| 39 | 1418 | 47 | 322 |
| 37 | 1610 | 17 | 325 |
| 20 | 1830 | 37 | 367 |
| 21 | 1898 | 45 | 395 |
| 47 | 1918 | 25 | 431 |
| 35 | 1938 | 5 | 441 |
| 15 | 1968 | 20 | 449 |
| 3 | 1976 | 19 | 454 |
| 28 | 1976 | 22 | 503 |
| 1 | 2166 | 55 | 510 |
| 16 | 2172 | 14 | 519 |
| 17 | 2188 | 41 | 565 |
| 33 | 2238 | 46 | 566 |
| 30 | 2312 | 23 | 570 |
| 38 | 2429 | 1 | 602 |
| 2 | 2503 | 9 | 609 |
| 14 | 2564 | 53 | 610 |
| 24 | 2571 | 56 | 631 |
| 9 | 2708 | 2 | 641 |
| 42 | 2729 | 40 | 643 |
| 44 | 2971 | 32 | 653 |
| 7 | 3125 | 24 | 664 |
| 43 | 3125 | 26 | 671 |
| 25 | 3650 | 52 | 684 |
| 46 | 3706 | 6 | 693 |
| 50 | 3947 | 33 | 758 |
| 49 | 4538 | 42 | 844 |
| 18 | 4695 | 10 | 1014 |
| 31 | 4919 | 3 | 1076 |
| 10 | 5518 | 44 | 1077 |
| | | 35 | 1469 |
| | | 34 | 1596 |
| | | 18 | 1820 |
| | | 30 | 2021 |
| | | 11 | 2585 |
| | | 4 | 2800 |

### Example 19

### Estimation of Insert Copy Number

This example describes the relationship of multiplicity of infection, gene copy number, and protein expression. Three DNA assays were developed using the INVADER Assay system (Third Wave Technologies, Madison, WI). One of the assays detects a portion of the bovine α-lactalbumin 5' flanking region. This assay is specific for bovine and does not detect the porcine or human α -lactalbumin gene. This assay will detect two copies of the α -lactalbumin gene in all control bovine DNA samples and also in bovine mammary epithelial cells. The second assay detects a portion of the extended packaging region from the MLV virus. This assay is specific for this region and does not detect a signal in the 293 human cell line, bovine mammary epithelial cell line or bovine DNA samples. Theoretically, all cell lines or other samples not infected with MLV should not produce a signal. However, since the 293GP cell line was produced with the extended packaging region of DNA, this cell line gives a signal when the assay is run. From the initial analysis, it appears that the 293GP cell line contains two copies of the extended packing region sequence that are detected by the assay. The final assay is the control assay. This assay detects a portion of the insulin-like growth factor I gene that is identical in bovine, porcine, humans and a number of other species. It is used as a control on every sample that is run in order to determine the amount of signal that is generated from this sample for a two copy gene. All samples that are tested should contain two copies of the control gene.

DNA samples can be isolated using a number of methods. Two assays are then performed on each sample. The control assay is performed along with either the bovine α-lactalbumin assay or the extended packaging region assay. The sample and the type of information needed will determine which assay is run. Both the control and the transgene detection assay are run on the same DNA sample, using the exact same quantity of DNA.

The data resulting from the assay are as follows (Counts indicate arbitrary fluorescence units):
Extended Packaging Region or α -Lactalbumin Background counts
Extended Packaging Region or α -Lactalbumin counts
Internal Control background counts
Internal Control counts

To determine net counts for the assay the background counts are subtracted from the actual counts. This occurs for both the control and transgene detection assay. Once the net counts are obtained, a ratio of the net counts for the transgene detection assay to the net counts of the control assay can be produced. This value is an indication of the number of copies of transgene compared to the number of copies of the internal control gene (in this case IGF-1). Because the transgene detection assay and the control assay are two totally different assays, they do not behave exactly the same. This means that one does not get an exact 1:1 ratio if there are two copies of the transgene and two copies of the control gene in a specific sample. However the values are generally close to the 1:1 ratio. Also, different insertion sites for the transgene may cause the transgene assay to behave differently depending on where the insertions are located.

Therefore, although the ratio is not an exact measure of copy number, it is a good indication of relative copy number between samples. The greater the value of the ratio the greater the copy number of the transgene. Thus, a ranking of samples from lowest to highest will give a very accurate comparison of the samples to one another with regard to copy number. Table 5 provides actual data for the EPR assay:

| **Table 5** | | | | | | | |
|---|---|---|---|---|---|---|---|
| Sample # | Control Counts | Control Background Counts | Net Control Counts | Transgene Counts | Transgene Background Counts | Net Transgene Counts | Net Ratio |
| 293 | 116 | 44 | 72 | 46.3 | 46 | 0.3 | 0 |
| 293GP | 112 | 44 | 68 | 104 | 46 | 58 | .84 |
| 1 | 74 | 40 | 34 | 88 | 41 | 47 | 1.38 |
| 2 | 64 | 40 | 24 | 83 | 41 | 43 | 1.75 |
| 3 | 62 | 44 | 18 | 144 | 46 | 98 | 5.57 |

From this data, it can be determined that the 293 cell line has no copies of the extended packaging region/transgene. However the 293 GP cells appear to have two copies of the extended packaging region. The other three cell lines appear to have three or more copies of the extended packaging region (one or more additional copies compared to 293GP cells).

### Invader Assay Gene Ratio and Cell Line Protein Production

Bovine mammary epithelial cells were infected with either the CMV driven MN14 construct or the α -lactalbumin driven MN14 construct. The cells were infected at a 1000 to 1 vector to cell ratio. The infected cells were expanded. Clonal cell lines were established for both the α -LA and CMV containing cells from this initial pooled population of cells. Approximately 50 cell lines were produced for each gene construct. Individual cells were placed in 96 well plates and then passaged into the same well to allow the cells to grow to confluency. Once the cells lines reached confluency, they were assayed for MN14 production over a 24 hour period. The clonal production of MN14 from CMV cell lines ranged from 0 ng/ml/day to 5500 ng/ml/day. The average production of all cell clones was 1984 ng/ml/day. The α -LA cell clones showed similar trends. The clonal production of MN14 from a -LA cell lines ranged from 0 ng/ml/day to 2800 ng/ml/day. The average production of these cell clones was 622 ng/ml/day.

For further analysis of these clonal lines, fifteen CMV clones and fifteen α -LA clones were selected. Five highest expressing, five low expressing and five mid-level expressing lines were chosen. These thirty cell lines were expanded and banked. DNA was isolated from most all of the thirty cell lines. The cell lines were passed into 6 well plates and grown to confluency. Once at confluency, the media was changed every 24 hours and two separate collections from each cell line were assayed for MN14 production. The results of these two assays were averaged and these numbers were used to create Tables 6 and 7 below. DNA from the cell lines was run using the Invader extended packaging region assay and the results are shown below. The Tables show the cell line number, corresponding gene ratio and antibody production.

| **Table 6** | | |
|---|---|---|
| CMV Clonal Cell Line Number | Invader Gene Ratio | MN14 Production (ng/ml) |
| 6 | 0.19 | 104 |
| 7 | 1.62 | 2874 |
| 10 | 2.57 | 11202 |
| 18 | 3.12 | 7757 |
| 19 | 1.62 | 2483 |
| 21 | 1.53 | 3922 |
| 22 | 0 | 0 |
| 29 | 0.23 | 443 |
| 31 | 3.45 | 5697 |
| 32 | 0.27 | 346 |
| 34 | 0.37 | 305 |
| 38 | 1.47 | 2708 |
| 41 | 1.54 | 5434 |
| 49 | 2.6 | 7892 |
| 50 | 1.56 | 5022 |
| Average of All Clones | 1.48 | 3746 |

| **Table 7** | | |
|---|---|---|
| α-LA Clonal Cell Line Number | Invader Gene Ratio | MN14 Production (ng/ml) |
| 4 | 4.28 | 3600 |
| 6 | 1.15 | 959 |
| 12 | 0.35 | 21 |
| 17 | 0.54 | 538 |
| 28 | 0.75 | 60 |
| 30 | 1.73 | 2076 |
| 31 | 0.74 | 484 |
| 34 | 4.04 | 3332 |
| 41 | 1.33 | 771 |
| Average of All Clones | 1.66 | 1316 |

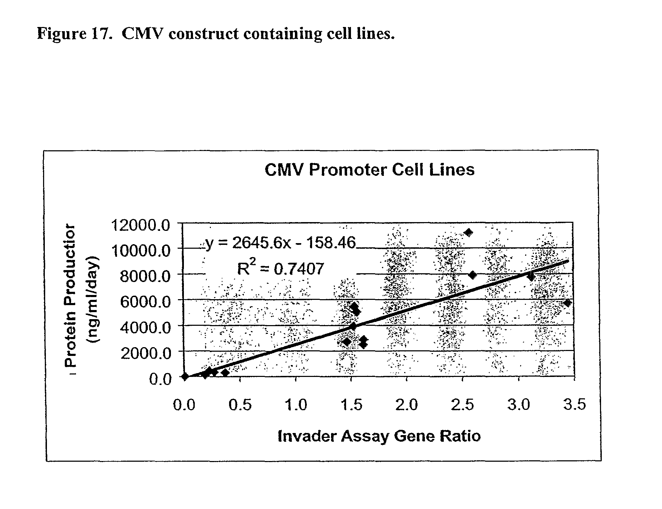

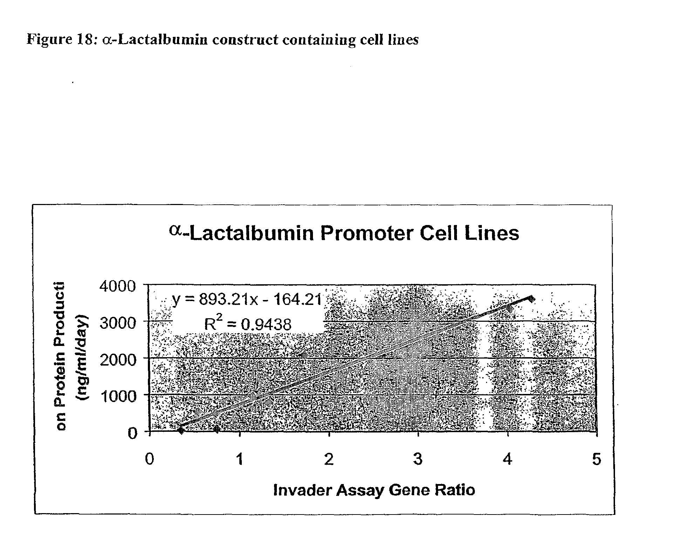

The graphs (Figs. 17 and 18) show the comparison between protein expression and invader assay gene ratio. The results indicate that there is a direct correlation between invader assay gene ratio and protein production. It also appears that the protein production has not reached a maximum and if cells containing a higher invader assay gene ratio were produced, higher protein production would occur.

### Invader Assay Gene Ratio and Multiple Cell Line Infections

Two packaging cell lines (293GP) produced using previously described methods were used to produce replication defective retroviral vector. One of the cell lines contains a retroviral gene construct that expresses the botulinum toxin antibody gene from the CMV promoter (LTR-Extended Viral Packaging Region-Neo Gene-CMV Promoter-Bot Light Chain Gene-IRES-Bot Heavy Chain Gene-LTR), the other cell line contains a retroviral gene construct that expresses the YP antibody gene from the CMV promoter (LTR-Extended Viral Packaging Region-Neo Gene-CMV Promoter-YP Heavy Chain Gene-IRES-YP Light Chain Gene-WPRE-LTR). In addition to being able to produce replication defective retroviral vector, each of these cell lines also produce either botulinum toxin antibody or YP antibody.

The vector produced from these cell lines was then used to re-infect the parent cell line. This procedure was performed in order to increase the number of gene insertions and to improve antibody production from these cell lines. The botulinum toxin parent cell line was infected with a new aliquot of vector on three successive days. The titer of the vector used to perform the infection was 1 X 10⁸ cfu/ml. Upon completion of the final 24 hour infection, clonal selection was performed on the cells and the highest protein producing line was established for botulinum toxin antibody production. A similar procedure was performed on the YP parent cell line. This cell line was also infected with a new aliquot of vector on three successive days. The titer of the YP vector aliquots was 1 X 10⁴. Upon completion of the final 24 hour infection, clonal selection was performed on the cells and the highest protein producing line was established for YP production.

Each of the parent cell lines and the daughter production cell lines were examined for Invader gene ratio using the extended packaging region assay and for protein production. The Bot production cell line, which was generated using the highest titer vector had the highest gene ratio. It also had the highest protein production, again suggesting that gene copy number is proportional to protein production. The YP production cell line also had a higher gene ratio and produced more protein than its parent cell line, also suggesting that increasing gene copy is directly related to increases in protein production. The data is presented in Table 8.

| **Table 8** | | |
|---|---|---|
| Cell Line | Invader Gene Ratio | Antibody Production (Bot/YP) |
| Bot Parent Cell Line | 1.12 | 4.8 µg/ml |
| Bot Production Cell Line | 3.03 | 55 µg/ml |
| YP Parent Cell Line | 1.32 | 4 µg/ml |
| YP Production Cell Line | 2.04 | 25 µg/ml |

### Example 20

### Transfection with Lentivirus Vectors

This example describes methods for the production of lentivirus vectors and their use to infect host cells at a high multiplicity of infection. Replication-defective viral particles are produced by the transient cotransfection of the plasmids described in U.S. Pat. No. 6,013,516 in 293T human kidney cells. All plasmids are transformed and grown in E. coli HB101 bacteria following standard molecular biology procedures. For transfection of eukaryotic cells, plasmid DNA is purified twice by equilibrium centrifugation in CsCl-ethidium bromide gradients. A total of 40 µg DNA is used for the transfection of a culture in a 10 cm dish, in the following proportions: 10 µg pCMVΔR8, 20 µg pHR', and 10 µg env plasmids, either MLV/Ampho, MLV/Eco or VSV-G. 293T cells are grown in DMEM supplemented with 10% fetal calf serum and antibiotics in a 10% CO₂ incubator. Cells are plated at a density of 1.3x10⁶/10 cm dish the day before transfection. Culture medium is changed 4 to 6 hrs before transfection. Calcium phosphate-DNA complexes are prepared according to the method of Chen and Okayama (Mol. Cell. Biol., 7:2745, 1987), and incubated overnight with the cells in an atmosphere of 5% CO₂. The following morning, the medium is replaced, and the cultures returned to 10% CO₂. Conditioned medium is harvested 48 to 60 hrs after transfection, cleared of cellular debris by low speed centrifugation (300µg 10 min), and filtered through 0.45 µm low protein binding filters.

To concentrate vector particles, pooled conditioned medium harvested as described above is layered on top of a cushion of 20% sucrose solution in PBS and centrifuged in a Beckman SW28 rotor at 50,000µg for 90 min. The pellet is resuspended by incubation and gentle pipetting in 1-4 ml PBS for 30-60 min, then centrifuged again at 50,000xg for 90 min in a Beckmann SW55 rotor. The pellet is resuspended in a minimal volume (20-50 µl) of PBS and either used directly for infection or stored in frozen aliquots at -80° C.

The concentrated lentivirus vectors are titered and used to transfect an appropriate cell line (*e*.*g*., 293 cells, Hela cells, rat 208F fibroblasts)) at a multiplicity of infection of 1,000. Analysis of clonally selected cell lines expressing the exogenous protein will reveal that a portion of the selected cell lines contain more than two integrated copies of the vector. These cell lines will produce more of the exogenous protein than cell lines containing only one copy of the integrated vector.

### Example 21

### Expression and Assay of G-protein Coupled Receptors

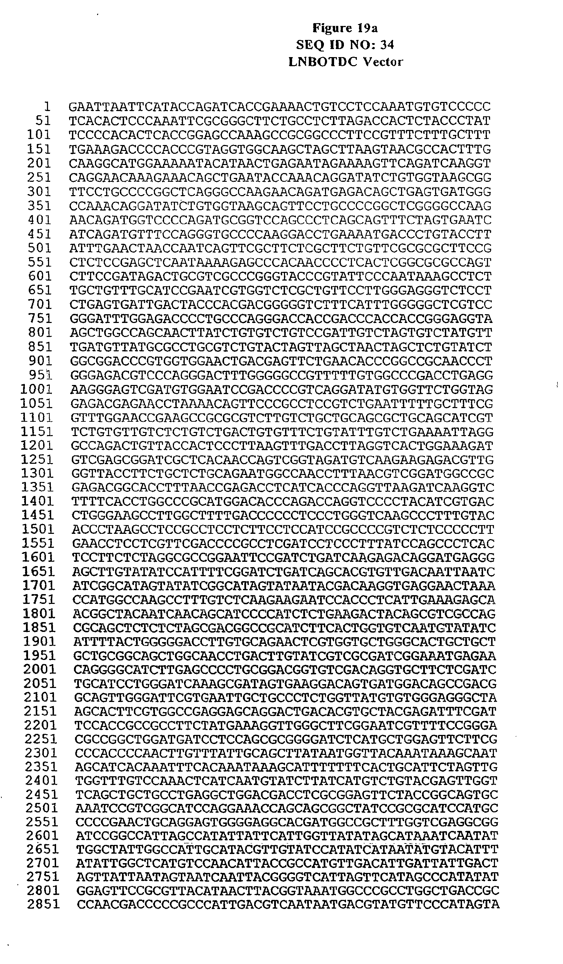

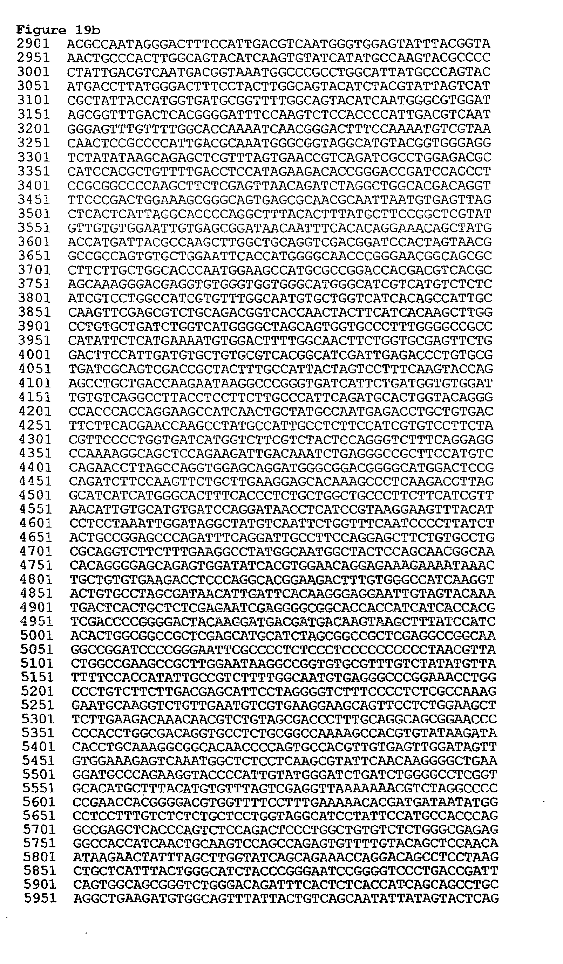

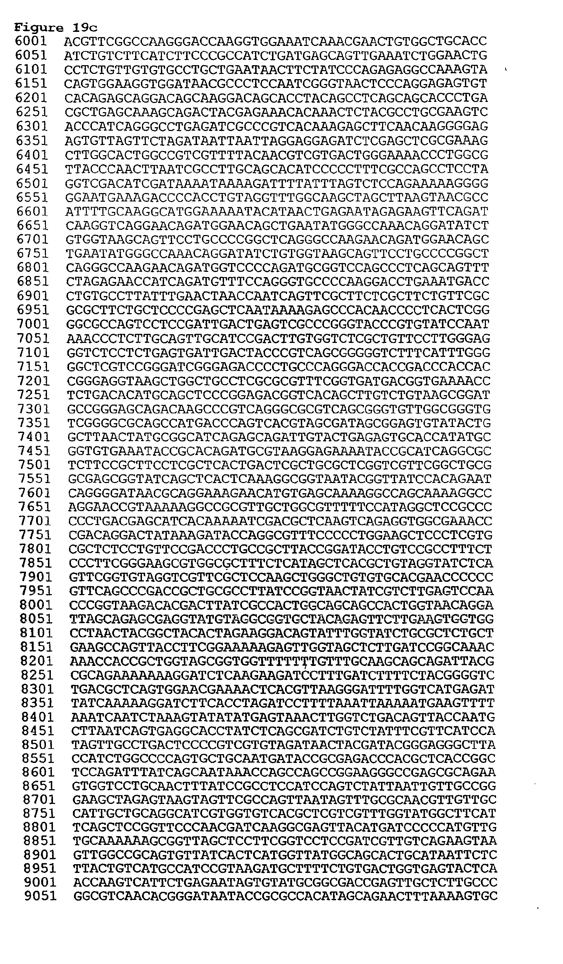

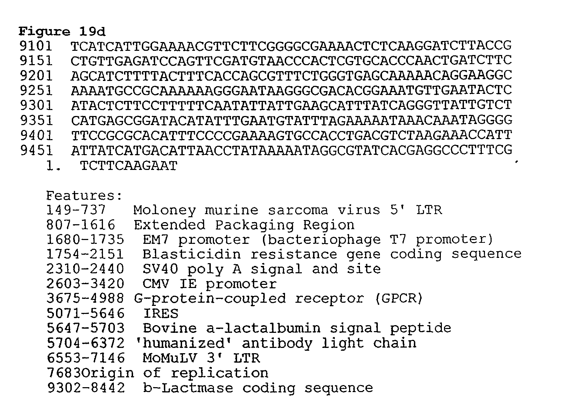

This example describes the expression of a G-Protein Coupled Receptor protein (GPCR) from a retroviral vector. This example also describes the expression of a signal protein from an IRES as a marker for expression of a difficult to assay protein or a protein that has no assay such as a GPCR. The gene construct (SEQ ID NO: 34; Figure 19) comprises a G-protein-coupled receptor followed by the IRES-signal peptide-antibody light chain cloned into the MCS of pLBCX retroviral backbone. Briefly, a PvuII/PvuII fragment (3057 bp) containing the GPCR-IRES-antibody light chain was cloned into the StuI site of pLBCX. pLBCX contains the EM7 (T7) promoter, Blasticidin gene and SV40 polyA in place of the Neomycin resistance gene from pLNCX.

The gene construct was used to produce a replication defective retroviral packaging cell line and this cell line was used to produce replication defective retroviral vector. The vector produced from this cell line was then used to infect 293GP cells (human embryonic kidney cells). After infection, the cells were placed under Blasticidin selection and single cell Blasticidin resistant clones were isolated. The clones were screened for expression of antibody light chain. The top 12 light chain expressing clones were selected. These 12 light chain expressing clones were then screened for expression of the GPCR using a ligand binding assay. All twelve of the samples also expressed the receptor protein. The clonal cell lines and there expression are shown in Table 9.

| **Table 9** | | |
|---|---|---|
| Cell Clone Number | Antibody Light Chain Expression | GPCR Expression |
| 4 | + | + |
| 8 | + | + |
| 13 | + | + |
| 19 | + | + |
| 20 | + | + |
| 22 | + | + |
| 24 | + | + |
| 27 | + | + |
| 30 | + | + |
| 45 | + | + |
| 46 | + | + |
| 50 | + | + |

### Example 22

### Multiple infection of 293 cells with replication defective retroviral vector

This example describes the multiple serial transfection of cells with retroviral vectors. The following gene construct was used to produce a replication defective retroviral packaging cell line.
5' LTR = Moloney murine sarcoma virus 5' long terminal repeat.
EPR = Moloney murine leukemia virus extended packaging region.
Blast =Blasticidin resistance gene.
CMV = Human cytomegalovirus immediate early promoter.
Gene =Gene encoding test protein
WPRE = RNA transport element
3' LTR = Moloney murine leukemia virus 3' LTR.

This packaging cell line was then used to produce a replication defective retroviral vector arranged as follows. The vector was produced from cells grown in T150 flasks and frozen. The frozen vector was thawed at each infection. For infection # 3 a concentrated solution of vector was used to perform the infection. All other infections were performed using non-concentrated vector. The infections were performed over a period of approximately five months by placing 5 ml of vector/media solution on a T25 flask containing 30% confluent 293 cells. Eight mg/ml of polybrene was also placed in the vector solution during infection. The vector solution was left on the cells for 24 hours and then removed. Media (DMEM with 10% fetal calf serum) was then added to the cells. Cells were grown to full confluency and passaged into a new T25 flask. The cells were then grown to 30% confluency and the infection procedure was repeated. This process was repeated 12 times and is outlined Table 10 below. After infections 1, 3, 6, 9 and 12, cells left over after passaging were used to obtain a DNA sample. The DNA was analyzed using the INVADER assay to determine an estimate of the number of vector inserts in the cells after various times in the infection procedure. The results indicate that the number of vector insertions goes up over time with the highest level being after the 12^{th} infection. Since a value of 0.5 is approximately an average of one vector insert copy per cell, after twelve infections the average vector insert copy has yet to reach two. These data indicates that the average vector copy per cell is a little less that 1.5 copies per cell. Also, there was no real change in gene copy number from infection #6 to infection #9. Furthermore, these data indicate that transfection conducted at a standard low multiplicity of infection fail to introduce more than one copy of the retroviral vector into the cells.

| **Table 10** | | |
|---|---|---|
| Cell Line or Infection Number | Vector Titer (CFU/ml) | "Invader" Gene Ratio |
| 293 | | 0.053 |
| Infection #1 | 1.05 X 10³ | 0.39 |
| Infection #2 | 1.05 X 10³ | |
| Infection #3 | 7.6 X 10⁴ | 0.45 |
| Infection #4 | 1.05 X 10³ | |
| Infection #5 | 1.05 X 10³ | |
| Infection #6 | 1.05 X 10³ | 0.54 |
| Infection #7 | 1.05 X 10³ | |
| Infection #8 | 1.05 X 10³ | |
| Infection #9 | 1.05 X 10³ | 0.52 |
| Infection #10 | 1.05 X 10³ | |
| Infection #11 | 1.05 X 10³ | |
| Infection #12 | 1.05 X 10³ | 0.69 |

### Example 23

### Production of YP antibody

This Example demonstrates the production of Yersinia pestis antibody by bovine mammary epithelial cells and human kidney fibroblast cells (293 cells). Cells lines were infected with the α-LA YP vector. Both of the cell lines produced YP antibody. All of the antibody is active and the heavy and light chains are produced in a ratio approximating 1:1.

### Example 24

### Transduction of Plant Protoplasts

This Example describes a method for transducing plant protoplasts. Tobacco protoplasts of Nicotiana tabacum c.v. Petit Havanna are produced according to conventional processes from a tobacco suspension culture (Potrykus and Shillito, Methods in Enzymology, vol. 118, Plant Molecular Biology, eds. A. and H. Weissbach, Academic Press, Orlando, 1986). Completely unfolded leaves are removed under sterile conditions from 6-week-old shoot cultures and thoroughly wetted with an enzyme solution of the following composition: Enzyme solution: H₂O, 70 ml; sucrose, 13 g; macerozyme R 10, 1 g; cellulase, 2 g; "Onozuka" R 10 (Yakult Co. Ltd., Japan) Drisellase (Chemische Fabrik Schweizerhalle, Switzerland), 0.13 g; and 2(n-morpholine)-ethanesulphonic acid (MES), 0.5 ml pH 6.0

Leaves are then cut into squares from 1 to 2 cm in size and the squares are floated on the above-mentioned enzyme solution. They are incubated overnight at a temperature of 26°C in the dark. This mixture is then gently shaken and incubated for a further 30 minutes until digestion is complete.

The suspension is then filtered through a steel sieve having a mesh width of 100 µm, rinsed thoroughly with 0.6M sucrose (MES, pH 5.6) and subsequently centrifuged for 10 minutes at from 4000 to 5000 rpm. The protoplasts collect on the surface of the medium which is then removed from under the protoplasts, for example using a sterilized injection syringe.

The protoplasts are resuspended in a K₃ medium [sucrose (102.96 g/1; xylose (0.25 g/1); 2,4-dichlorophenoxyacetic acid (0.10 mg/1); 1-naphthylacetic acid (1.00 mg/1); 6-benzylaminopurine (0.20 mg/1); pH 5.8](Potrykus and Shillito, supra) that contains 0.4M sucrose.

To carry out the transformation experiments, the protoplasts are first of all washed, counted and then resuspended, at a cell density of from 1 to 2.5x10⁶ cells per ml, in a W₅ medium [154 mM NaCl, 125 mM CaCl₂ x 2H₂O, 5 mM KCl, 5 mM glucose, pH 5.6), which ensures a high survival rate of the isolated protoplasts. After incubation for 30 minutes at from 6 to 8°C, the protoplasts are then used for the transduction experiments.

The protoplasts are exposed to a pseudotyped retroviral vector (e.g., a lentiviral vector) encoding a protein of interest driven by a plant specific promoter. The vector is prepared as described above and is used at an MOI of 1,000. The protoplasts are then resuspended in fresh K₃ medium (0.3 ml protoplast solution in 10 ml of fresh K3 medium). Further incubation is carried out in 10 ml portions in 10 cm diameter petri dishes at 24°C in the dark, the population density being from 4 to 8x10⁴ protoplasts per ml. After 3 days, the culture medium is diluted with 0.3 parts by volume of K₃ medium per dish and incubation is continued for a further 4 days at 24°C and 3000 lux of artifical light. After a total of 7 days, the clones that have developed from the protoplasts are embedded in nutrient medium that contains 50 mg/l of kanamycin and has been solidified with 1% agarose, and are cultured at 24°C in the dark in accordance with the "bead-type" culturing method (Shillito, et al., Plant Cell Reports, 2, 244-247 (1983)). The nutrient medium is replaced every 5 days by a fresh amount of the same nutrient solution. Analysis of the clones indicates that express the gene of interest.

### Example 25

### Stability of Vector Insertions in Cell Lines Over Time

Two cell lines that contain gene inserts of the LN-CMV-Bot vector were analyzed for their ability to maintain the vector inserts over a number of passages with and without neomycin selection. The first cell line is a bovine mammary epithelial cell line that contains a low number of insert copies. The second cell line is a 293GP line that contains multiple copies of the vector insert. At the start of the experiment, cell cultures were split. This was at passage 10 for the bovine mammary epithelial cells and passage 8 for the 293GP cells. One sample was continually passaged in media containing the neomycin analog G418, the other culture was continually passaged in media without any antibiotic. Every 3-6 passages, cells were collected and DNA was isolated for determination of gene ratio using the INVADER assay. Cell were continually grown and passaged in T25 flasks. The results of the assays are shown below:

| **Table 11** | | |
|---|---|---|
| **Low Gene Copy Cell Line** | | |
| Cell Line and Treatment | Passage Number | INVADER Gene Ratio |
| BMECBot #66 + G418 | 10 | 0.67 |
| BMEC/Bot #66 - G418 | 10 | 0.89 |
| BMECBot #66 + G418 | 16 | 0.67 |
| BMEC/Bot #66 - G418 | 16 | 0.64 |
| BMEC/Bot #66 + G418 | 21 | 0.62 |
| BMEC/Bot #66 - G418 | 21 | 0.58 |
| BMEC/Bot #66 + G418 | 27 | 0.98 |
| BMEC/Bot #66 - G418 | 27 | 0.56 |
| BMEC/Bot #66 + G418 | 33 | 0.80 |
| BMEC/Bot #66 - G418 | 33 | 0.53 |

| **Table 12** | | |
|---|---|---|
| **High Gene Copy Cell Line** | | |
| Cell Line and Treatment | Passage Number | INVADER Gene Ratio |
| 293GP/Bot #23 + G418 | 8 | 3.46 |
| 293GP/Bot #23 - G418 | 8 | 3.73 |
| 293GP/Bot #23 + G418 | 14 | 3.28 |
| 293GP/Bot #23 - G418 | 14 | 3.13 |
| 293GP/Bot #23 + G418 | 17 | 3.12 |
| 293GP/Bot #23 - G418 | 17 | 2.91 |
| 293GP/Bot #23 + G418 | 22 | 3.6 |
| 293GP/Bot #23 - G418 | 22 | 2.58 |
| 293GP/Bot #23 + G418 | 28 | 2.78 |
| 293GP/Bot #23 - G418 | 28 | 3.44 |
| 293GP/Bot #23 + G418 | 36 | 2.6 |
| 293GP/Bot #23 - G418 | 36 | 2.98 |

These data show that there are no consistent differences in gene ratio between cells treated with G418 and those not treated with antibiotic. This suggests that G418 selection is not necessary to maintain the stability of the vector gene insertions. Also, these vector inserts appear to be very stable over time.

### Example 26

### Transduction in the Absence of Selectable Marker

This example describes the transduction of host cells with a retroviral construct comprising a gene of interest and lacking a selectable marker. The retroviral vector utilized expresses the gene of interest from the CMV promoter (LTR-Extended Viral Packaging Region-Neo Gene-CMV Promoter-Gene of Interest-WPRE-LTR). A Neo (-) version was constructed by removing the Neo gene with a BsaBI/NruI restriction digest, followed by re-ligation.

### A. VIP Co-Transfection

The Vector Initial Production (VIP) method was utilized to generate host cells expressing the gene of interest. This method utilized initial co-transfection of the plasmid encoding the gene of interest and pHCMV-G DNA into 293GP^{SD} cells to produce pseudotyped virus. The procedure for producing pseudotyped virus was carried out as described (Yee et al., Meth. Cell Biol. 43:99 [1994].

Approximately 16 T150 Flasks were seeded with 293GP^{SD} cells such that the cells were 70-90% confluent on the day of VIP co-transfection. The media in the 293GP^{SD} flasks were changed with harvest medium 2 hours prior to transfection. 293GP^{SD} cells were then co-transfected with 864 µg of plasmid DNA and 864 µg of VSV-G plasmid DNA using the standard calcium phosphate co-precipitation procedure (Graham and Van der Eb, Virol. 52:456 [1973]). Briefly, pHCMV-G DNA, construct DNA, 1:10 TE, and 2M CaCl₂ were combined and mixed. 2X HBS (37°C) was placed into a separate tube. While bubbling air through the 2X HBS, the DNA/1:10 TE/2M CaCl₂ mixture was added drop wise. The transfection mixture was allowed to incubate at room temperature for 20 minutes. Following the incubation period, the correct amount of transfection mixture was added to each culture vessel. The plates or flasks were returned to 37°C, 5% CO₂ incubator for approximately six hours. Following the incubation period, the transfections were checked for the presence of crystals/precipitate by viewing under an inverted scope. The transfection media was then removed from culture vessels by aspiration with a sterile Pasteur pipet and vacuum pump and fresh harvest medium was added to each culture vessel. The culture vessels were incubated at 37°C, 5% CO₂ for 36 hr. Vector was then concentrated as described in Example 27.

### B. Generation of Host Cells Expressing the Gene of Interest

The culture medium containing virus encoding the gene of interest was used to infect the 293 cells as follows. Cells were grown in the absence of Neo selection during all stages of the infection, growth, and clonal selection. 200 µl containing 1000-5000 cells of a diluted (dilutions were made in media containing polybrene at a final concentration of 8 µg/ml) 293 cell suspension were plated in 2-6 wells of a 96 well plate. Cells were incubated at 37°C & 5% CO₂ for 1-4 hours until cells have plated. The media was removed and 50-100 µl of concentrated vector was added to the desired number of wells. Cells were incubated at 37°C & 5% CO₂ for 1 hour. Media containing polybrene was added back to a final volume of 200 µl. Cells were incubated at 37°C & 5% CO₂ overnight. At 30-40% confluency, wells were pooled and passaged to 6 well plate and subsequently T25.

Cells were then diluted into 96 well plates to a concentration of one cell per well in order to perform clonal selection. Cells from the T25 flasks were counted and then diluted to 5 cells/ml. 200 µl of the diluted solution was added to each well of a 96 well plate. Plates were indubated at 37°C & 5% CO₂ until cell are confluent and are then screened for protein production using ELISA.

### C. Results

Copy number was determined using the method described in Example 19 above. The top 24 clones were chosed based on ELISA assay from culutures in 96 well plates. The clones were expanded to 6 well and then T25 flasks. The productivity per day was determined by ELISA assay and the top 10 clones were expanded to T150 and frozen.

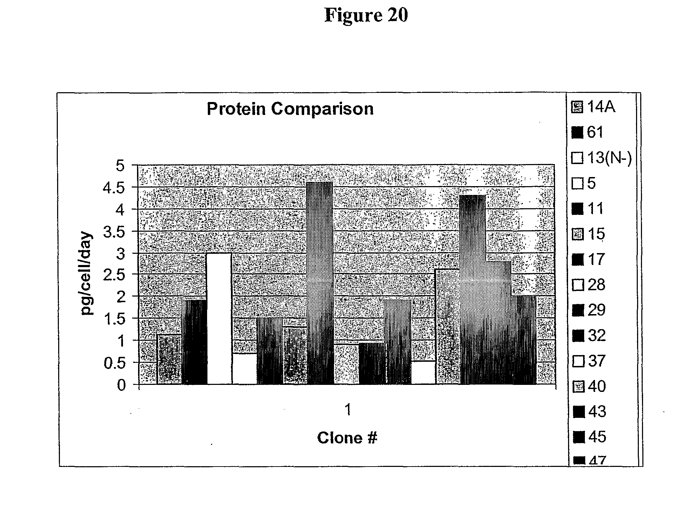

Figure 20 and Table 13 show the results of this experiment. Cell lines derived from colony number 13, which lacked a selectable marker, shows an expression level of 3 pg/cell/day. The other cells lines containing a copy number of 1 (colonies 14A, 37, and 40) showed a lower level of expression. This example demonstrates that cell lines derived from integrated vectors lacking a selectable marker and grown under non-selective conditions a) express protein from an exogenous gene, and B) express protein at a higher level than in the presence of a selectable marker.

**Table 13**

| Colony # | pg/cell/day | copy number |
|---|---|---|
| 14A | 1.14 | 1 |
| 61(2 copy) | 1.9 | 2 |
| 13(Neo-) | 3 | 1 |
| 5 | 0.7 | 2 |
| 11 | 1.5 | 3 |
| 15 | 1.3 | 3 |
| 17 | 4.6 | 3 |
| 28 | 0.9 | 2 |
| 29 | 0.92 | 2 |
| 32 | 1.9 | 2 |
| 37 | 0.52 | 1 |
| 40 | 2.61 | 1 |
| 43 | 4.3 | 3 |
| 45 | 2.8 | 2 |

### Example 27

### Concentration of Pseudotyped Retroviral Vectors

The VSV G-pseudotyped viruses were concentrated to a high titer by one cycle of ultracentrifugation. However, in certain embodiments, two cycles are performed for further concentration. The culture medium collected and filtered as described in Example 26 which contained pseudotyped virus was transferred to Oakridge centrifuge tubes (50 ml Oakridge tubes with sealing caps, Nalge Nunc International) previously sterilized by autoclaving. The virus was sedimented in a JA20 rotor (Beckman) at 48,000 x g (20,000 rpm) at 4°C for 120 min. The culture medium was then removed from the tubes in a biosafety hood and the media remaining in the tubes was aspirated to remove the supernatant. The virus pellet was resuspended to 0.5 to 1% of the original volume in 0.1X HBSS. The resuspended virus pellet was incubated overnight at 4°C without swirling. The virus pellet could be dispersed with gentle pipetting after the overnight incubation without significant loss of infectious virus. The titer of the virus stock was routinely increased 100- to 300-fold after one round of ultracentrifugation. The efficiency of recovery of infectious virus varied between 30 and 100%.

The virus stock was then subjected to low speed centrifugation in a microfuge for 5 min at 4°C to remove any visible cell debris or aggregated virions that were not resuspended under the above conditions. It was noted that if the virus stock is not to be used for injection into oocytes or embryos, this centrifugation step may be omitted.

In some embodiments, the virus stock is subjected to another round of ultracentrifugation to further concentrate the virus stock. The resuspended virus from the first round of centrifugation is pooled and pelleted by a second round of ultracentrifugation that is performed as described above. Viral titers are increased approximately 2000-fold after the second round of ultracentrifugation.

Amplification of retroviral sequences in co-cultures may result in the generation of replication competent retroviruses, thus affecting the safety of the packaging cell line and vector production. Therefore, the cell lines were screened for production of replication competent vector. The 208F cells were expanded to approximately 30% confluency in a T25 flask (~10⁵ cells). The cells were then infected with 5 ml of infectious vector at 10⁵ CFU/ml + 8 ug/ml polybrene and grown to confluency (~24 h), followed by the addition of media supplemented with G418. The cells were then expand to confluency and the media collected. The media from the infected cells was used to infect new 208F cells. The cells were plated in 6-well plates at 30% confluency (~10⁵ cells) using the following dilutions: undiluted, 1:2, 1:4, 1:6, 1:8, 1:10. Cells were expanded to confluency, followed by the addition of G418. The cells were then maintained under selection for 14 days to determine the growth of any neo resistant colonies, which indicate the presence of replication competent virus.

### Example 28

### Cell Line Stability Analysis of GPEx Created CHO Cell Lines

This example describes a comparison of cell line stability in the presence and absence of selection.

### A. Methods

Two T75 flasks per cell line were set up for the stability test: one in the presence of selection (G418) and one without selection. The seed for each set of T75s was a T150 of each cell line in log phase. One ml from each T150 was used to inoculate into 9 mls of PFCHO media (HyClone, Ogden, UT) (non-selected) and into PFCHO+G418 (400 µg/ml). Every 2-3 days 1 ml of media was collected for protein determination and cell counts. Media samples were kept at -20°C for the duration of the experiment. Cells were then passaged 1:10 into new flasks. The assay was terminated after completion of 40 generations. All the media samples collected over the 40 generations for each cell line were then assayed on the same ELISA plate for protein expression. Protein production was measured in picograms/cell/day. The analysis was performed on five cell lines (#1, 42, 137, 195 and 233). Protein assays were performed using an ELISA assay. Cell counting was performed using an Innovatis Cedex Model AS20 using manufacturers recommended procedures. The data is shown below.

### B. Results

**Cell Line #1:**

| Sample Collection Date | Productivity (pg/cell/day) Cells Grown in G418 | Productivity (pg/cell/day) Cells Grown without G418 |
|---|---|---|
| 11/30 | 0.23 | 0.07 |
| 12/3 | 0.14 | 0.06 |
| 12/6 | 0.18 | 0.25 |
| 12/10 | 0.28 | 0.10 |
| 12/13 | 0.40 | 0.08 |
| 12/16 | 0.86 | 0.10 |
| 12/19 | 0.64 | 0.05 |
| 12/23 | 1.05 | 0.10 |
| 12/26 | 0.98 | 0.13 |
| 12/30 | 0.39 | 0.13 |
| 1/3 | 0.77 | 0.25 |
| 1/6 | 0.75 | 0.21 |
| 1/9 | 0.32 | 0.06 |

**Cell Line #42:**

| Sample Collection Date | Productivity (pg/cell/day) Cells Grown in G418 | Productivity (pg/cell/day) Cells Grown without G418 |
|---|---|---|
| 11/30 | 0.25 | 0.39 |
| 12/3 | 0.12 | 0.12 |
| 12/6 | 0.32 | 0.31 |
| 12/9 | 0.20 | 0.25 |
| 12/12 | 0.22 | 0.24 |
| 12/16 | 0.23 | 0.43 |
| 12/19 | 0.44 | 0.37 |
| 12/23 | 0.29 | 0.20 |
| 12/26 | 0.36 | 0.47 |
| 12/30 | 0.35 | 0.27 |
| 1/3 | 0.33 | 0.28 |

**Cell Line #137:**

| Sample Collection Date | Productivity (pg/cell/day) Cells Grown in G418 | Productivity (pg/cell/day) Cells Grown without G418 |
|---|---|---|
| 11/30 | 0.10 | 0.10 |
| 12/3 | 0.05 | 0.02 |
| 12/6 | 0.09 | 0.12 |
| 12/10 | 0.13 | 0.08 |
| 12/13 | 0.10 | 0.22 |
| 12/16 | 0.14 | ND |
| 12/19 | 0.16 | 0.02 |
| 12/23 | 0.19 | 0.05 |
| 12/26 | 0.17 | 0.07 |
| 12/30 | 0.17 | 0.04 |
| 1/3 | 0.36 | 0.14 |
| 1/6 | 0.24 | 0.27 |
| 1/9 | 0.11 | 0.09 |

**Cell Line #195:**

| Sample Collection Date | Productivity (pg/cell/day) Cells Grown in G418 | Productivity (pg/cell/day) Cells Grown without G418 |
|---|---|---|
| 11/30 | 1.03 | 0.30 |
| 12/3 | 0.11 | 0.08 |
| 12/6 | 0.18 | 0.22 |
| 12/10 | 0.23 | 0.39 |
| 12/13 | 0.24 | 0.77 |
| 12/16 | 0.18 | 0.76 |
| 12/19 | 0.37 | 0.85 |
| 12/23 | 0.73 | 0.30 |
| 12/26 | 1.03 | 0.51 |
| 12/30 | 0.97 | 0.27 |
| 1/3 | 0.54 | 0.48 |

**Cell Line #233:**

| Sample Collection Date | Productivity (pg/cell/day) Cells Grown in G418 | Productivity (pg/cell/day) Cells Grown without G418 |
|---|---|---|
| 11/30 | 0.38 | 0.17 |
| 12/3 | 0.12 | 0.04 |
| 12/6 | 0.15 | 0.13 |
| 12/10 | 0.12 | 0.10 |
| 12/13 | 0.35 | 0.14 |
| 12/16 | 0.37 | 0.08 |
| 12/19 | 0.15 | 0.14 |
| 12/23 | 0.16 | 0.07 |
| 12/26 | 0.32 | 0.19 |
| 12/30 | 0.41 | 0.10 |
| 1/3 | 0.35 | 0.28 |

To determine whether neo selection had an effect on protein expression over 40 generations, analysis of variance was performed on the data. The model included the following variables: antibiotic selection, line, generation and interations between each variable. The data indicate that there was no effect of including G418 in the media (p>0.10) on cell productivity over the 40 generations. The p-values for each cell line are shown in the table below. There was also no significant decrease in cell productivity over time in any of the cell lines grown with or without G418.

| Cell Line | p-Value |
|---|---|
| 1 | 0.51 |
| 42 | 0.29 |
| 137 | 0.15 |
| 195 | 0.53 |
| 233 | 0.27 |

## Claims

1. Use of a stable host cell for production and purification of a protein of interest, said host cell comprising a genome, said genome comprising at least ten integrated pseudotyped retroviral vectors, wherein said retroviral vector comprises at least one exogenous gene encoding said protein of interest operably linked to a promoter, and wherein said pseudotyped retroviral vectors lacks a gene encoding a selectable marker.

2. The use of claim 1, wherein said retroviral vector further comprises a secretion signal sequence operably linked to said exogenous gene.

3. The use of claim 1 or 2, wherein said retroviral vector further comprises an RNA stabilizing element operably linked to said exogenous gene.

4. The use of any one of claims 1 to 3, wherein said pseudotyped retroviral vector comprises a G glycoprotein selected from the group consisting of vesicular stomatitis virus, Piry virus, Chandipura virus, Spring viremia of carp virus and Mokola virus G glycoproteins.

5. The use of any one of claims 1 to 4, wherein said retroviral vector comprises long terminal repeats selected from the group consisting of MoMLV, MoMuSV, and MMTV long terminal repeats.

6. The use of any one of claims 1 to 5, wherein said host cell is clonally derived or non-clonally derived.

7. The use of any one of claims 1 to 6, wherein genome is stable for greater than 10 passages.

8. The use of claim 7, wherein said genome is stable for greater than 100 passages.

9. The use of any one of claims 1 to 8, wherein said integrated exogenous gene is stable in the absence of selection.

10. The use of any one of claims 1 to 9, wherein said at least one exogenous gene is selected from the group consisting of genes encoding antigen binding proteins, pharmaceutical proteins, kinases, phosphatases, nucleic acid binding proteins, membrane receptor proteins, signal transduction proteins, ion channel proteins, and oncoproteins.

11. The use of any one of claims 1 to 10, wherein said genome comprises at least 100 integrated retroviral vectors.

12. The use of any one of claims I to 11, wherein said host cell expresses greater than about 3 picograms of said exogenous protein per cell per day.

13. The use of any one of claims 1 to 12, wherein said host cell expresses greater than about 10 picograms of said exogenous protein per cell per day.

## Patentansprüche

1. Verwendung einer stabilen Wirtszelle zur Herstellung und Reinigung von einem Protein von Interesse, wobei die Wirtszelle ein Genom umfasst, das Genom umfassend mindestens zehn integrierte pseudotypisierte retroviralen Vektoren, wobei der retrovirale Vektor mindestens ein exogenes Gen umfasst, das das Protein von Interesse kodiert, und funktionell an einen Promotor verknüpft ist, und wobei den pseudotypisierten retroviralen Vektoren ein Gen fehlt, das einen selektierbaren Marker kodiert.

2. Verwendung nach Anspruch 1, wobei der retrovirale Vektor ferner eine Sequenz eines Sekretionssignals funktionell verknüpft an das exogene Gen umfasst.

3. Verwendung nach Anspruch 1 oder 2, wobei der retrovirale Vektor ferner ein RNA stabilisierendes Element funktionell verknüpft an das exogene Gen umfasst.

4. Verwendung nach einem der Ansprüche 1 bis 3, wobei der pseudotypisierte retrovirale Vektor ein G-Glycoprotein umfasst, ausgewählt aus der Gruppe bestehend aus dem Vesicular stomatitis Virus, Piry Virus, Chandipura-Virus, Virus der Frühlingsvirämie der Karpfen und Mokola Virus G Glykoproteinen.

5. Verwendung nach einem der Ansprüche 1 bis 4, wobei der retrovirale Vektor lange terminale Wiederholungen umfasst, ausgewählt aus der Gruppe bestehend aus MoMLV, MoMuSV und MMTV langen terminalen Wiederholungen.

6. Verwendung nach einem der Ansprüche 1 bis 5, wobei die Wirtszelle klonal abgeleitet oder nicht-klonal abgeleitet ist.

7. Verwendung nach einem der Ansprüche 1 bis 6, wobei das Genom für mehr als 10 Passagen stabil ist.

8. Verwendung nach Anspruch 7, wobei das Genom für mehr als 100 Passagen stabil ist.

9. Die Verwendung nach einem der Ansprüche 1 bis 8, wobei das integrierte Gen in Abwesenheit von Selektion stabil ist.

10. Die Verwendung nach einem der Ansprüche 1 bis 9, wobei das mindestens eine exogene Gen ausgewählt ist aus der Gruppe bestehend aus Genen kodierend Antigen-bindende Protein, pharmazeutische Proteine, Kinasen, Phosphatasen, Nukleinsäure-bindende Proteine, Membranrezeptor-Proteine, Signaltransduktionsproteine, Ionenkanalproteine und Oncoproteine.

11. Die Verwendung nach einem der Ansprüche 1 bis 10, wobei das Genom mindestens 100 integrierte retrovirale Vektoren umfasst.

12. Verwendung nach einem der Ansprüche 1 bis 11, wobei die Wirtszelle mehr als ungefähr 3 Pikogramm des exogenen Proteins pro Zelle pro Tag exprimiert.

13. Verwendung nach einem der Ansprüche 1 bis 12, wobei die Wirtszelle mehr als ungefähr 10 Pikogramm des exogenen Proteins pro Zelle pro Tag exprimiert.

## Revendications

1. Utilisation d'une cellule hôte stable pour la production et la purification d'une protéine d'intérêt, ladite cellule hôte comprenant un génome, ledit génome comprenant au moins dix vecteurs rétroviraux pseudotypés intégrés, méthode dans laquelle ledit vecteur rétroviral comprend au moins un gène exogène encodant ladite protéine d'intérêt liée de manière opérationnelle à un promoteur, et dans laquelle lesdits vecteurs rétroviraux pseudotypés ne comportent pas de gène encodant un marqueur sélectionnable.

2. Utilisation de la revendication 1, dans laquelle ledit vecteur rétroviral comprend, en outre, une séquence de signaux de sécrétion liée de manière opérationnelle liée audit gène exogène.

3. Utilisation des revendications 1 ou 2, dans laquelle ledit vecteur rétroviral comprend un élément stabilisant d'ARN lié de manière opérationnelle audit gène exogène.

4. Utilisation de l'une quelconque des revendications 1 à 3, dans lesquelles ledit vecteur rétroviral pseudotypé comprend une glycoprotéine G sélectionnée dans un groupe de glycoprotéines G des virus suivants : stomatite vésiculeuse, virus Piry, virus Chandipura, virus de la virémie printanière de la carpe et virus Moloka.

5. Utilisation de l'une quelconque des revendications 1 à 4, dans lesquelles le dit vecteur rétroviral comprend de longues séquences terminales répétées sélectionnées à partir d'un groupe comprenant les longues séquences terminales répétées MoMLV, MoMuSV et MMTV.

6. Utilisation de l'une quelconque des revendications 1 à 5, dans lesquelles ladite cellule hôte est dérivée par clonage ou n'est pas dérivée par clonage.

7. Utilisation de l'une quelconque des revendications 1 à 6, dans lesquelles le génome est stable pendant plus de 10 passages.

8. Utilisation de la revendication 7, dans laquelle le génome est stable pendant plus de 100 passages.

9. Utilisation de l'une quelconque des revendications 1 à 8, dans lesquelles ledit gène exogène intégré est stable en l'absence de sélection.

10. Utilisation de l'une quelconque des revendications 1 à 9, dans lesquelles au moins un gène exogène est sélectionné à partir d'un groupe constitué de gènes encodant des protéines de liaison aux antigènes, des protéines pharmaceutiques, des kinases, des phosphatases, des protéines de liaison à l'acide nucléique, des protéines récepteur de membrane, des protéines intervenant dans la transduction de signal, des protéines des canaux ioniques et des oncoprotéines.

11. Utilisation de l'une quelconque des revendications 1 à 10, dans lesquelles ledit génome comprend au moins 100 vecteurs rétroviraux intégrés.

12. Utilisation de l'une quelconque des revendications 1 à 11, dans lesquelles ladite cellule hôte exprime plus de 3 picogrammes environ de ladite protéine exogène par cellule et par jour.

13. Utilisation de l'une quelconque des revendications 1 à 12, dans lesquelles ladite cellule hôte exprime plus de 10 picogrammes environ de ladite protéine exogène par cellule et par jour.
